(19) Europäisches Patentamt / European Patent Office / Office européen des brevets

(11) **EP 1 782 770 A1**

(12) **EUROPEAN PATENT APPLICATION**
published in accordance with Art. 158(3) EPC

(43) Date of publication:
**09.05.2007 Bulletin 2007/19**

(51) Int Cl.:
*A61F 7/08* (2006.01)   *C09K 5/16* (2006.01)

(21) Application number: **05765738.9**

(22) Date of filing: **14.07.2005**

(86) International application number:
**PCT/JP2005/013011**

(87) International publication number:
**WO 2006/006658 (19.01.2006 Gazette 2006/03)**

(84) Designated Contracting States:
**DE FR GB HU IT**

(30) Priority: **14.07.2004 JP 2004207839**

(71) Applicant: **Mycoal Products Corporation
Tochigi-chi, Tochigi 328-0067 (JP)**

(72) Inventors:
• **Dodo, Toshihiro,**
c/o Mycoal Products Corp.,
Tochigi-shi, Tochigi 3280067 (JP)
• **Kimura, Hisao,**
c/o Mycoal Products Corp.,
Tochigi-shi, Tochigi 3280067 (JP)
• **Aida, Michio,**
c/o Mycoal Products Corp.,
Tochigi-shi, Tochigi 3280067 (JP)

(74) Representative: **Thun, Clemens et al
Mitscherlich & Partner
Sonnenstrasse 33
80331 München (DE)**

(54) **HEATING ELEMENT FOR FOOT WARMING AND PROCESS FOR PRODUCING THE SAME**

(57)   To provide a foot warming heat generating body having excellent shape holding properties and capable of continuing the heat generation over a long period of time by using a heat generating composition having excellent shape holding properties and capable of causing the heat generation without necessity of the removal of water such as water absorption and dehydration by a substrate after molding.

The foot warming heat generating body is **characterized in that** a heat generating composition molded body made of a heat generating composition which contains, as essential components, an exothermic substance, a carbon component, a reaction accelerator and water, has a water mobility value showing a surplus water content of from 0.01 to 20, with the water in the heat generating composition not functioning as a barrier layer, and is capable of causing an exothermic reaction upon contact with air is laminated on a substrate; a covering material is put thereon; the periphery of the heat generating composition molded body is sealed; the heat generating composition molded body has a shape retaining degree of 70 or more; and at least a part of the substrate or the covering material has permeability to air.

FIG.10(a)

## FIG.10(b)

## FIG.10(c)

**Description**

[Technical Field]

The present invention relates to a foot warming heat generating body for supplying heat to a foot and to a process for producing a foot warming heat generating body.

[Background Art]

Hitherto, it has been proposed to warm a foot by applying to a footwear such as shoes and slippers a heat generating body which utilizes a heat generating body composition containing, as the major component, a metal power such as an iron powder in a powdered state or a pasty state and which utilizes reaction heat with oxygen in air.
For example, Patent Document 1 proposes to form an accommodating part for a heat generating body in an insole of a shoe and to provide a heat generating body as accommodated in an air-permeable bag in this accommodating part.
In addition, Patent Document 2 proposes a heat generating body for foot such as shoes, in which a heat generating agent is accommodating in a flat air-permeable bag having a shape responsible to a partial shape of the foot and a non-turnover adhesive layer is provided on one surface of the air-permeable bag, thereby bonding to the surface of a foot due to the non-turnover adhesive layer.
Patent Document 3 proposes a method of fixing a heat generating composition by an adhesive.
Patent Document 4 employs a method of laminating a viscous creamy heat generating composition containing a thickener on a water absorptive substrate in a film-like or sheet-like form by a printing method such as screen printing, covering by a covering material and sealing the entirety of the peripheries of the substrate and the covering material with a hot melt based adhesive by pressure sealing (adhesive sealing).
In a foot warming heat generating body prepared by using a powdered heat generating composition having neither moldability nor shape holding properties and heat sealing and enclosing it by the charging system, the high-speed production was difficult, the movement of the heat generating composition within the accommodating bag occurred at the time of use, the heat generating body itself was deformed, and a feeling for use was remarkably poor. On the other hand, a foot warming heat generating body prepared by a pasty heat generating composition requiring a water absorptive substrate and adhesive sealing and enclosing it by the printing system has such advantages that the high-speed production is possible, that the heat generating composition does not move within the accommodating bag at the time of use and that the heat generating body itself is not deformed. However, it involved such problems that the exothermic time is short and that the deformation of the water absorptive substrate is caused to produce a problem in the mechanical strength. Thus, it was problematic as utility goods. There was not available a foot warming heat generating body in which the high-speed production is possible, the exothermic time is long, a heat generating composition does not move within an accommodating bag at the time of use, a heat generating body itself is not deformed, the mechanical strength is strong, and a feeling for use is well.
That is, in the conventional foot warming heat generating bodies made of a powdered heat generating composition, there were involved such problems that the whole of the foot warming heat generating body is thick; a touch is rough so that a feel is bad; the flexibility is lowered so that they hardly adapt to a complicated unevenness on the surface of the body or a curved surface with a low curvature; the extensibility or shrinkability is lowered so that the heat generating composition moves following the movement of the body; the heat generating body is deformed so that the follow-up properties to the surface of the heat generating body are poor; and a feeling for use becomes remarkably deteriorated.
In the conventional foot warming heat generating bodies made of a powdered heat generating composition, only a rough curved surface shape can be formed, the curved surface is not adaptive depending upon the kind of a shoe, warping partially occurs, and the foot is oppressed to cause a foot pain. Thus, there were limits in shoes which can be used so that large dissatisfaction is produced.
In the conventional foot warming heat generating bodies made of a powdered heat generating composition, wettability is given to the heat generating composition by water. However, since the blending ratio of water is low to such extent that it is suitable for an exothermic reaction, the heat generating composition is powdery and poor in fluidity; it is difficult to uniformly distribute the heat generating composition within a prescribed range on the substrate; the thickness of the heat generating composition in the inside of the foot warming heat generating body is not constant; during the use, the heat generating composition moves within an inner bag, or the heat generating body slips off or is broken to cause deviation; and the thickness of the heat generating composition in the inside of the foot warming heat generating body is not constant. Thus, in the case where such a foot warming heat generating body is fixed to the body and used, there were caused serious problems such that since the exothermic temperature distribution is not constant, when it is fixed to the same location and used, it causes a burn, a strong stimulus is given to the skin, and it may possibly generate skin injuries such as redness, rash and eruption.
In a method of fixing a powdered heat generating composition by a bonding agent, in the actual production, it is substantially impossible to bond the powered heat generating composition to the inside of a bag, and even when boding could be done, the bonding strength is weak so that complete fixing is impossible, peeling is caused during the use, the product becomes a plate-like material having poor flexibility, a feeling for use is poor, and unevenness or scattering of the temperature is caused. Thus, such a method fails in practicality.

On the other hand, in the case of a viscous pasty or creamy heat generating composition using a thickener, though the moldability is good and uniformity of the thickness can be kept, desired exothermic amount and exothermic time are not obtained due to not only insufficient draining of surplus water but also influences of the thickener or binding agent so that it is impossible to make the size large and to perform the heat generation over a long period of time. Thus, the practicality was limited.

Also, in order to discharge out the surplus water from the heat generating composition laminate, the surplus water was absorbed by paper which is a part of the substrate. However, when the paper absorbs water, the mechanical strength is extremely lowered. Thus, peeling is caused at the time of use, and a part of the heat generating composition laminate leaks out, resulting in problems such as soiling of socks, etc.

Also, in order to absorb the surplus water on the substrate, etc., the surroundings of the heat generating composition laminate was contact bond sealed by using an adhesive layer in place of a heat seal. However, even in this case, the sealing force is weak. In particular, when water is absorbed on the substrate, etc., the adhesive strength is weakened. Thus, peeling is caused at the time of use, and a part of the heat generating composition laminate leaks out, resulting in problems such as soiling of socks, etc.

Also, in a method of intermittently moving a substrate and throwing down a heat generating composition during stopping of the substrate, there was a problem that since stopping and starting of the substrate are frequently repeated, the production speed becomes slow.

In a method of not only moving a substrate at a fixed rate but also throwing down a heat generating composition on the substrate while moving a throwing port from which the heat generating composition is thrown down at the same rate as in the substrate, since stopping and starting of the substrate are not substantially repeated, the production rate can be enhanced. However, not only a complicated mechanism for moving the throwing port from which the heat generating composition is thrown down at the same rate as in the substrate is required, but also the heat generating composition is poor in fluidity because it contains a powder and water. Thus, there was involved such a problem that the rate at which the mechanism is moved is largely limited.

In the conventional powdered heat generating compositions, wettability is given by water. Since water is blended only in an amount such that the blending ratio of water is adaptive to the exothermic reaction, the fluidity is extremely poor. Thus, it was markedly difficult to uniformly distribute the heat generating composition within a prescribed range on the substrate merely by throwing down it.

For that reason, in putting a covering material thereon and performing sealing, the distribution of the heat generating composition is made uniform to some extent by using a roll, etc. However, the distribution of the heat generating composition was liable to be deviated toward the sender direction of the bag due to properties of the powdered heat generating composition.

Also, a semi-kneaded heat generating composition is a heat generating composition in which all of components including a binding agent are blended in a proper blending ratio, and a tablet-making step must be introduced. Thus, the process became complicated.

Also, in a non-viscous slurry-like heat generating composition, the shape cannot be held, and a fixed shape cannot be molded. Thus, it is molded via a complicated process such as paper-making.

Also, in the case of a method in which the water content in a heat generating composition is slightly increased and water is absorbed on a substrate which contains a water absorptive material such as paper in a part of the structure thereof, thereby causing the heat generation, the mechanical strength of the substrate which has absorbed water is lowered, resulting in a problem such that the paper is peeled away at the time of use.

A heat generating composition having moldability, shape holding properties and an exothermic characteristic such that the heat generation is continued over a long period of time and a heat generating body using the same and a simple production process thereof have been awaited.

[Patent Document 1] JP-UM-A-61-8013

[Patent Document 2] JP-A-2-172460

[Patent Document 3] JP-A-62-347

[Patent Document 4] JP-A-9-276317


[Disclosure of the Invention]


[Problems that the Invention is to Solve]


The invention is to provide a foot warming heat generating body having excellent shape holding properties and capable of continuing the heat generation over a long period of time by using a heat generating composition having excellent shape holding properties and capable of causing the heat generation without necessity of the removal of water such as water absorption and dehydration by a substrate after molding.

[Means for Solving the Problems]

As set forth in claim 1, a foot warming heat generating body of the invention is characterized in that a heat generating composition molded body made of a heat generating composition which contains, as essential components, an exothermic substance, a carbon component, a reaction accelerator and water, has a water mobility value showing a surplus water content of from 0.01 to 20, with the water in the heat generating composition not functioning as a barrier layer, and is capable of causing an exothermic reaction upon contact with air is laminated on a substrate; a covering material is put thereon; the periphery of the heat generating composition molded body is sealed; the heat generating composition molded body has a shape retaining degree of 70 or more; and at least a part of the substrate or the covering material has permeability to air.

Also, a foot warming heat generating body as set forth in claim 2 is characterized in that in the foot warming heat generating body as set forth in claim 1, the heat generating composition contains a component resulting from a contact treatment of a heat generating mixture containing at least an iron powder, a carbon component, a reaction accelerator and water as essential components with an oxidizing gas.

Also, a foot warming heat generating body as set forth in claim 3 is characterized in that in the foot warming heat generating body as set forth in claim 1, the iron powder is covered on at least a part of the surface thereof by an iron oxide film, the iron oxide film has a thickness of 3 nm or more, and the iron powder at least contains from 20 to 100 % by weight of an active iron powder having a region of an oxygen-free iron component in at least one region selected from a central part region of the iron powder and a region beneath the iron oxide film.

Also, a foot warming heat generating body as set forth in claim 4 is characterized in that in the foot warming heat generating body as set forth in claim 1, the iron powder comprising particles, a surface of each of which is at least partially covered with a wustite film and contains from 20 to 100 % by weight of an active iron powder having an amount of wustite of from 2 to 50 % by weight in terms of an X-ray peak intensity ratio.

Also, a foot warming heat generating body as set forth in claim 5 is characterized in that in the foot warming heat generating body as set forth in claim 1, the heat generating composition molded body is compressed.

Also, a foot warming heat generating body as set forth in claim 6 is characterized in that in the foot warming heat generating body as set forth in claim 1, an air-permeable sticky layer is provided for the purpose of bonding of the substrate, the covering material or the heat generating composition molded body.

Also, a foot warming heat generating body as set forth in claim 7 is characterized in that in the foot warming heat generating body as set forth in claim 1, the sealing is heat seal.

Also, a foot warming heat generating body as set forth in claim 8 is characterized in that in the foot warming heat generating body as set forth in claim 7, the heat seal is formed by heat sealing after temporary adhesion, and an adhesive component which constitutes the sticky layer and a component of a heat seal material which constitutes the heat seal layer are copresent in the heat seal part.

Also, a foot warming heat generating body as set forth in claim 9 is characterized in that in the foot warming heat generating body as set forth in claim 1, the heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

Also, a foot warming heat generating body as set forth in claim 10 is characterized in that in the foot warming heat generating body as set forth in claim 1, a core material is provided in a part of the foot warming heat generating body.

Also, a foot warming heat generating body as set forth in claim 11 is characterized in that in the foot warming heat generating body as set forth in claim 1, the substrate, the covering material, the core material or the heat generating composition molded body is subjected to compression processing.

Also, a foot warming heat generating body as set forth in claim 12 is characterized in that in the foot warming heat generating body as set forth in claim 1, the foot warming heat generating body is formed corresponding to the shape of a prescribed site of the foot.

Also, a foot warming heat generating body as set forth in claim 13 is characterized in that in the foot warming heat generating body as set forth in claim 1, a plural number of the heat generating composition molded bodies are sectioned by a sectioned part having the seal formed therein, and a plural number of sectional exothermic parts are disposed.

Also, a foot warming heat generating body as set forth in claim 14 is characterized in that in the foot warming heat generating body as set forth in claim 1, the sectional exothermic parts and the sectioned parts have a difference of altitude, the sectional exothermic parts and the sectioned parts are covered by an air permeability adjusting material, and air is taken in from the sides of the both end parts of the air permeability adjusting material.

Also, a foot warming heat generating body as set forth in claim 15 is characterized in that in the foot warming heat generating body as set forth in claim 14, the air permeability adjusting material is an air-impermeable raw material.

Also, a foot warming heat generating body as set forth in claim 16 is characterized in that in the foot warming heat generating body as set forth in claim 1, a fixing measure is provided in at least a part of the exposed surface of the substrate or the covering material.

Also, a foot warming heat generating body as set forth in claim 17 is characterized in that in the foot warming heat generating body as set forth in claim 1, the fixing measure is an adhesive layer, and the adhesive layer contains at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous substance, a moisturizer, a functional substance, and a mixture thereof.

As set forth in claim 18, a process for producing a foot warming heat generating body of the invention is a process for producing the foot warming heat generating body as set forth in claim 1, which is characterized in that the production process is a basic process for successively carrying out a first step, a second step, a third step and a fourth step; and a step selected from the following first step, second step (second A step, second B step, second C step, and second D step), third step (third A step, third B step, and third C step), fourth step (fourth A step), fifth step, sixth step (sixth A step and sixth B step), seventh step (seventh A step), eighth step, ninth step and tenth step inclusive of duplicated steps thereof is arbitrarily mediated in the basic process, as the need arises:

First step: production step of heat generating composition
Second step: molding step (substrate and magnet), second A step: force-through die molding step (trimming die and leveling plate), second B step: cast molding method (casting die and leveling plate), second C step: force-in die molding step (trimming die and pushing plate), second D step: in-mold compression step
Third step: lamination, spraying and coating step for heat generating composition, etc., third 3A step: setting-up step of air-permeable adhesive polymer, third B step: lamination, spraying and coating step for substrate, etc., third C step: surface treatment step of heat generating composition
Fourth step: covering step (covering material), fourth A step: covering step (underlay material)
Fifth step: pressurizing step
Sixth step: sealing step, sixth A step: temporary adhesion and heat sealing step, sixth B step: deadhesion step
Seventh step: setting-up step of non-slip layer, seventh A step: setting-up step of air adjusting material
Eighth step: punching-out step of heat generating body
Ninth step: accommodating step of foot warming heat generating body in air-impermeable accommodating bag
Tenth step: punching-out step of outer bag

[Advantages of the Invention]

As described previously, the foot warming heat generating body of the invention brings the following advantages.

1) Because of the matters that the foot warming heat generating body is formed in an ultra-thin form and that the core material is used, since the shape retaining degree of the heat generating composition molded body is 70 or more, it is not necessary that the shape be held by employing a reduced pressure. Thus, all films of porous films, perforated films, and so on can be used; the selection width of an air hole in the air-permeable part is extremely widened; the exothermic characteristic can be more painstakingly designed; the generation of an abnormal high temperature point and/or an abnormal high temperature part due to uneven distribution of the heat generating composition can be surely prevented; the generation of a moderate-temperature burn can be surely prevented; the safety at the time of use is more enhanced; and a more comfortable foot temperature can be obtained.
2) Since a non-water absorptive packaging material is used and the heat generating composition laminate is sealed therein by heat sealing, the mechanical strength of the packaging material is not deteriorated, and soiling due to the leakage of the heat generating composition does not occur during and after the use.
3) In using the foot warming heat generating body, by breaking an air-tight storage bag, the heat generation is immediately started, a required exothermic temperature is quickly obtained, and the required exothermic temperature can be kept over a long period of time.
4) The foot warming heat generating body can be shaped in various forms, can adapt to an arbitrary site of the foot with high fitness to complicated irregular shapes in an arbitrary site of the foot such as curved parts and bending parts and can effectively warm an arbitrary site of the foot which is required to be warmed. As a result, a warmth taking effect is obtained.
5) By providing a core material in a part of at least one member of the substrate or the covering material, the shape is more stabilized before, during and after the use, and the heat generating composition laminate is more stabilized during the use. Thus, a feeling for use is excellent.

6) An adhesive layer or a non-slip layer is formed on the exposed surface of any one of the substrate or the covering material; the foot warming heat generating body can be easily fixed in an arbitrary site of the foot or the movement of the foot warming heat generating body itself in a footwear can be prevented; and a desired place can be kept at a proper temperature over a long period of time.

7) Since molding can be achieved by lamination, the fluidity is remarkably high as compared with the conventional powdered heat generating compositions. For example, the heat generating composition can be continuously laminated in a prescribed range on the substrate which is sent at a high speed of 50 m/min or more preciously, uniformly and very thinly by force-through die molding, printing or coating.

8) In performing high-speed production, in order to synchronize a feed unit of the substrate, a lamination unit by a force-through die molding method of a non-viscous heat generating composition, a lamination unit of the covering material, a molding unit such as a punching-out unit of a foot warming heat generating body for punching out the resulting laminate into a shape so as to cover an arbitrary site of the foot, a packaging unit for sealing the foot warming heat generating body in an air-tight bag, and the like, it is only required to achieve the adjustment so as to synchronize the operation speed. Thus, it is possible to achieve the production of high-speed continuous operation by a simple unit construction.

[Best Modes for Carrying Out the Invention]

The foot warming heat generating body of the invention is a foot warming heat generating body, wherein a heat generating composition molded body made of a heat generating composition which contains, as essential components, an exothermic substance, a carbon component, a reaction accelerator and water, has a water mobility value showing a surplus water content of from 0.01 to 20, with the water in the heat generating composition not functioning as a barrier layer, and is capable of causing an exothermic reaction upon contact with air is laminated on a substrate; a covering material is put thereon; the periphery of the heat generating composition molded body is sealed; the heat generating composition molded body has a shape retaining degree of 70 or more; and at least a part of the substrate or the covering material has permeability to air. Because of the matters that the foot warming heat generating body is formed in an ultra-thin form and that the core material is used, since the shape retaining degree of the heat generating composition molded body is 70 or more, it is not necessary that the shape be held by employing a reduced pressure. Thus, all films of porous films, perforated films, and so on can be used; the selection width of an air hole in the air-permeable part is extremely widened; the exothermic characteristic can be more painstakingly designed; the generation of an abnormal high temperature point and/or an abnormal high temperature part due to uneven distribution of the heat generating composition can be surely prevent; the generation of a moderate-temperature burn can be surely prevented; the safety at the time of use is more enhanced; and a more comfortable foot temperature can be obtained.

Incidentally, in the invention, what water does not function as a barrier layer and causes an exothermic reaction upon contact with air means that water in a heat generating composition does not function as a barrier layer which is an air intercepting layer and immediately after the production of a heat generating composition, comes into contact with air, thereby immediately causing an exothermic reaction.

The foot warming heat generating body of the invention is a foot warming heat generating body of a molding system by molding a heat generating composition in an arbitrary shape of the foot and in an arbitrary thickness or size on a substrate by a molding system such as force-in die molding, force-through die molding, compression molding, and cast molding, laminating, putting a covering material thereon and sealing the periphery of a heat generation composition molded body. It is also possible to make it easy to release the heat generating composition molded body from a die by using a magnet at the time of molding.

The shape of the foot warming heat generating body of the invention is not particularly limited. Specifically, for example, the foot warming heat generating body may be formed into an arbitrary shape by forming it corresponding to the planar shape of the whole of the foot or forming it corresponding to the planar shape of a part of the foot, for example, forming it corresponding to the planar shape of the toe part of the foot, forming it corresponding to the planar shape of the arch of the foot, forming it corresponding to the planar shape of the extended part of the arch of the foot, and forming it corresponding to the planar shape of the heel.

Furthermore, the size of the foot warming heat generating body of the invention is not particularly limited, but it may be properly determined such that it can be used for foot warming.

With respect to the shape of the heat generating composition molded body of the invention, the heat generating composition molded body may be formed into a shape so as to cover an arbitrary site of the foot, for example, a shape of covering a part of the sole side of the foot, a shape of covering the whole of the sole side of the foot, a shape of covering a part of the instep side of the foot, a shape of covering the whole of the instep side of the foot, a shape of covering a part or the whole of the sole side or instep side of the foot and a part or the whole of the lateral side of the foot, or a shape of covering a part or the whole of the sole side of the foot, a part or the whole of the lateral side of the foot and a part or the whole of the instep side of the foot. Furthermore, a concave or the like may be present in the central part of the heat generating composition molded body or the like. In the invention, a heat generation composition compressed body which is a compressed heat generating composition molded body is included in the heat generating composition molded body, too.

Furthermore, in the case where the exothermic part is constituted of plural sectional heat exothermic parts and sectioned parts which is a heat seal part, when accommodated in the sectional exothermic parts, the whole of the exothermic part may be formed in a foot shape or the like. Furthermore, the heat generating composition molded body may be formed into a foot shape or the like, thereby forming the shape of the sectional exothermic parts into a foot shape or the like.

As the part of the sole side of the foot, fingers, finger bases, pad parts, the arch of the foot, the heel, and so on are representative. Examples of the shape of the foot warming heat generating body of covering the bottom side of the finger include a semicircular shape and a semi-elliptical shape.

Furthermore, examples of the shape of the substrate and the covering material of covering finger bases, pad parts, the arch of the foot, the heel, and so on include a rectangular shape, a square rectangular shape, a trapezoidal shape, an oval shape, an elliptical shape, a circular shape, a semi-elliptical shape, a semicircular shape, and a horseshoe shape.

Examples of the shape of covering the whole of the sole side of the foot include an insole shape the same as the insole of a shoe. In addition, a shape in which an expanding part corresponding to the arch of the foot is extended in a constricted portion corresponding to the arch of the foot of the insole shape can be enumerated.

Incidentally, in this case, for example, since it can be interpreted that a fixed height is present in the arch of the foot in viewing from the lateral size, it is also possible to interpret that the shape is a shape of covering the sole side of the foot as described below, especially the whole of the arch portion of the foot in the sole side of the foot and a part of the lateral side of the foot.

Examples of the shape of covering a part or the whole of the sole side of the foot and a part of the lateral side of the foot include a shape of covering the whole of the sole side of the foot and a portion going around the heel side from the sole side of the foot and extending to the ankle, with the portion being a portion in the backside of the ankle. In this case, the shape may be a shape in which the whole of the sole side of the foot is formed in an insole form and a swollen part of covering a portion going around the heel side and extending to the ankle, with the portion being a portion in the backside of the ankle are continued. The substrate and the covering material are easily deformed so as to fix to the belly of the heel corresponding to the belly of the heel.

Here, in the case where the substrate and the covering material are extensible and/or stretchable, it is possible to much more enhance the fitness such that the substrate and the covering material are well fitting to the complicated uneven shape of the foot by, for example, making them partial extend corresponding to the bully of the heel, thereby well fitting to the bully of the heel.

Examples of the shape of covering a part or the whole of the sole side of the foot, a part or the whole of the lateral side of the foot and a part of the instep side include a shape of covering a portion going around the toe from the whole of the sole side of the foot or the bottom side of foot fingers and extending to the instep side of foot finger and a shape of slit-toe socks and/or a shape of socks.

Here, examples of the shape of covering a portion going around the toe from the whole of the sole side of the foot or the bottom side of foot fingers and extending to the instep side of foot finger include a shape in which a swollen part of covering the toe and the finger instep side is continued to the insole side of covering the whole of the sole of the foot. In this case, though the fingers are somewhat uneven, the swollen part extends corresponding to the unevenness of the fingers and has complicated unevenness so as to cover the fingers and the toe, whereby it becomes fit to the fingertips.

Furthermore, examples of the shape of slit-toe socks and/or the shape of socks include a shape in which socks are formed in a shape in which they are continued in the center of the bottom and symmetrically divided right and left, after laminating, this laminate is folded in the center of the bottom, and an end edge going from the toe through the instep and extending to the ankle and an end edge extending from the heel to the ankle are welded; and a shape in which socks are formed in a shape in which a portion of from the ankle to the toe of a sock is divided right and left, the divided parts are continued in the center of the bottom, and a swollen part of covering from the heel to the ankle in the backside is continued in the center in the backend part, and after laminating, the both side edges of the swollen part and the back end edge of a portion from the ankle to the toe are welded, and the both side edges of the portion from the ankle to the toe are also welded.

According to the foot warming heat generating body of the invention in which the swollen part corresponding to the arch of the foot is continued, the heat generating composition is laminated in a thin-film state between the flexible substrate and covering material. Thus, the whole of the foot warming heat generating body becomes thin so that it becomes possible to easily deform the swollen part corresponding to the concave of the arch of the foot. As a result, it is possible to efficiently warm the whole of the sole of the foot while fitting to the concave of the arch of the foot.

Examples of the application of the foot warming heat generating body include a foot warming heat generating body which is directly applied to the foot, a foot warming heat generating body which is directly applied to a footwear, and a foot warming heat generating body which is stuck to socks from the outside, thereby feeding heat to the foot. In the case where the footwear is a closing footwear, examples thereof include foot warmer heat generating bodies for leather shoes, rubber shoes, fabric shoes, canvas shoes, chemical shoes, or sabot shoes.

As described previously, since the foot warming heat generating body of the invention is prepared by molding and laminating the heat generating composition on the surface of the substrate by force-through die molding or cast molding, etc. , it is possible to uniformly laminate the heat generating composition in a thin film form. It is also possible to form the heat generating composition so as to make the layer thickness partially thick and to obtain a digital compression effect in addition to the thermal effect.

That is, by further laminating the heat generating composition at least one time on a part of the upper surface of the heat generating composition as laminated on the surface of the substrate, it is possible to form a partially thick site.

In this way, by making a part of the heat generating composition thick, it is possible to control the distribution of exothermic amount and to make the heat generating composition thick in a site which is liable to become cold, such as the toe, thereby enhancing a warmth taking effect.

In this case, by forming the heat generating composition molded body as laminated on the surface of the substrate such that its thickness becomes thick in a meridian point or a section in the vicinity thereof, an indirect moxibustion or digital compression effect can be enhanced.

As a matter of course, the number of the site in which the heat generating composition is made thick is not limited to a single site, but the heat generating composition may be made thick in two or more plural sites.

Now, in general, in the foot warming heat generating body, the feed of air is poor in relation to the use state or application site. Accordingly, in designing a foot warming heat generating body, so far as the foot warming heat generating body does not cause leakage, a foot warming heat generating body in which the average pore size is relatively large, thereby improving the feed of air is desired.

An uneven part may be provided in a part of at least one member of the substrate, the underlay material, the covering material and the heat generating composition, or the heat generating composition may be provided in a concave part as provided in at least one member of the substrate and the covering material.

A foot warming heat generating body having an uneven part due to the presence of absence of the heat generating composition is useful, too.

The heat generating composition molded body may be formed so as to have a structure having at least two or more layers having a different component ratio from each other.

Furthermore, at least a part of the surface of the heat generating composition molded body may be covered by an air-permeable adhesive layer of a netlike polymer, etc., or an underlay material such as non-woven fabrics may be provided between the air-permeable adhesive layer and the covering material. In addition, a pressurizing treatment or the like may be carried out, or unevennesses may be formed on the entire surface or a part of at least one member of the heat generating composition molded body, the substrate, the covering material and the underlay material. In this way, the movement of the laminate between the substrate and the covering material may be prevented. Though the pressurizing treatment is not limited, examples thereof include a method in which after filling the heat generating composition in a die cavity, the heat generating composition in the die cavity is compressed by a compression machine such as a rubber roll or a die roll having a convex press die which comes into the die cavity; a method in which the heat generating composition molded body interposed between the substrate and the covering material is compressed by the foregoing compression machine; and a method in which after heat sealing the periphery of the heat generating composition molded body, the heat generating composition molded body is compressed by the foregoing compression machine.

A raw material of the substrate or covering material is not limited so far as it functions as an accommodating bag of the heat generating composition. Usually, raw materials which are used in chemical body warmers or heat generating bodies can be used. Examples of the raw material include air-impermeable raw materials, air-permeable raw materials, water absorptive raw materials, non-water absorptive raw materials, non-extensible raw materials, extensible raw materials, stretchable raw materials, non-stretchable raw materials, foamed raw materials, non-foamed raw materials, non-heat sealable raw materials, and heat sealable raw materials. The raw material can be properly used depending upon a desired utility in a desired form such as films, sheets, non-woven fabrics, woven fabrics, and composites thereof. In general, the substrate is made of an air-impermeable film or sheet, and the covering material is made of an air-permeable film or sheet or non-woven fabric, and vice versa. The both may be air-permeable. As the underlay material, an air-permeable underlay material and an air-impermeable underlay material may be used for different purposes.

The packaging material of the accommodating bag may be of a single-layered structure or multilayered structure, and its structure is not limited. Furthermore, though the packaging material is composed of at least a substrate and a covering material, a packaging material for laminating the heat generating composition molded body is the substrate, and a packaging material for covering on the heat generating composition molded body is the covering material regardless of whether the packaging material is air-permeable or air-impermeable. An embodiment of a multilayered structure in which an air-impermeable packaging material is the substrate and an air-permeable packaging material is the covering material will be hereunder described as one example. That is, in this embodiment, the substrate is made of layer A/layer B, layer A/layer B/layer C, or layer A/layer B/layer C/layer D; and the covering material is made of layer F/layer G, layer E/layer F/layer G, or layer F/layer H/layer G. Examples of the layer A include thermoplastic resin films (for example, polyethylene), heat seal layers (for example, polyethylene and EVA), and water absorptive papers; examples of the layer B include non-woven fabrics of a thermoplastic resin (for example, nylons), non-water absorptive papers, water absorptive papers, thermoplastic resin films (for example, polyethylene films, polypropylene films, polyester films, and polyamide (for example, nylons) films), wicks (for example, non-water absorptive papers and water absorptive papers); examples of the layer C include adhesive layers, non-water absorptive papers, water absorptive papers, thermoplastic resin films (for example, polyethylene), non-slip layers, and non-woven fabrics of a thermoplastic resin (for example, polyesters and nylons); examples of the layer D include separators, thermoplastic resin films (for example, polyethylene), and non-woven fabrics; examples of the layer E include heat seal layers; examples of the layer F include porous films or perforated films made of a thermoplastic resin (for example, polyethylene), films made of a thermoplastic resin (for example, polyethylene), non-water absorptive papers, and water absorptive papers; examples of the layer G include non-woven fabrics of a thermoplastic resin (for example, polyesters and nylons); and examples of the layer H include non-water absorptive papers and water absorptive papers. Examples of the substrate or covering material include heat seal layer made of polyethylene obtained by using a metallocene catalyst/polypropylene film, polyethylene-made heat seal layer/ polypropylene film, EVA-made heat seal layer/polypropylene film, EVA-made heat seal layer/polypropylene film/adhesive layer/separator, EVA-made heat seal layer/polyethylene film/nylon non-woven fabric, non-woven fabric/porous film, heat seal layer made of polyethylene obtained by using a metallocene catalyst/polyethylene film/nylon non-woven fabric, heat seal layer made of polyethylene obtained by using a metallocene catalyst/polypropylene film/polypropylene non-woven fabric, non-woven fabric/(paper and/or perforated (provided by a needle or laser) film)/porous film, non-woven fabric/ (paper and/or porous film) /perforated (provided by a needle or laser) film, and non-woven fabric/(paper and/or porous film)/non-woven fabric. A method for laminating the respective layers is not limited. The respective layers may be directly laminated; the respective layers may be laminated via an air-permeable adhesive layer or a laminating agent layer; and the respective layers may be laminated by hot melt extrusion or the like. Furthermore, in the invention, it is to be noted that polyethylene produced by using a metallocene catalyst is also included in the polyethylene.

For example, in the case of laminating the foregoing raw material such as non-woven fabrics and porous films via an air-permeable sticky layer, examples of a method for forming the air-permeable sticky layer include a method in which a sticky substance is fibrillated by an appropriate system such as a curtain spray system, a melt blow system or a slot spray system for blowing and spreading a sticky substance via hot air under heat melting and spread and accumulated on an appropriate supporting substrate made of a porous film, an air-permeable substrate, a separator, etc., thereby forming a porous sticky layer.

A thickness of each of the substrate, the covering material, the underlay material, and the raw material constituting the same varies depending upon the utility and is not limited. The thickness is usually from 5 to 5, 000 $\mu$m, preferably from 10 to 500 $\mu$m, and more preferably from 20 to 250 $\mu$m.

The air-impermeable raw material is not limited so far as it is air-impermeable. Examples thereof include films, sheets or coatings made of a polymer (for example, polyethylene, polypropylene, nylons, polyacrylates, polyesters, polyvinyl alcohols, and ethylene-vinyl acetate copolymers) and laminates thereof with a metal (including a semiconductor) compound (for example, silicon oxide) or composite raw materials using the same.

Of the foregoing air-impermeable raw materials, examples of a film having high air impermeability include films provided with a single layer or multiple layers of a thin film having a metal including a semiconductor or a compound thereof provided on an air-impermeable raw material film. Examples of the metal including a semiconductor include silicon, aluminum, and alloys or mixtures containing such a metal. Examples of the metal (including a semiconductor) compound include oxides, nitrides and oxynitrides of the foregoing metals or alloys or mixtures. Examples of the layer include silicon oxide layers, aluminum oxide layers, and silicon oxynitride layers; layers obtained by laminating an arbitrary layer of these layers on a polyester-made film; and layers obtained by further laminating a stretched polyolefin film (for example, a biaxially stretched polypropylene film) thereon.

The air-permeable raw material is not limited so far as it is air-permeable. Examples thereof include air-permeable films (for example, porous films and perforated films); materials having air permeability by themselves (for example, papers and non-woven fabrics); materials prepared by laminating at least one of papers and air-permeable films and non-woven fabrics so as to have air permeability; materials prepared by providing an air-impermeable packaging material comprising a non-woven fabric having a polyethylene film laminated thereon with fine pores by using a needle, etc. so as to have air permeability; non-woven fabric whose air permeability is controlled by laminating a fiber and heat bonding under pressure; porous films; and materials prepared by sticking a non-woven fabric onto a porous film. The "perforated film" as referred to herein is a film prepared by providing an air-impermeable film (for example, polyethylene films) with fine pores by using a needle so as to have air permeability.

The air permeability is not limited so far as the heat generation can be kept. In the case of use in usual heat generation, the air permeability is usually from 50 to 10,000 $g/m^2/24$ hr, preferably from 70 to 5,000 $g/m^2/24$ hr, more preferably from 100 to 2, 000 $g/m^2/24$ hr, and further preferably from 100 to 700 $g/m^2/24$ hr in terms of moisture permeability by the Lyssy method.

When the moisture permeability is less 50 g/m$^2$/24 hr, the heat value is small and a sufficient thermal effect is not obtained, and therefore, such is not preferable. On the other hand, when it exceeds 10,000 g/m$^2$/24 hr, the exothermic temperature is high so that a problem in safety may possibly be generated, and therefore, such is not preferable. However, there is no limitation even when the moisture permeability exceeds 10,000 g/m$^2$/24 hr depending upon the utility, or even in the use at a moisture permeability closed to the open system, according to circumstances.

The stretchable packaging material is not particularly limited so far as it is stretchable. That is, it is only required that the stretchable packaging material is stretchable as a whole. The stretchable packaging material may be formed of a single material or a composite material of stretchable substrates or a combination of a stretchable substrate and a non-stretchable substrate.

Examples of the stretchable packaging material include single materials (for example, natural rubbers, regenerated rubbers, synthetic rubbers, elastomers, and stretchable shape memory polymers) and mixtures thereof, mixed materials or blended materials of such a stretchable raw material and a non-stretchable raw material or fabrics constituted of a combination of these materials, films, yarns, strands, ribbons, tapes, and stretchable films with a scrim structure.

The porous film is not limited and can be properly selected among porous films obtained by stretching a film made of a polyolefin based resin (for example, polyethylene, linear low density polyethylene, and polypropylene) or a fluorine based resin (for example, polytetrafluoroethylene) and a filler.

The non-woven fabric is not limited. Single non-woven fabrics of a single fiber or composite fiber made of a material such as rayon, nylons (polyamides), polyesters, polyacrylates, polypropylene, vinylon, polyethylene, polyurethane, cupra, cotton, cellulose, and pulp, or laminates of blended or accumulated fiber layers of such fibers are useful. Furthermore, from the standpoint of production process, dry non-woven fabrics, wet non-woven fabrics, spunbonds, spunlaces, and the like can be used. Non-woven fabrics made of a composite fiber having a core-sheath structure are also useful. A non-woven fabric in the side which is brought into contact with the skin is preferably a napping (fluffy) non-woven fabric. Also, stretchable non-woven fabrics and non-stretchable non-woven fabrics are useful.

The water absorptive raw material is not particularly limited so far as it is a water absorptive film or sheet.

The water absorptive raw material is not particularly limited so far as it has water absorption properties consequently regardless of whether or not the raw material has water absorption properties by itself.

Specific examples thereof include water absorptive foamed films or sheets having water absorption properties (for example, foamed bodies of water absorptive foamed polyurethane, etc.) or papers, non-woven fabrics or woven fabrics formed of a fiber having water absorption properties, non-woven fabrics or woven fabrics containing a fiber having water absorption properties, and water absorptive materials such as water absorptive porous films or sheets. Besides, there are enumerated materials in which regardless of the presence or absence of water absorption properties, a water absorbing agent is contained, impregnated, kneaded, transferred or carried on a foamed film or sheet, a non-woven fabric, a woven fabric or porous film or sheet, thereby imparting or increasing water absorption properties; and materials in which regardless of the presence or absence of water absorption properties, a water absorptive raw material such as water absorptive foamed films or sheets, papers, non-woven fabrics, woven fabrics, and porous films or sheets as cut in a planar shape according to the invention is attached to one side or both sides of the material according to the invention, thereby imparting water absorption properties.

In particular, in the heat generating body of the invention, for the purpose of forming the plane which is brought into contact with the skin into a comfortable plane by imparting water absorption properties against sweat, etc., in order that in the case of sweating, the sweat is absorbed, it is preferable that a packaging material in the plane which is brought into contact with the skin is constituted of a packaging material using a non-woven fabric or a woven fabric containing, as the major component, a water absorptive fiber having a water retention of 20 % or more. Examples of the water absorptive fiber having a water retention of 20 % or more include cottons, silks, hemps, wools, polyacrylonitrile based synthetic fibers, polyamide based synthetic fibers, polyvinyl alcohol based synthetic fibers, acetate fibers, triacetate fibers, and regenerated fibers. In addition, non-woven fabrics having a highly water absorptive polymer held in a non-woven fabric can be used as the non-woven fabric having excellent water absorption properties. Incidentally, non-woven fabrics or woven fabrics containing such a fiber as the major component are relatively good with respect to the feeling against the skin.

In addition, highly water absorptive packaging materials having high absorption properties of sweat can be used as the packaging material. Examples thereof include non-woven fabrics containing a fiber whose surface is coated with a highly water absorptive resin, non-woven fabrics containing a hollow fiber having a number of fine pores on the surface thereof, and non-woven fabrics containing a fiber having a capillary action by forming a number of pouches or plural layers in the cross-sectional shape.

Besides, non-woven fabrics or films having a water absorptive inorganic compound held on a non-sticky surface of a packaging material can be used. Examples thereof include non-woven fabrics resulting from holding a powder (for example, diatomaceous earth, zeolite, and silica gel) on a non-woven fabric and films resulting from holding a relatively large amount of a powder (for example, silica and alumina) on a synthetic resin (for example, polyethylene).

Furthermore, films or sheets made of a polymer such as the foregoing polyethylene (polyethylene as produced by using a metallocene-containing catalyst), polyvinyl chloride, and polyvinylidene chloride or coated materials thereof, or films or sheets resulting from a processing treatment such as embossing of the foregoing materials may be provided as a non-slip layer in at least a part of the exposed part of the foot warming heat generating body.

The core material is not limited so far as it functions as a core material. Examples thereof include films or sheets made of a polymer such as polyethylene, polypropylene, nylons, polyacrylates, polyesters, polyvinyl alcohol, and ethylene-vinyl acetate copolymers; and papers such as thin papers such as crepe paper and kraft paper and thick papers such as corrugated cardboard liner papers, corrugated cardboard cores, and coat poles, or laminates containing one or two or more kinds thereof, and composite raw materials using them.

In the case of carrying out the temporary adhesion by contact bond sealing, a bonding layer constituted of an adhesive is provided in at least one of the substrate or the covering material, the substrate and the covering material are gathered, and the substrate and the covering material are temporarily adhered to each other via the bonding layer by pressurizing, thereby forming a temporary adhering part. Furthermore, a heat seal part may be constituted of only a heat sealed region by after forming a heat seal part having a width narrower than the temporary adhering part, moving at least a part of the accommodated heat generating composition molded body into the temporary adhering part, thereby deadhering the temporary adhering part.

Furthermore, in order to prevent oozing of the aqueous solution from the heat generating composition laminate into the covering material and/or a body to be warmed, an underlay material made of a perforated film or sheet, an air-permeable adhesive layer made of a netlike polymer, etc., a non-woven fabric made of polyethylene, etc., or the like may be provided between the heat generating composition laminate and the air-permeable covering material.

Now, the foregoing heat warming heat generating body is a heat warming heat generating body composed of a single exothermic part which is made of a heat generating composition laminate as provided by using the foregoing heat generating composition and molding it between packaging materials which constitute an accommodating bag by force-through die molding or cast molding or the like, or a heat warming heat generating body in which a gathered exothermic part is formed by gathering of two or more plural sectional exothermic parts as disposed at intervals and fixed.

The foot warming heat generating body of the invention is a foot warming heat generating body of a lamination system resulting from laminating and molding the heat generating composition on the substrate, putting the covering material thereon and sealing the surroundings of the heat generating composition laminate.

In the sectional exothermic part or the heat generating composition molded body of the invention, its maximum width is usually from 0.5 to 60 mm, preferably from 0.5 to 50 mm, more preferably from 1 to 50 mm, further preferably from 3 to 50 mm, still further preferably 3 to 30 mm, even further preferably from 5 to 20 mm, even still further preferably from 5 to 15 mm, and most preferably from 5 to 10 mm. Furthermore, its maximum height is usually from 0.1 to 30 mm, preferably from 0.1 to 10 mm, more preferably from 0.3 to 10 mm, further preferably from 1 to 10 mm, and still further preferably from 2 to 10 mm. Moreover, its longest length is usually from 5 to 300 mm, preferably from 5 to 200 mm, more preferably from 5 to 100 mm, further preferably from 20 to 150 mm, and still further preferably from 30 to 100 mm.
A capacity of the sectional exothermic part or a volume of the heat generating composition molded body is usually from 0. 015 to 500 cm$^3$, preferably from 0.04 to 30 cm$^3$, more preferably from 0.1 to 30 cm$^3$, further preferably from 1 to 30 cm$^3$, and still further preferably from 3 to 20 cm$^3$.
In the sectional exothermic part, when the sectional exothermic part which is an accommodating region of the heat generating composition is filled with the heat generating composition molded body, a volume ratio of the volume of the heat generating composition molded body which is an occupying region of the heat generating composition molded body to the capacity of the sectional exothermic part which is an accommodating region of the heat generating composition is usually from 0. 6 to 1, preferably from 0. 7 to 1, more preferably from 0.8 to 1, and further preferably from 0.9 to 1.0.
Furthermore, a width of the sectioned part which is a space between the sectional exothermic parts is not limited so far as sectioning can be achieved. It is usually from 0.1 to 50 mm, preferably from 0.3 to 50 mm, more preferably from 0.3 to 50 mm, further preferably from 0.3 to 40 mm, still further preferably from 0.5 to 30 mm, even further preferably from 1.0 to 20 mm, and even still further preferably from 3 to 10 mm.
Incidentally, the heat generating composition molded body or the sectional exothermic part may have any shape. The shape may be a planar shape, and examples thereof include a circular shape, an elliptical shape, a polygonal shape, a star shape, and a flower shape. Also, the shape may be a three-dimensional shape, and examples thereof include a polygonal pyramidal shape, a conical shape, a frustum shape, a spherical shape, a parallelepiped shape, a cylindrical shape, a semi-pillar shape, a semicylindroid shape, a semicylidrical shape, a pillar shape, and a cylindroid shape. Furthermore, in these shapes, the corner may be rounded, thereby processing the corner in a curvilinear or curved state, or the central part may be provided with a concave.
Furthermore, the "volume of the heat generating composition molded body of the invention" as referred to herein means a volume of the heat generating composition molded body or compressed heat generating composition molded body.
Furthermore, the "capacity of the sectional exothermic part" as referred to herein means an internal capacity of the sectional exothermic part having a heat generating composition molded body accommodated therein.

If desired, a cutting line such as a perforation can be provided in the sectioned part. This perforation may be provided to a degree that the bending properties are improved or to a degree that cutting by hand is possible, such as a degree that a foot warming heat generating body can be molded in a size adaptive to the location of application, etc. of a human body. Such a degree is not limited and may be determined depending upon the desire.

The "perforation" as referred to in the invention includes one which is intermittently cut for the purpose of improving flexural properties of the sectioned part and one which is intermittently cut such that cutting by hand is possible. Its degree, length and aperture are not limited but are determined depending upon the desire. The perforation may be provided in all sectioned parts or may be partially provided.

The shape is not particularly limited, and examples thereof include a circle, an ellipse, a rectangle, a square, and a cut line (linear shape). For example, in the perforation which is intermittently cut such that cutting by hand is possible, a circular hole having an aperture of from $\phi 10$ to 1,200 $\mu$m can be enumerated. The aperture of the hole is more preferably from $\phi 20$ to 500 $\mu$m.

It is preferable that the holes are positioned lined up in the length and width. Furthermore, a shortest space between outer peripheries of the adjacent holes in the length and width is not limited so far as it is satisfactory with flexural properties and possibility of cutting by hand. The shortest space is preferably from 10 to 2,000 $\mu$m, more preferably from 10 to 1,500 $\mu$m, further preferably from 20 to 1,000 $\mu$m, still further preferably from 20 to 500 $\mu$m, and even further preferably from 20 to 200 $\mu$m. The cutting properties by hand are remarkably improved by a balance between the aperture of the hole and the shortest space of outer peripheries of the adjacent holes in the length and width.

The hole may be a cut line, and its length may be a length corresponding to the aperture or may be larger than the aperture. A shortest space between ends of the adjacent cut lines in the length and width is corresponding to the shortest space between outer peripheries of the adjacent holes.

For example, an aperture of the hole of from $\phi 10$ to 2,000 $\mu$m is corresponding to a length of from 10 to 2,000 $\mu$m, and a shortest space between outer peripheries of the adjacent holes in the length and width of from 10 to 2,000 $\mu$m is corresponding to a shortest space between ends of the adjacent cut lines in the length and width of from 10 to 2,000 $\mu$m.

In the case of a cut line, since it is long in one direction, its length can be prolonged and may be from 10 to 50,000 $\mu$m. A shortest space between the adjacent cut lines in the length and width may be from 1 to 5,000 $\mu$m.

In the exothermic part, a magnetic substance may be contained in a part of the exothermic part or a single sectional exothermic part.

A non-slip layer or an adhesive layer may be provided in at least a part of the exposed surface of the heat warming heat generating body. In addition, for the purpose of protection until the use, a separator can also be superposed on the non-slip layer. By providing a cut such as a split in the separator, it may be made easy to achieve peeling.

The thickness of the non-slip layer is not limited but is preferably from 5 to 1,000 $\mu$m, more preferably from 10 to 500 $\mu$m, and further preferably from 15 to 250 $\mu$m.

The non-slip layer is not limited so far as it has a non-slip effect. Examples of the constitution of the non-slip layer include a method of providing a non-slip agent such as adhesives and weakly sticky substances or a non-slip material such as a hook and loop fastener called Magic Tape or Velcro Fastener, a film, a sheet, and an expanded sheet by means of, coating, sticking, lamination, etc. in the foot warming heat generating body. The shape, the number, the setting-up region and the setting-up place are not limited, and the non-slip layer may be provided entirely or partially on one surface of the foot warming heat generating body. A single non-slip layer or plural non-slip layers may be provided in a shape of every kind such as a circular form, a rectangular form, a netlike form, a stripped form, and a dot-like form. Furthermore, the non-slip layer may be provided on the whole of one surface in a shape adaptive to the foot warming heat generating body. For example, the non-slip layers may be disposed in a small parallel piece form continuously extending from the upper edge to the lower edge among the plural sectional exothermic parts.

For example, the non-slip agent may be provided on the entire surface on the air-permeable part of the foot warming heat generating body; a porous resin formed by fibrillating the non-slip agent may be provided on the foot warming heat generating body; a porous weakly sticky layer on a separator may be transferred and fixed onto the surface of the foot; or the non-slip agent may be provided directly on the surface of the foot warming heat generating body in a cobweb state or a linear or circular state by a melt blow method. Furthermore, an expanded polyurethane or a film of a polyolefin based resin resulting from polymerization using a metallocene catalyst may be laminated; a polyolefin based resin is laminated plainly or in a pattern-like form or printed; or a granular weakly sticky substance or a soft vinyl chloride resin may be uniformly dispersed and fixed upon heating, thereby forming a number of protrusions. Furthermore, a non-slip agent resulting from spraying a solution or dispersion of an elastomer or a plastisol in a stripped form or polka-dotted form can be used. The non-slip layer may be provided entirely or partially on the foot warming heat generating body within the range where the function as the foot warming heat generating body is not hindered. In addition, the adhesiveness between the non-slip agent and the substrate may be improved by applying heat or pressure by using a roll, etc. Furthermore, as the non-slip agent, all of hot melt based, solvent based and emulsion based non-slip agents can be used. Furthermore, materials to which a film, a sheet or an expanded sheet containing the same is stuck may be used.

Examples of the weakly sticky substance include styrene based elastomers such as SIS, SBS, SEBS, and SIPS; acrylic elastomers containing, as a component, an acrylic acid based or methacrylic acid based alkyl ester; olefin based elastomers; and urethane based elastomers. Examples of other non-slip agents include mixtures of a capsule containing an expanding agent (a volatile solvent such as isobutane) in a core of a resin such as a soft vinyl chloride based resin, a natural rubber, a styrene-butadiene rubber, a urethane rubber, and an ethylene-vinyl acetate copolymer and a binder resin. In addition, the tackiness may be adjusted by mixing a tackifier such as petroleum resins.

Examples of the adhesive which constitutes the adhesive layer include acrylic adhesives, vinyl acetate based adhesives (for example, vinyl acetate resin based emulsions and ethylene-vinyl acetate resin based hot melt adhesives), polyvinyl alcohol based adhesives, polyvinyl acetal based adhesives, vinyl chloride based adhesives, polyamide based adhesives, polyethylene based adhesives, cellulose based adhesives, chloroprene (neoprene) based adhesives, nitrile rubber based adhesives, polysulfide based adhesives, butyl rubber based adhesives, silicone rubber based adhesives, styrene based adhesives (for example, styrene based hot melt adhesives), rubber based adhesives, and silicone based adhesives. Of these, for the reasons that the adhesive strength is high, that the cost is cheap, that the long-term stability is good, and that even by applying warmth, a lowering of the adhesive strength is small, rubber based adhesives, acrylic adhesives and adhesives containing a hot melt based high-molecular substance are desired.

Examples of the hot melt based adhesive include known hot melt based adhesives imparted with adhesion. Specific examples thereof include styrene based adhesives made of, as a base polymer, an A-B-A type block copolymer (for example, SIS, SBS, SEBS, and SIPS), vinyl chloride based adhesives made of, as a base polymer, a vinyl chloride resin, polyester based adhesives made of, as a base polymer, a polyester, polyamide based adhesives made of, as a base polymer, a polyamide, acrylic adhesives made of, as a base polymer, an acrylic resin, polyolefin based adhesives made of, as a base polymer, a polyolefin (for example, polyethylene, super low density polyethylene, polypropylene, ethylene-$\alpha$-olefin copolymers, and ethylene-vinyl acetate copolymers), 1,2-polybutadiene based adhesives made of, as a base polymer, 1, 2-polybutadiene, and polyurethane based adhesives made of, as a base polymer, polyurethane; adhesives made of a modified body of the foregoing adhesive whose adhesion is improved or whose stability is changed; and mixtures of two or more kinds of these adhesives. Adhesive layers constituted of an expanded adhesive and adhesive layers constituted of a crosslinked adhesive can also be employed.

The non-aromatic hot melt based adhesive is not limited so far as it is made of, as a base polymer, a hot melt based adhesive not containing an aromatic ring. Examples thereof include olefin based hot melt based adhesives and acrylic hot melt based adhesives. As the non-aromatic polymer which is the base polymer not containing an aromatic ring, there are enumerated polymers or copolymers of an olefin or a diene. Examples thereof include olefin polymers. The olefin polymer includes polymers or copolymers of ethylene or an $\alpha$-olefin. Also, polymers resulting from adding a diene (for example, butadiene and isoprene) as other monomer thereto may be employed.

The $\alpha$-olefin is not limited so far as it is a monomer having a double bond in the terminal thereof. Examples thereof include propylene, butene, heptene, hexene, and octene.

The "aromatic hot melt based adhesive" as referred to herein is a hot melt based adhesive whose base polymer contains an aromatic ring. Examples thereof include styrene based hot melt based adhesives represented by A-B-A type block copolymers.

In the foregoing A-B-A type block copolymers, the A block is a non-elastic polymer block made of a monovinyl substituted aromatic compound A such as styrene and methylstyrene; and the B block is an elastic polymer block made of a conjugated diene such as butadiene and isoprene. Specific examples thereof include a styrene-butadiene-styrene block copolymer (SBS), a styrene-isoprene-styrene block copolymer (SIS), and hydrogenated types thereof (for example, SEBS and SIPS), and mixtures thereof.

The bonding layer for fixing the air permeability adjusting material is constituted of a bonding agent or an adhesive which is usually used. In particular, an adhesive is useful, and the foregoing adhesives for constituting the adhesive layer can be used.

Furthermore, a method for providing a bonding layer is not limited so far as the air permeability adjusting material can be fixed. The bonding layer may be entirely provided or partially or intermittently provided. Examples of its shape include various shapes such as a netlike shape, a stripe-like shape, a dot-like shape, and a band-like shape.

In the case where the exothermic part of the foot warming heat generating body is constituted of sectional exothermic parts and sectioned parts, an air permeability adjusting material may be provided so as to cover the exothermic part.

The "air permeability adjusting material" as referred to herein is a packaging material for providing a spacial part in the surroundings of the sectional exothermic part communicating with the outside by covering a vertical part between the sectional exothermic part and the sectioned part as provided alternately utilizing a difference of altitude. The spacial part communicates with the outside through an air hole to which the air permeability adjusting material is related. Welding of the air permeability adjusting material and the sectional exothermic part or the sectioned part is carried out via a bonding layer. Furthermore, the air permeability of the air permeability adjusting material is lower than that of the air-permeable surface of the exothermic part or may be impermeable to air.

The raw material of the air permeability adjusting material is not limited so far as it has lower air permeability than that of the air-permeable surface of the exothermic part. Examples thereof include non-woven fabrics, thermoplastic synthetic resin films, thermoplastic synthetic resin films having a metallic thin film, air-impermeable multilayered structures made of a laminate of a non-woven fabric and the foregoing thermoplastic synthetic resin films, synthetic resin expanded bodies, and multilayered structures containing the same.

The bonding layer for fixing the air permeability adjusting material to a raw material in a region which is brought into direct contact with the heat generating composition is not limited so far as it can achieve fixing. Examples of a material constituting the bonding layer include an adhesive, a heat seal material, and a bonding agent.

The fixing region between the air permeability adjusting material and the exothermic part is not limited so far as the both can be fixed and air can go in and out from at least the periphery of the sectional exothermic part. However, the following can be enumerated.

1) The fixing region is fixed in the both ends of the exothermic part or heat generating body.
2) A space is provided entirely in a substantially central part of the exothermic part, and other exothermic part region is defined as the fixing region.
3) A substantially top part of each sectional exothermic part and a substantially central part of each sectioned part are defined as the fixing region.

Here, any material can be used as the air permeability adjusting material so far as its air permeability does not exceed an air permeability of the air-permeable raw material. Examples of an air permeability adjusting material having a bonding layer and utilizing a plastic film include PE/adhesive, PP/adhesive, polyester/adhesive, PE/non-woven fabric/air-permeable adhesive, PE/non-woven fabric/PE/adhesive, PE/PET/M/PE/non-woven fabric/air-permeable adhesive, PE/heat seal material, PE/non-woven fabric/heat seal material, PE/non-woven fabric/PE/heat seal material, and PE/polyester/M/PE/non-woven fabric/heat seal material. Here, M represents a metal (for example, aluminum and silver), a semiconductor (for example, silicon oxide, silicon oxynitride, silicon nitride, and aluminum oxide), or a metal oxide, oxynitride or nitride. Furthermore, a portion for placing fixing means such as an adhesive layer and a heat sealing agent layer is not limited, and whether it is provided partially or entirely may be properly determined depending upon the intended purpose.

The bonding substance which constitutes the bonding layer is not limited so far as the air permeability adjusting material can be fixed to the foot warming heat generating body, and examples thereof include an adhesive.

The raw material which constitutes the accommodating bag and the heat seal material and adhesive can be used as the air permeability adjusting material, the raw material which constitutes the bonding layer, the heat seal material, or the adhesive.

The air permeability adjusting material is not limited so far as it has lower air permeability than a material which directly contact with the heat generating composition. In general, the moisture permeability according to the Lyssy method of the air-permeability raw material is preferably not more than 50 $g/m^2/24$ hr, more preferably not more than 10 $g/m^2/24$ hr, further preferably not more than 2 $g/m^2/24$ hr, and still further preferably not more than 1 $g/m^2/24$ hr, and raw materials which are usually called as an air-impermeable raw material can also be used. Examples thereof include films, sheets, foamed bodies, non-woven fabrics, woven fabrics, and molded bodies made of an arbitrary combination thereof. Other examples include thermoplastic synthetic resin films, thermoplastic synthetic resin films having a metal thin film, thermoplastic synthetic resin films having a metal compound thin film, air-impermeable laminated structures made of a laminate of a non-woven fabric and the foregoing thermoplastic synthetic resin film, synthetic resin foamed bodies, gas cushioning bodies, and multilayered structures containing the same. In order to effectively achieve heat insulation of the air-permeable layer constituted of an air-permeable adjusting material, thermoplastic synthetic resin films having a metal thin film, gas cushioning bodies, and multilayered structures containing the same are preferable. The raw materials which are used in the foregoing substrate and covering material can be used.

So far as the foot warming heat generating body exclusive of the air permeability adjusting material is a foot warming heat generating body which is made of a sectional exothermic part for accommodating the heat generating composition and a sectioned division as a seal part and which has a difference of altitude, the heat generating composition and the accommodating bag and the raw material constituting it are not limited. However, a foot warming heat generating body in which a heat generating composition molded body as produced from a moldable heat generating composition containing surplus water as a connecting substance by the molding system is accommodated in an air-permeable accommodating bag is preferable.

This adhesive layer is not particularly limited so far as it can be directly adhered to an adherend such as the body surface, clothing, and shoes. Examples thereof include layers formed of an adhesive of every kind.

At least a part of the substrate, the covering material, the air permeability adjusting material, the non-slip layer, the adhesive layer and the separator which constitute the foot warming heat generating body may be provided with at least one kind of characters, designs, symbols, numerals, patterns, photographs, pictures, and colored parts.

Each of the substrate, the covering material, the air permeability adjusting material, the adhesive layer, the non-slip layer and the separator which constitute the foot warming heat generating body may be transparent, opaque, colored, or colorless. Furthermore, a layer constituting at least one layer of the layers constituting the respective materials and layers may be colored to a color different from those of other layers.

For the storage or transportation or the like, the foot warming heat generating body may be sealed and accommodated in an outer bag which is an air-impermeable accommodating bag.

The outer bag is not limited so far as it is air-impermeable and may be made of a laminate. Examples thereof include nylon, polyester and polypropylene films which are subjected to a moisture-proof treatment with OPP, CPP, polyvinylidene chloride, metal oxides such as aluminum oxide and silicon oxide, etc., aluminum foils, and aluminum-deposited plastic films. As one example thereof, there is enumerated a foot warming heat generating body in which the produced foot warming heat generating body is mediated between two air-impermeable films or sheets; simultaneously with or after this mediation, the two films or sheets are punched out into a size of equal to or larger than the foot warming heat generating body; and simultaneously with or after this punching-out, the two films or sheets are sealed in the periphery of the foot warming heat generating body.

The heat generating composition is not limited so far as it is a heat generating composition which contains, as essential components, an iron powder, a carbon component, a reaction accelerator and water, contains surplus water so as to have a water mobility value of from 0.01 to 20, has moldability due to the surplus water, with the water in the heat generating composition not functioning as a barrier layer, and is capable of causing an exothermic reaction upon contact with air.

In addition, if desired, at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof may be further added to the heat generating composition.

Furthermore, in the heat generating composition of the invention or the like, although there is no particular limitation for the compounding ratio thereof, it is preferred to select the compounding ratio such that the amount of the reaction accelerator is from 1.0 to 50 parts by weight, the amount of water is from 1.0 to 60 parts by weight, the amount of the carbon component is from 1.0 to 50 parts by weight, the amount of the water retaining agent is from 0.01 to 10 parts by weight, the water absorptive polymer is from 0. 01 to 20 parts by weight, the amount of the pH adjusting agent is from 0.01 to 5 parts by weight, and the amount of the hydrogen formation inhibitor is from 0.01 to 12 parts by weight, respectively based on 100 parts by weight of the iron powder; and that the heat generating composition has a water mobility value of from 0.01 to 20.

In addition, the following components may be added in compounding ratios as described below to the iron powder to the heat generating composition. That is, the amount of the metal other than iron is from 1.0 to 50 parts by weight, the amount of the metal oxide other than iron oxide is from 1.0 to 50 parts by weight, the amount of the surfactant is from 0.01 to 5 parts by weight, the amount of each of the hydrophobic polymer compound, the aggregate, the fibrous material, the functional substance, the organosilicon compound and the pyroelectric substance is from 0.01 to 10 parts by weight, the amount of each of the moisturizer, the fertilizer component and the heat generating aid is from 0.01 to 10 parts by weight, and the amount of the acidic substance is from 0.01 to 1 part by weight based on 100 parts by weight of the iron powder. Incidentally, a magnetic material may further be compounded, and its compounding ratio may be properly determined depending upon the desire.

Incidentally, these compounding ratios can also be applied in a reaction mixture and a heat generating mixture. Furthermore, a water mobility value of the reaction mixture is usually less than 0.01.

As the water, one from a proper source may be employed. Its purity and kind and the like are not particularly limited.

In the case of the heat generating composition, the content of water is preferably from 1 to 70 % by weight, more preferably from 1 to 60 % by weight, further preferably from 7 to 60 % by weight, still further preferably from 10 to 50 % by weight, and even further preferably from 20 to 50 % by weight of the heat generating composition.

Furthermore, in the case of the reaction mixture or heat generating mixture prior to the contact treatment with an oxidizing gas, the content of water is preferably from 0.5 to 20 % by weight, more preferably from 1 to 20 % by weight, further preferably from 3 to 20 % by weight, and still further preferably from 4 to 15 % by weight of the reaction mixture or heat generating mixture.

The carbon component is not particularly limited so far as it contains carbon as a component. Examples thereof include carbon black, graphite, active carbon, carbon nanotubes, carbon nanohorns, and flullerenes. Carbon which has become conductive by doping or the like is also employable. There are enumerated active carbons as prepared from coconut shell, wood, charcoal, coal, bone carbon, etc. and carbons as prepared from other raw materials such as animal products, natural gases, fats, oils, and resins. In particular, active carbons having an adsorption retaining ability are preferable.

Furthermore, it is not always required that the carbon component is present alone. In the case where an iron powder containing the carbon component and/or covered by the carbon component is used in the heat generating composition, it is to be noted that the heat generating composition contains the carbon component even though the carbon component is not present alone.

The reaction accelerator is not particularly limited so far as it is able to promote the reaction of the heat generating substance. Examples thereof include metal halides, nitrates, acetates, carbonates, and metal sulfates. Examples of metal halides include sodium chloride, potassium chloride, magnetic chloride, calcium chloride, ferrous chloride, ferric chloride, sodium bromide, potassium bromide, ferrous bromide, ferric bromide, sodium iodide, and potassium iodide. Examples of nitrates include sodium nitrate and potassium nitrate. Examples of acetates include sodium acetate. Examples of carbonates include ferrous carbonate. Examples of metal sulfates include potassium sulfate, sodium sulfate, and ferrous sulfate.

The water retaining agent is not limited so far as it is able to retain water. Examples thereof include porous materials derived from plants having high capillary function and hydrophilicity such as wood meal, pulp powder, active carbon, saw dust, cotton cloth having a number of cotton fluffs, short fiber of cotton, paper dust, and vegetable materials, water-containing magnesium silicate based clay minerals such as active clay and zeolite, pearlite, vermiculite, silica based porous substances, coralline stone, and volcanic ash based substances (for example, terraballoon, shirasu balloon, and *taisetsu* balloon). In order to increase a water retaining ability and enhance a shape holding ability of such a water retaining agent, the water retaining agent may be subjected to a processing treatment such as baking and/or pulverization. The water absorptive polymer is not particularly limited so far as it is a resin having a crosslinking structure and having a water absorption magnification of ion-exchanged water of 3 times or more of the dead weight. Furthermore, a water absorptive polymer the surface of which is crosslinked may be employed. Conventionally known water absorptive polymers and commercial products may also be employed.

Examples of the water absorptive polymer include poly(meth)acrylic acid crosslinked materials, poly(meth)-acrylic acid salt crosslinked materials, sulfonic group-containing poly(meth)acrylic ester crosslinked materials, polyoxyalkylene group-containing poly(meth)acrylic ester crosslinked materials, poly(meth)acrylamide crosslinked materials, crosslinked materials of a copolymer of a (meth)acrylic acid salt and a (meth)acrylamide, crosslinked materials of a copolymer of a hydroxyalkyl (meth) acrylate and a (meth)acrylic acid salt, polydioxolane crosslinked materials, crosslinked polyethylene oxide, crosslinked polyvinylpyrrolidone, sulfonated polystyrene crosslinked materials, crosslinked polyvinylpyridine, saponification products of a starch-poly (meth) acrylonitrile graft copolymer, starch-poly(meth)acrylic acid (salt) graft crosslinked copolymers, reaction products of polyvinyl alcohol and maleic anhydride (salt), crosslinked polyvinyl alcohol sulfonic acid salts, polyvinyl alcohol-acrylic acid graft copolymers, and polyisobutylene maleic acid (salt) crosslinked polymers. These water absorptive polymers may be used alone or in combination with two or more kinds thereof.

**[0073]** [0010] Of these water absorptive polymers, water absorptive polymers having biodegradation properties are not limited so far as they are a biodegradable water absorptive polymer. Examples thereof include polyethylene oxide crosslinked materials, polyvinyl alcohol crosslinked materials, carboxymethyl cellulose crosslinked materials, alginic acid crosslinked materials, starch crosslinked materials, polyamino acid crosslinked materials, and polylactic acid crosslinked materials.

The pH adjusting agent is not limited so far it is able to adjust the pH. Examples thereof include alkali metal weak acid salts and hydroxides and alkaline earth metal weak acid salts and hydroxides such as $Na_2CO_3$, $NaHCO_3$, $Na_3PO_4$, $Na_2HPO_4$, $Na_5P_3O_{10}$, NaOH, KOH, $Ca(OH)_2$, $Mg(OH)_2$, and $Ca_3(PO_4)_2$.

The hydrogen formation inhibitor is not limited so far as it is able to inhibit the formation of hydrogen. Examples thereof include one member or two or more members selected from the group consisting of sulfur compounds, oxidizing agents, alkaline substances, sulfur, antimony, selenium, phosphorus, and tellurium. Incidentally, examples of sulfur compounds include compounds with an alkali metal or an alkaline earth metal, metal sulfides such as calcium sulfide, metal sulfites such as sodium sulfite, and metal thiosulfates such as sodium thiosulfate.

**[0074]** [0013] Examples of the oxidizing agent include nitrates, oxides, peroxides, halogenated oxygen acid salts, permanganates, and chromates.

The aggregate is not limited so far as it is useful as a filler and/or is useful for making the heat generating composition porous. Examples thereof include fossilized coral (for example, coral fossil and weathered coral fossil), bamboo charcoal, *bincho* charcoal, silica-alumina powders, silica-magnesia powders, kaolin, crystalline cellulose, colloidal silica, pumice, silica gel, silica powders, mica powders, clays, talc, synthetic resin powders or pellets, foamed synthetic resins such as foamed polyesters or polyurethanes, diatomaceous earth, alumina, and cellulose powder. Incidentally, it is to be noted that kaolin and crystalline cellulose are not contained in the heat generating composition of the invention.

The fibrous material is an inorganic fibrous material and/or an organic fibrous material. Examples thereof include rock wool, glass fibers, carbon fibers, metal fibers, pulps, papers, non-woven fabrics, woven fabrics, natural fibers such as cotton and hemp, regenerated fibers such as rayon, semi-synthetic fibers such as acetates, synthetic fibers, and pulverized products thereof.

The functional substance is not limited so far as it is a substance having any function. Examples thereof include at least one member selected from minus ion emitting substances and far infrared ray radiating substances. The minus ion emitting substance is not limited so far as it emits a minus ion as a result either directly or indirectly, and examples thereof include ferroelectric substances such as tourmaline, fossilized coral, granite, and calcium strontium propionate, and ores containing a radioactive substance such as radium and radon. The far infrared ray radiating substance is not limited so far as it radiates far infrared rays. Examples thereof include ceramics, alumina, zeolite, zirconium, and silica.

The surfactant includes anionic surfactants, cationic surfactants, nonionic surfactants, and ampholytic surfactants. Especially, nonionic surfactants are preferable, and examples thereof include polyoxyethylene alkyl ethers, alkylphenol·ethylene oxide adducts, and higher alcohol phosphoric acid esters.

The organosilicon compound is not limited so far as it is a compound having at least an Si-O-R bond and/or an Si-N-R bond and/or an Si-R bond. The organosilicon compound is in the form of a monomer, a lowly condensed product, a polymer, etc. Examples thereof include organosilane compounds such as methyltriethoxysilane; and dimethylsilicone oil, polyorganosiloxane, or silicone resin compositions containing the same.

The pyroelectric substance is not limited so far as it has pyroelectricity. Examples thereof include tourmaline, hemimorphic ores, and pyroelectric ores. Tourmaline or achroite which is a kind of tourmaline is especially preferable. Examples of the tourmaline include dravite, schorl, and elbaite.

The moisturizer is not limited so far as it is able to hold moisture. Examples thereof include hyaluronic acid, collagen, glycerin, and urea.

The fertilizer component is not limited so far as it is a component containing at least one of three elements of nitrogen, phosphorus and potassium. Examples thereof include a bone powder, urea, ammonium sulfate, calcium perphosphate, potassium chloride, and calcium sulfate.

The hydrophobic polymer compound is not limited so far as it is a polymer compound having a contact angle with water of 40° or more, preferably 50° or more, and more preferably 60° or more in order to improve the draining in the composition. The shape of the hydrophobic polymer compound is not limited, and examples thereof include powdery, particulate, granular, and tablet shapes. Examples of the hydrophobic polymer compound include polyolefins such as polyethylene and polypropylene, polyesters, and polyamides.

Examples of the heat generating aid include metal powders, metal salts, and metal oxides such as Cu, Mn, $CuCl_2$, $FeCl_2$, manganese dioxide, cupric oxide, triiron tetroxide, and mixtures thereof.

As the metal oxide other than iron oxide, any material can be employed so far as it does not hinder the oxidation of iron by an oxidizing gas, and examples thereof include manganese dioxide and cupric oxide.

The acidic substance may be any of an inorganic acid, an organic acid, or an acidic salt. Examples thereof include hydrochloric acid, sulfuric acid, nitric acid, acetic acid, oxalic acid, citric acid, malic acid, maleic acid, chloroacetic acid, iron chloride, iron sulfate, iron oxalate, iron citrate, aluminum chloride, ammonium chloride, and hypochlorous acid.

As the "iron powder" as referred to herein, usual iron powders, iron alloy powders and active iron powders such as iron powders comprising particles, a surface of each of which is at least partially covered with an oxygen-containing film, and iron alloy powders comprising particles , a surface of each of which is at least partially covered with an oxygen-containing film, are preferable. Incidentally, the "iron oxide film" as referred to herein is a film made of oxygen-containing iron such as iron oxide, hydroxide or oxyhydroxide. Furthermore, the "active iron powder" as referred to herein is a powder in which an iron oxide film is formed at least locally on the surface of an iron powder, from which an oxidation reaction promoting effect is obtained by a local cell as formed between an iron matrix and an iron oxide film or a pit inside and outside the iron oxide film.

The iron powder is not limited, and examples thereof include cast iron powders, atomized iron powders, electrolyzed iron powders, reduced iron powders, sponge iron powders, and iron alloy powders thereof. In addition, the iron powder may contain carbon or oxygen, and an iron powder containing 50 % or more of iron and other metals may be employed. The kind of the metal which is contained as an alloy, etc. is not particularly limited so far as the iron component works as a component of the heat generating composition. Examples of such a metal include metals such as aluminum, manganese, copper, nickel, silicon, cobalt, palladium, and molybdenum, and semiconductors. The metal of the invention includes a semiconductor. Such a metal or alloy may be contained only in the surface or the interior, or may be contained in both the surface and the interior.

In the iron powder of the invention, the content of the metal other than iron is usually from 0.01 to 50 % by weight, and preferably from 0.1 to 10 % by weight based on the whole of the iron powder.

Examples of the iron powder having an oxygen-containing film on at least a part of the surface of the iron include:

(A) an active iron powder in which the surface of an iron component is at least partially oxidized, which is obtained by contact treating the essential components of the heat generating composition or the essential components to which acidic substances or other necessary components are added with an oxidizing gas, thereby partially oxidizing the iron component;

(B) an active iron powder in which the content of wustite is from 2 to 50 % by weight in terms of an X-ray peak intensity ratio to iron;

(C) an iron powder having an iron oxide film having a thickness of 3 nm or more on the surface thereof; and

(D) a mixture of an active iron powder and an iron powder other than an active iron powder.

With respect to (A), although the mechanism is not elucidated in detail, it is assumed that upon contact between the oxidizing gas and the components, not only an iron oxide film, namely, an oxygen-containing film is formed on the surface of the iron powder due to the oxidation of the components, especially the oxidation of the iron powder, but also the surface of active carbon is oxidized and/or the oxidized iron component is adhered, whereby hydrophilicity is imparted or improved, and coupling between the components or structurization takes place through the mediation of water. That is, it is assumed that some kind of a change in the function occurs such that an iron oxide film is formed on the surface of the iron powder, the shape of the iron powder particle becomes irregular, a strain is generated due to the oxidation, or a water-containing pit is formed, whereby the iron powder is activated and exothermic rising properties are improved.

Furthermore, the case where magnetite ($Fe_3O_4$) is present in the iron oxide film is preferable because the conductivity is excellent, and the case where hematite ($Fe_2O_3$) is present in the iron oxide film is also preferable because the iron oxide film becomes porous. Moreover, it is assumed that the carbon component is oxidized on the surface thereof and becomes a carbon component which is rich in oxides on the surface thereof, whereby the hydrophilicity increases and the activity increases.

The thickness of the iron oxide film which is an oxygen-containing film covering the surface of the iron powder, as measured by the Auger electron spectroscopy, is usually 3 nm or more, preferably from 3 nm to 100 μm, more preferably from 30 nm to 100 μm, further preferably from 30 nm to 50 μm, still further preferably from 30 nm to 1 μm, even further preferably from 30 nm to 500 nm, and even still further preferably from 50 nm to 300 nm.

When the thickness of the oxygen-containing film of iron is 3 nm or more, the thickness of the oxygen-containing film of iron is able to exhibit a promoting effect of the oxidation reaction, and upon contact with an oxidizing gas such as air, is able to immediately initiate the oxidation reaction. When the thickness of the oxygen-containing film of iron is 100 μm or more, though the heat generation time may possibly be shortened, such is applicable depending upon the utility.

Furthermore, according to the active iron powder, by using a reaction mixture containing, as essential components, an iron powder, a reaction accelerator and water and having a water content of from 0.5 to 20 % by weight and a water mobility value showing a surplus water content of less than 0.01, the reaction rate at the time of the contact treatment with an oxidizing gas can be raised, thereby achieving a time required for regulating a temperature rise of the reaction mixture at 1 °C or more within 10 minutes. By shortening a time required for arrival at a prescribed temperature or higher, proper activation can be achieved, and unnecessary oxidation on the iron powder can be prevented.

Furthermore, the heat generating composition prepared by adding a carbon component, etc. to a heat generating mixture as produced by contact treating the reaction mixture with an oxidizing gas or adjusting the water content so as to have a water mobility value of from 0.01 to 50 is properly tacky, has excellent moldability and is able to be applied with a molding method such as a force-through die molding method and a cast molding method, whereby heat generating bodies of various shapes can be produced. In particular, a heat generating composition having a water mobility value of from 0.01 to 20 is excellent because it initiates an exothermic reaction immediately after contacting with air, has excellent exothermic rising properties and has excellent moldability.

The contact treatment method of the reaction mixture with an oxidizing gas is not particularly limited so far as it is able to contact treat a reaction mixture containing, as essential components, an iron powder, a reaction accelerator and water and having a water content of from 0.5 to 20 % by weight and a water mobility value of less than 0.01 with an oxidizing gas and regulate a temperature rise of the reaction mixture at 1 °C or more.

Specific examples thereof include:

(1) a process for producing a heat generating mixture containing an iron powder having an iron oxide film on the surface thereof by subjecting a reaction mixture of an iron powder, a reaction accelerator and water in an oxidizing gas atmosphere to a self-exothermic reaction, thereby partially oxidizing the iron powder;

(2) a process for producing a heat generating mixture by subjecting a reaction mixture of an iron powder, a reaction accelerator, an acidic substance and water in an oxidizing gas atmosphere to a self-exothermic reaction;

(3) a process for producing a heat generating mixture by subjecting a reaction mixture of an iron powder, a reaction accelerator, a carbon component and water in an oxidizing gas atmosphere to a self-exothermic reaction;

(4) a process for producing a heat generating mixture by subjecting a reaction mixture of an iron powder, a reaction accelerator, an acidic substance, a carbon component and water in an oxidizing gas atmosphere to a self-exothermic reaction;

(5) a process for producing a heat generating mixture containing a partially oxidized iron powder by carrying out the method as set forth above in any one of (1) to (4), wherein the reaction mixture or heat generating mixture as set forth above in any one of (1) to (4) contains a component other than the foregoing components;

(6) a process for producing a heat generating mixture by carrying out the method as set forth above in any one of (1) to (5) under circumstances heated so as to have temperature of at least 10 °C higher than the circumferential temperature;

(7) a process for producing a heat generating mixture by carrying out the method as set forth above in any one of (1) to (6) by blowing an oxidizing gas;

(8) a process for producing a heat generating mixture by carrying out the method as set forth above in (7) by blowing the oxidizing gas heated so as to have a temperature of at least 10 °C higher than the circumferential temperature;

(9) a process for producing a heat generating composition by carrying out the method as set forth above in any one of (1) to (8) by contact treating with an oxidizing gas until the temperature exceeds a maximum temperature which is a maximum point of temperature rise by the exothermic reaction;

(10) a process for producing a heat generating mixture by carrying out the method as set forth above in any one of (1) to (8) by contact treating with an oxidizing gas until the temperature exceeds a maximum temperature by the exothermic reaction and drops by at least 10 to 20 °C from the maximum temperature;

(11) a process for producing a heat generating composition by carrying out the method as set forth above in any one of (1) to (8) by contact treating with an oxidizing gas until the temperature exceeds a maximum temperature which is a maximum point of temperature rise by the exothermic reaction and after intercepting the oxidizing gas, holding it until the temperature of at least the reaction mixture drops by at least 10 to 20 °C from the maximum temperature; and

(12) a process for producing a heat generating mixture by heating the reaction mixture or heat generating mixture as set forth above in any one of (1) to (5) under oxidizing gas circumstances while regulating a temperature rise at 1 °C or more.

In addition, a heat generating mixture as prepared by adding other components to the heat generating mixture and further treating with an oxidizing gas may be employed.

Incidentally, the circumstances of the reaction mixture at the time of contact treatment with an oxidizing gas are not limited so far as the reaction mixture is brought into contact with an oxidizing gas under circumstances of 0 °C or higher and a temperature rise of the reaction mixture is regulated at 1 °C or more within 10 minutes. In the case where the contact treatment is carried out in an open system, the circumstances may be either the state that the reaction mixture is present in a lid-free vessel or the state that an oxidizing gas such as air comes into a vessel through an air-permeable sheet-like material such as non-woven fabrics.

Furthermore, the contact treatment with an oxidizing gas may be carried out with or without stirring in a fluidized or non-fluidized state and may be carried out in a batch or continuous system.

Examples of the final heat generating composition include:

1) a heat generating composition containing, as a heat generating composition raw material, a heat generating mixture produced in the process as set forth above in any one of (1) to (12);

2) a heat generating composition obtained by adding other components to the heat generating composition as set forth above in 1); and

3) a heat generating composition obtained by adjusting the water content of the heat generating composition as set forth above in 1) or 2).

The order of the timing of adding other components than the essential components and the timing of adjusting the water content is not limited.

Here, the water content in the reaction mixture and also the heat generating mixture prior to the treatment with an oxidizing gas is usually from 0.5 to 20 % by weight, preferably from 1 to 15 % by weight, more preferably from 2 to 10 % by weight, further preferably from 3 to 10 % by weight, and still further preferably from 6 to 10 % by weight.

The temperature of the reaction mixture after the contact with an oxidizing gas is not limited so far as the temperature rise is regulated at 1 °C or more. The temperature of the reaction mixture after the contact with an oxidizing gas is preferably from 1 to 80 °C, more preferably from 1 to 70 °C, further preferably from 1 to 60 °C, and still further preferably from 1 to 40 °C.

The circumferential temperature at the time of contact between the reaction mixture and the oxidizing gas is not limited so far as the temperature of the reaction mixture is raised to a prescribed temperature or higher. The circumferential temperature at the time of contact between the reaction mixture and the oxidizing gas is preferably 0 °C or higher, more preferably from 0 to 250 °C, further preferably from 10 to 200 °C, still further preferably from 20 to 150 °C, even further preferably from 25 to 100 °C, and even still further preferably from 25 to 50 °C.

The time of contact between the reaction mixture and the oxidizing gas is not limited so far as the time required for regulating a temperature rise at 1 °C or more is within 10 minutes. The time of contact between the reaction mixture and the oxidizing gas is preferably from one second to 10 minutes, more preferably from one second to 7 minutes, further preferably from one second to 5 minutes, still further preferably from 2 seconds to 5 minutes, even further preferably from 2 seconds to 3 minutes, and even still further preferably from 2 seconds to one minute.

The temperature of the oxidizing gas is not limited so far as the foregoing circumferential temperature is kept.

As the "oxidizing gas" as referred to herein, any gas can be used as the oxidizing gas so far as it is oxidizing. Examples thereof include an oxygen gas, air, and mixed gases of an inert gas (for example, a nitrogen gas, an argon gas, and a helium gas) and an oxygen gas. Although the mixed gas is not limited so far as it contains oxygen, mixed gases containing 10 % or more of an oxygen gas are preferable, and of these, air is especially preferable. If desired, a catalyst such as platinum, palladium, iridium, and compounds thereof can also be used.

The oxidation reaction can be carried out under stirring in an oxidizing gas atmosphere optionally under a pressure and/or upon irradiation of ultrasonic waves.

The optimal condition of the oxidation reaction may be properly experimentally determined.

An amount of the oxidizing gas to be used is not limited but may be adjusted depending upon the kind of the oxidizing gas, the kind and particle size of the iron powder, the water content, the treatment temperature, the treatment method, and the like.

In the case of an open system, there is no limitation so far as a necessary amount of oxygen can be taken in. In order to prevent fly of the reaction mixture or contamination of dusts, etc., the system may be surrounded by an air-permeable raw material such as non-woven fabrics and woven fabrics. So far as the system is in an air-permeable state, it is to be noted that the system is an open system.

In the case where air is used in the system of blowing an oxidizing gas, for example, the amount of air is preferably from 0.01 to 1, 000 L/min, more preferably from 0.01 to 100 L/min, and further preferably from 0.1 to 50 L/min per 200 g of the iron powder under one atmosphere. In the case of other oxidizing gas, the amount of the oxidizing gas may be converted on the basis of the case of air.

If desired, a peroxide may be added. Examples of the peroxide include hydrogen peroxide and ozone.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of the contact treatment with a mixed oxidizing gas at the time of adjusting the water content are not limited, and examples thereof include those in an oxidizing gas atmosphere and those in blowing of an oxidizing gas.

A method for measuring a temperature rise of the heat generating composition is as follows.

1) A heat generating composition is allowed to stand in a state that it is sealed in an air-impermeable outer bag for one hour under a condition that the circumferential temperature is 20 ± 1 °C.
2) A magnet is provided in the vicinity of a central part of the back side of a polyvinyl chloride-made supporting plate (3 mm in thickness x 600 mm in length x 600 mm in width) of a footed supporting table so as to cover a cavity shape of a molding die.
3) A temperature sensor is placed on the central part of the supporting plate.
4) A polyethylene film (25 $\mu$m in thickness x 250 mm in length x 200 mm in width) as provided with an adhesive layer having a thickness of about 80 $\mu$m is stuck onto the supporting plate via a sticky layer such that the center of the polyethylene film is positioned at the sensor.
5) The heat generating composition is taken out from the outer bag.
6) A template (250 mm in length x 200 mm in width) having a cavity (80 mm in length x 50 mm in width x 3 mm in height) is placed above the central part of the polyethylene film; a sample is placed in the vicinity of the cavity; a force-in die plate is moved along the template; the sample is charged into the cavity while stuffing; and the sample is leveled while stuffing along the template plane (force-in die molding), thereby filling the sample in the die. Next, the magnet beneath the supporting plate is removed, and the temperature measurement is started.

With respect to the measurement of the exothermic temperature, the temperature is measured for 10 minutes at a measurement timing of 2 seconds using a data collector, and exothermic rising properties are judged in terms of the temperature after elapsing 3 minutes.

The heat generation test of the heat generating body follows the JIS temperature characteristic test.

In the iron powder or active iron powder in the oxidizing gas-treated heat generating composition, at least a part of the surface thereof is covered by an oxygen-containing film of iron. The degree of covering on the surface of the oxygen-containing film of iron is not limited so far as at least a part of the surface thereof is covered, and the surface may be entirely covered. In the case of the heat generating composition of the invention, since an ion of the reaction accelerator such as a chlorine ion is contained in the heat generating composition, there is no corrosion effect of the oxide film due to anti-corrosion effect by the ion of the reaction accelerator such as a chlorine ion. Thus, the oxidation reaction which is a sort of corrosion is not hindered. In particular, in the case where an oxygen-containing film of iron is prepared while the ion of the reaction accelerator such as a chlorine ion exists together, the subject effect is large. In the case where a metal other than iron is present on the surface, it is only required that at least other part of the metal portion other than iron is covered by the oxygen-containing film of iron.

In the iron powder of the invention, not only a region where (1) entire (uniform) corrosion, (2) pitting or crevice corrosion, (3) stress corrosion cracking, or the like is generated, but also irregularities or crevices are formed. For that reason, it is assumed that the iron powder of the invention has hydrophilicity and oxidation catalytic properties (FeO, etc.) in its own portion. In producing the heat generating composition, it is important that the iron powder has an oxygen-containing film in its own portion without relying upon mixing. In particular, in the iron component as prepared by contact treating the iron component and the reaction accelerator and water as essential components with an oxidizing gas, it is thought that a reaction active part composed mainly of an oxide, a hydroxide, a chlorine ion, a hydrogen ion, etc. is formed, whereby exothermic reactivity and hydrophilicity are improved and exothermic rising properties and moldability are remarkably improved.

With respect to (B), the amount of FeO (wustite) which is contained in the iron component containing a prescribed amount of wustite is usually from 2 to 50 % by weight, preferably from 2 to 40 % by weight, more preferably from 2 to 30 % by weight, further preferably from 5 to 30 % by weight, and still further preferably from 6 to 30 % by weight in terms of an X-ray peak intensity ratio of iron. When the amount of FeO (wustite) exceeds 50 % by weight, though the exothermic rising properties are good, the duration of heat generation becomes short. On the other hand, when it is less than 2 % by weight, the exothermic rising properties become dull.

The thickness of the oxygen-containing film of a prescribed amount or the oxygen-containing film of iron powder containing wustite and the amount of wustite are applied to the heat generating composition or the heat generating composition molded body at the time of lamination.

An iron powder containing a carbon component and/or covered by a carbon component is also preferable. Although a proportion of the carbon component is not limited so far as a ratio of the iron component to the carbon component is 50 % by weight or more, an iron powder in which the surface thereof is partially covered by from 0.3 to 3.0 % by weight of a conductive carbonaceous substance is useful. Examples of the conductive carbonaceous substance include carbon black, active carbon, carbon nanotubes, carbon nanohorns, and flullerenes. Ones which have become conductive by doping are also employable. Examples of the iron powder include reduced iron powders, atomized iron powders, and sponge iron powders. In particular, the case where the conductive carbonaceous substance is active carbon and the iron powder is a reduced iron powder is useful as a heat generating body.

Furthermore, in order to efficiently carry out covering by a conductive carbonaceous substance, an oil such as a spindle oil may be added in an amount of from 0.01 to 0.05 % by weight to such an extent that the fluidity of the iron powder is not hindered.

In the case of measuring the water mobility value of the heat generating composition in the heat generating body and the thickness and amount of wustite of the iron oxide film of iron powder in the mixture or the heat generating composition in the heat generating body, the heat generating composition or mixture may be measured according to the following items.

1) Water mobility value:
The heat generating composition is taken out from the heat generating body and measured according to the foregoing method of measuring a water mobility value.
2) Thickness and amount of wustite of iron oxide film of iron powder:
A measuring sample as prepared by dispersing the heat generating composition, the heat generating composition molded body, the heat generating composition compression molded body or the mixture in nitrogen-purged ion-exchanged water in a nitrogen atmosphere, separating the iron powder using a magnet and drying the iron powder in a nitrogen atmosphere is used.

The heat generating composition of the invention contains, as essential components, an iron powder, a carbon component, a reaction accelerator and water, and its production process is one which can be put into practical use on an industrial scale. A reaction mixture containing, as essential components, an iron powder, a reaction accelerator and water and having a water content of from 1 to 20 % by weight and a water mobility value showing a surplus water content of less than 0.01 is brought into contact with an oxidizing gas under circumstances at 0 °C or higher, a temperature rise of the reaction mixture is regulated at 1 °C or more within 10 minutes to produce a heat generating mixture, and the subject heat generating mixture is used as a raw material to form a heat generating composition. Alternatively, a heat generating composition may be formed by subsequently further adjusting the water content, or by further adding a carbon component, etc. and adjusting the water content.

In the invention, it has become possible to realize the contact treatment with an oxidizing gas within a short period of time by regulating the water content of the reaction mixture at a fixed amount or less, especially regulating the surplus water content of the reaction mixture at a fixed amount or less and carrying out an oxidizing contact treatment. By specifying the surplus water content and performing the treatment within a short period of time, adverse influences such as poor initial exothermic rising of the heat generating composition and shortening of the heat generation-retaining time can be avoided. Thus, it has become possible to establish an industrial mass-production process. Furthermore, although stirring or the like may not be achieved during the contact treatment with an oxidizing gas, when stirring or the like is achieved, the contact treatment with an oxidizing gas can be surely carried out.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of mixing at the time of adjusting the water content are not limited, and examples thereof include those in an oxidizing gas atmosphere and those in blowing of an oxidizing gas.

The "adjustment of the water content" as referred to herein means that after contact treating the heat generating mixture with an oxidizing gas, water or an aqueous solution of a reaction accelerator is added. Although the amount of addition of water or an aqueous solution of a reaction accelerator is not limited, examples thereof include the addition of a weight corresponding to a reduced weight by the contact treatment and the addition of a weight such that a desired water mobility value is obtained.

Whether or nor the adjustment of the water content is introduced may be properly determined depending upon the utility.

The heat generating composition of the invention contains, as essential components, an iron powder, a carbon component, a reaction accelerator and water and is started from a mixture obtained by contact treating a reaction mixture containing, as essential components, an iron powder, a reaction accelerator and water with an oxidizing gas. The heat generating composition of the invention is usually one obtained by adjusting the water content of a heat generating mixture and is a heat generating composition which is satisfactory in the exothermic rising, has a suitable amount of surplus water and has excellent moldability. Furthermore, it is possible to produce a heat generating body which can become promptly warm at the time of use.

Accordingly, at least the iron powder further including the carbon component has a history of oxidation by the contact treatment with an oxidizing gas, and it is thought that this is deeply related to excellent exothermic rising properties, exothermic endurance and excellent moldability.

When the iron powder which is contact treated with an oxidizing gas according to the invention is used, the amount of addition of the carbon component (for example, active carbon) in the heat generating composition can be reduced by, for example, 20 % or more. By reducing the amount of addition of the carbon component, the costs are lowered.

According to the production process of the heat generating mixture of the invention, it is possible to obtain a heat generating composition having excellent exothermic rising properties, excellent hydrophilicity, and excellent moldability. In particular, a heat generating composition having remarkably excellent moldability and exothermic characteristics together can be obtained while specifying the water availability value at from 0.01 to 50, in particular 0. 01 to 20.

The heat generating composition as produced by the production process of the invention is remarkably improved with respect to exothermic rising properties. Thus, the amount of addition of the carbon component (such as active carbon) in the heat generating composition can be reduced by, for example, 20 % or more so that it can contribute to a reduction in costs.

Furthermore, since the hydrophilicity is remarkably improved, the moldability with a mold is remarkably improved. Thus, since after molding, collapsed pieces of the heat generating composition are not scattered on the surroundings of the heat generating composition molded body, sealing can be appropriately achieved so that a heat generating body free from sealing cut can be produced. In this way, heat generating composition molded bodies of various shapes can be produced, and heat generating bodies of various shapes are formed.

Furthermore, in view of improving the exothermic rising properties of the heat generating composition, the following are preferable.

1) A heat generating composition obtained by a contact treatment (self heat generation) of a mixture of the essential components of the heat generating composition, or a mixture of the foregoing mixture and an acidic substance or other necessary components with an oxidizing gas, a heat generating composition obtained by additionally adjusting the water content of the foregoing heat generating composition, or a heat generating composition obtained by adding and mixing other components in the foregoing heat generating composition.
2) Any one of the following active iron powders having an oxygen-containing film (for example, oxides) on at least a part of the surface thereof is used as the iron powder: (a) an iron powder having an oxygen-containing film of iron having a thickness, as measured by the Auger electron spectroscopy, of 3 nm or more on the surface thereof and (b) an iron powder having a content of wustite of from 2 to 50 % by weight in terms of an X-ray peak intensity ratio to iron.
3) A mixture of an active iron powder having an oxygen-containing film (for example, oxides) on at least a part of the surface thereof and an iron powder not having an oxygen-containing film is used as the iron powder. In this case, a mixture containing 60 % by weight or more of an active iron powder and less than 40 % by weight of an iron powder other than the active iron is preferable.

In the case of storing the heat generating composition which is treated with an oxidizing gas or the heat generating composition containing an active iron powder, or a material utilizing the same over a long period of time, it is preferred to combine a hydrogen formation inhibitor therewith. This is because in this way, a heat generating body having excellent exothermic characteristics, which is inhibited in the formation of hydrogen, is free from swelling of the outer bag at the time of storage, etc. and has satisfactory exothermic rising properties, is obtained.

Furthermore, since a marketed heat generating body in which a heat generating composition is accommodated in an accommodating bag is provided on the assumption that it is accommodated in an outer bag which is an air-impermeable accommodating bag and is storable over a long period of time, it is preferred to use a heat generating composition containing a hydrogen formation inhibitor. Since the heat generating composition which has passed through the contact treatment with an oxidizing gas is an active composition, it is important that the heat generating composition contains a hydrogen formation inhibitor. Also, this efficacy is further strengthened by using a pH adjusting agent together.

Furthermore, so far as the reaction characteristics and exothermic characteristics are not affected, the heat generating composition having a water mobility value of less than 0.01 may contain a flocculant aid, a flocculant, an agglomeration aid, a dry binder, a dry binding agent, a dry binding material, a sticky raw material, a thickener, an excipient, or a water-soluble polymer in an amount ranging from 0.01 to 3 parts by weight respectively.

The "flocculant aid" as referred to herein is a flocculant aid as described in Japanese Patent No. 3,161,605 (JP-T-11-508314) such as gelatin, natural gum, and corn syrup.

The "flocculant" as referred to herein is a flocculant as described in JP-T-2002-514104 such as corn syrup and maltitol syrup.

The "agglomeration aid" as referred to herein is an agglomeration aid as described in JP-T-2001-507593 such as corn syrup.

The "dry binder" as referred to herein is a dry binder as described in JP-T-2002-514104 such as microcrystalline cellulose, maltodextrin, and mixtures thereof.

The "dry binding agent" as referred to herein is a dry binding agent as described in JP-T-2001-507593 such as maltodextrin and sprayed lactose.

The "dry binding material" as referred to herein is a dry binding material as described in JP-T-11-508314 such as microcrystalline cellulose, maltodextrin, and mixtures thereof.

The "sticky raw material" or the "binder" as referred to herein is a sticky raw material or binder as described in JP-A-4-293989 such as water glass, polyvinyl alcohol (PVA), and carboxymethyl cellulose (CMC).

The "thickener" as referred to herein is a thickener as described in JP-A-6-343658 such as corn starch and potato starch.

The "excipient" as referred to herein is an excipient as described in JP-A-7-194641 such as $\alpha$-starch and sodium alginate.

As the "water-soluble polymer" as referred to herein, the water-soluble polymer in the adhesive layer can be used.

The particle size of the water-insoluble solid component constituting the moldable heat generating composition of the invention is not limited so far as the heat generating composition has moldability. In the case where any one of length, width and height as the size of the heat generating composition molded body as molded from the heat generating composition is small, the moldability is improved by making the particle size small.

In addition, it is preferable in view of molding that the particle size of the solid component constituting the moldable heat generating composition is small. A maximum particle size of the water-insoluble solid component exclusive of the reaction accelerator and water in the components constituting the moldable heat generating composition is preferably not more than 2.5 mm, more preferably not more than 930 $\mu$m, further preferably not more than 500 $\mu$m, still further preferably not more than 300 $\mu$m, even further preferably not more than 250 $\mu$m, and even still further preferably not more than 200 $\mu$m. Moreover, 80 % or more of the particle size of the solid component is usually not more than 500 $\mu$m, preferably not more than 300 $\mu$m, more preferably not more than 250 $\mu$m, further preferably not more than 200 $\mu$m, still further preferably not more than 150 $\mu$m, and even further preferably not more than 100 $\mu$m.

Incidentally, with respect to the particle size of the water-insoluble solid component, separation is conducted using a sieve, and the particle size of the component which has passed through the sieve is calculated from an opening of the sieve. That is, sieves of 8, 12, 20, 32, 42, 60, 80, 100, 115, 150, 200, 250 and 280 meshes and a receiving dish are combined in this order from up to down. About 50 g of water-insoluble solid component particles are placed on the uppermost 8-mesh sieve and shaken for one minute using an automatic shaker. Weights of the water-insoluble solid component particles on each of the sieves and the receiving dish are weighed. The total amount thereof is defined as 100 %, and the particle size distribution is determined from weight fractions. When the sum of all receiving dishes under the sieve of a specific mesh size becomes 100 % which is the total sum of the particle size distribution, the size ($\mu$m) calculated from the opening of the specific mesh is defined as the particle size of the water-insoluble solid component. Incidentally, each of the mesh sieves may be combined with other mesh sieves. Here, the particles which have passed through a 16-mesh sieve are defined to have a particle size of not more than 1 mm; the particles which have passed through a 20-mesh sieve are defined to have a particle size of not more than 850 $\mu$m; the particles which have passed through a 48-mesh sieve are defined to have a particle size of not more than 300 $\mu$m; the particles which have passed through a 60-mesh sieve are defined to have a particle size of not more than 250 $\mu$m; the particles which have passed through a 65-mesh sieve are defined to have a particle size of not more than 200 $\mu$m; the particles which have passed through an 80-mesh sieve are defined to have a particle size of not more than 180 $\mu$m; the particles which have passed through a 100-mesh sieve are defined to have a particle size of not more than 150 $\mu$m; the particles which have passed through a 115-mesh sieve are defined to have a particle size of not more than 120 $\mu$m; the particles which have passed through a 150-mesh sieve are defined to have a particle size of not more than 100 $\mu$m; and the particles which have passed through a 250-mesh sieve are defined to have a particle size of not more 63 $\mu$m, respectively. The same is applicable to mesh sizes of less than these mesh sizes.

Furthermore, the heat generating composition can be classified into a powder, a granulate heat generating composition (having a water mobility value of less than 0.01), a moldable heat generating composition (having a water mobility value of from 0.01 to 20), and a sherbet-like heat generating composition (having a water mobility value exceeding 20 but not more than 50) depending upon the state of adjustment of the water content or surplus water. The heat generating composition as classified depending upon the water mobility value is as described previously.

The "moldability" as referred to in the invention exhibits that a laminate of the heat generating composition having a cavity or concave die shape can be formed by force-through molding using a trimming die having a cavity or cast molding using a concave die and after molding including mold release, the molding shape of the heat generating composition molded body is held. When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, sealing can be achieved without causing cutting in seal. The presence of the spots causes insufficient sealing.

Next, with respect to the moldability, a measurement device, a measurement method and a judgment method will be described below.

1) Measurement device:

With respect to the measurement device, a stainless steel-made molding die (a plate having a size of 2 mm in thickness x 200 mm in length x 200 mm in width and having a cavity as treated by R5 in four corners of 60 mm in length x 40 mm in width in a central part thereof) and a fixable leveling plate are disposed above a travelable endless belt, and magnets (two magnets having a size of 12.5 mm in thickness x 24 mm in length x 24 mm in width are disposed in parallel) are disposed under the endless belt. The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness x 200 mm in length x 200 mm in width is placed on the endless belt of the measurement device, a polyethylene film having a size of 70 $\mu$m in thickness x 200 mm in length x 200 mm in width is placed thereon, and a stainless steel-made molding die is further placed thereon. Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1.8 m/min. After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 $\mu$m is not present and the number of collapsed pieces of the heat generating composition molded body having a maximum length of from 300 to 800 $\mu$m is not more than 5, it is to be noted that the heat generating composition has moldability. The moldability is an essential property for a heat generating composition to be used in the molding system. If the heat generating composition does not have moldability, it is impossible to produce a heat generating body by the molding system.

The heat generating composition of the invention has resistance to compression. The "resistance to compression" as referred to herein means that a heat generating composition compressed body obtained by compressing a heat generating composition molded body as accommodated in a molding die within the die to such an extent that the thickness is 70 % of the die thickness holds 80 % or more of exothermic rising properties of the exothermic rising properties of the heat generating composition molded body before compression (a difference in temperature between one minute and 3 minutes after starting a heat generation test of the heat generating composition).

Here, the measurement method of exothermic rinsing properties for the resistance to compression will be described below.

1. Heat generating composition molded body:

1) A magnet is provided in the vicinity of a central part of the back side of a polyvinyl chloride-made supporting plate (5 mm in thickness x 600 mm in length x 600 mm in width) of a footed supporting table so as to cover a cavity shape of a molding die.
2) A temperature sensor is placed on the central part the surface of the supporting plate.
3) A polyethylene film (25 $\mu$m in thickness x 250 mm in length x 200 mm in width) as provided with an adhesive layer having a thickness of about 80 $\mu$m is stuck onto the supporting plate via a sticky layer such that the center of the polyethylene film is positioned at the sensor.
4) On an underlay plate (280 mm in length x 150 mm in width x 50 $\mu$m to 2 mm in thickness), a polyethylene film (230 mm in length x 155 mm in width x 25 $\mu$m to 100 $\mu$m in thickness) is placed such that one end of the polyethylene film is projected by about 20 mm outside the underlay plate and that one end thereof in the length direction is substantially coincident with one end of the underlay plate.
5) A template (230 mm in length x 120 mm in width x 3 mm in thickness) having a cavity (80 mm in length x 50 mm in width x 3 mm in height) is placed on the polyethylen film placed on the underlay plate; a template is placed on the polyethylene film such that one end thereof in the length direction is fitted to one end where the underlay plate and the polyethylene film are coincident with each other and that in the width direction, one end part of the width of the template is placed at a position of the central part by about 20 mm far from an opposing end to the side where the polyethylene film is projected outward from the underlay plate. Next, the resulting assembly is placed on the supporting plate together with the underlay plate.
6) A sample is placed in the vicinity of the cavity; a force-in die plate is moved along the molding die; the sample is charged into the cavity while stuffing; and the sample is leveled while stuffing along the template plane (force-in die molding), thereby filling the sample in the die.
7) Next, the magnet beneath the supporting plate is removed; the end portion of the projected polyethylene film is pressed; the underlay plate is removed; and the temperature measurement is started.

2. Heat generating composition compressed body:

1) to 6) are the same as in the case of the heat generating composition molded body.
8) A die having a convex having a thickness of 0.9 mm which can substantially tightly come into the cavity in relation of the cavity with an unevenness is fitted to the cavity and compressed by a roll press or plate press to prepare a heat generating composition compressed body having a thickness of 2.1 mm (compressed to 70 % of the die thickness) within the die.
9) The resulting assembly is placed on the supporting plate together with the underlay plate; the magnet beneath the supporting plate is removed; the end portion of the projected polyethylene film is pressed; the underlay plate is removed; and the temperature measurement is started.

With respect to the measurement of the exothermic temperature, the temperature is measured for 5 minutes at a measurement timing of 2 seconds using a data collector, and resistance to compression is judged in terms of a difference in temperature between after elapsing one minute and after elapsing 3 minutes.
The thickness after compression is preferably from 50 to 99.5 %, more preferably from 60 to 99.5 %, and further preferably from 60 to 95 % of the die thickness.
Incidentally, in the invention, it is to be noted that the heat generating composition molded body includes a heat generating composition compressed body.

Examples of the expanded sheet include sheets formed of at least one member selected from expanded polyurethane, expanded polystyrene, expanded ABS resins, expanded polyvinyl chloride, expanded polyethylene, and expanded polypropylene.
The fixing means is not limited so far as it has capability for fixing a thermal packaging body for joint surroundings or a material having an exothermic part to a prescribed part.
As the fixing means, an adhesive layer, a hook and eye, a hook and button, a hook and loop fastener such as Velcro, a magnet, a band, a string, and combination thereof can be arbitrarily used.
Incidentally, in the case of a band, fixing means for adjustment may be further constructed by a combination of a hook and loop fastener and an adhesive layer.
Here, the "hook and loop fastener" as referred to herein has a fastening function by a combination of a loop as a female fastener with a male fastener capable of fastening the female fastener thereto, which is known as trade names such as Magic Tape (a registered trademark), Magic Fastener (a registered trademark), Velcro Fastener, and Hook and Loop Tape. Examples of the material having a loop function include non-woven fabrics and woven fabrics of napped or hole-containing yarns. Such a material having a loop function (female fastener function) may be covered on the surface of a paddling forming the band, or the band may be constructed of such a material itself. Although the hook member which is the male fastener member is not particularly limited, examples thereof include hook members formed of a polyolefin based resin (for example, polyethylene and polypropylene), a polyamide, a polyester, etc. Although the shape of the hook is not particularly limited, a hook having a cross-sectional shape such as an I type, an inverted L type, an inverted J type, and a so-called mushroom type is preferable because it is easily hooked by the loop and does not give an extreme stimulus to the skin. Incidentally, the hook may be adhered to the entire area of a fastening tape, and only the hook may be used as a fastening tape while omitting a tape substrate.

The adhesive layer may contain at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

The adhesive of the invention is classified into a non-hydrophilic adhesive, a mixed adhesive, and a hydrophilic adhesive (for example, a gel).

The adhesive constituting the adhesive layer is not limited so far as it has an adhesive strength necessary for adhering to the skin or clothes. Adhesives of every form such as a solvent based adhesive, an aqueous adhesive, an emulsion type adhesive, a hot melt type adhesive, a reactive adhesive, a pressure-sensitive adhesive, a non-hydrophilic adhesive, and a hydrophilic adhesive are employable.

The adhesive layer includes one layer of a non-hydrophilic adhesive constituted of the non-hydrophilic adhesive and non-hydrophilic adhesive layers constituted of the non-hydrophilic adhesive.

It is to be noted that a material whose water absorption properties are improving by containing a water absorptive polymer or a water retaining agent in the non-hydrophilic adhesive layer is dealt as the non-hydrophilic adhesive layer.

A hot melt based adhesive may be provided between the hydrophilic adhesive layer and a substrate or a covering material. Furthermore, in the case where the hydrophilic adhesive is provided in a thermal packaging body for joint surroundings, there is no limitation. After seal treating a thermal packaging body for joint surroundings, a hydrophilic adhesive layer may be provided in the thermal packaging body for joint surroundings.

Furthermore, the adhesive layer may or may not have air permeability and may be properly selected depending upon the utility. With respect to the air permeability, the adhesive layer may be air-permeable as a whole. Examples thereof include an adhesive layer having air permeability as a whole of a region in which an adhesive is partially present and a portion where no adhesive is present is partially present.

In laminating an adhesive on an air-permeable substrate and/or a covering material in a stratiform state as it is, examples of a method for keeping its air permeability include a method in which an adhesive layer is partially laminated by printing or transferring an adhesive, thereby forming a non-laminated part as an air-permeable part; a method in which an adhesive is transferred in one direction while drawing a circle in a filament-like form or properly moved in the two-dimensional directions by transferring in a zigzag manner, whereby a space of the filament-like adhesive keeps air permeability or moisture permeability or the adhesive is foamed; and a method for forming a layer by a melt blow system. Examples of the adhesive which constitutes the non-hydrophilic adhesive layer include acrylic adhesives, polyvinyl acetate based adhesives (for example, vinyl acetate resin based emulsions and ethylene-vinyl acetate resin based holt melt adhesives), polyvinyl alcohol based adhesives, polyvinyl acetal based adhesives, vinyl chloride based adhesives, polyamide based adhesives, polyethylene based adhesives, cellulose based adhesives, chloroprene (neoprene) based adhesives, nitrile rubber based adhesives, polysulfide based adhesives, butyl rubber based adhesives, silicone rubber based adhesives, styrene based adhesives (for example, styrene based hot melt adhesives), rubber based adhesives, and silicone based adhesives. Of these, rubber based adhesives, acrylic adhesives, and adhesives containing a hot melt based polymer substance for the reasons that they are high in the adhesive strength, are cheap, are good in long-term stability, and are small in reduction of the adhesive strength even by providing heat.

In addition to the base polymer, if desired, the adhesive may be compounded with other components such as tackifiers (for example, petroleum resins represented by rosins, chroman-indene resins, hydrogenated petroleum resins, maleic anhydride-modified rosins, rosin derivatives, and C-5 based petroleum resins), phenol based tackifiers (especially, tackifiers having an aniline point of not higher than 50 °C; for example, terpene phenol based resins, rosin phenol based resins, and alkylphenol based resins), softeners (for example, coconut oil, castor oil, olive oil, camellia oil, and liquid paraffin), softeners, anti-aging agents, fillers, aggregates, adhesion adjusting agents, adhesion modifiers, coloring agents, anti-foaming agents, thickeners, and modifiers, thereby improving performance such as an improvement in adhesion to nylon-made clothes and mixed yarn clothes.

Examples of the hot melt based adhesive include known hot melt based adhesives imparted with adhesion. Specific examples thereof include styrene based adhesives made of, as a base polymer, an A-B-A type block copolymer (for example, SIS, SBS, SEBS, and SIPS), vinyl chloride based adhesives made of, as a base polymer, a vinyl chloride resin, polyester based adhesives made of, as a base polymer, a polyester, polyamide based adhesives made of, as a base polymer, a polyamide, acrylic adhesives made of, as a base polymer, an acrylic resin, polyolefin based adhesives made of, as a base polymer, a polyolefin (for example, polyethylene, super low density polyethylene, polypropylene, ethylene-α-olefin copolymers, and ethylene-vinyl acetate copolymers), 1,2-polybutadiene based adhesives made of, as a base polymer, 1, 2-polybutadiene, and polyurethane based adhesives made of, as a base polymer, polyurethane; adhesives made of a modified body of the foregoing adhesive whose adhesion is improved or whose stability is changed; and mixtures of two or more kinds of these adhesives. Adhesive layers constituted of a foamed adhesive and adhesive layers constituted of a crosslinked adhesive can also be employed.

The non-aromatic hot melt based adhesive is not limited so far as it is made of, as a base polymer, a hot melt based adhesive not containing an aromatic ring. Examples thereof include olefin based hot melt based adhesives and acrylic hot melt based adhesives. As the non-aromatic polymer which is the base polymer not containing an aromatic ring, there are enumerated polymers or copolymers of an olefin or a diene. Examples thereof include olefin polymers. The olefin polymer includes polymers or copolymers of ethylene or an α-olefin. Also, polymers resulting from adding a diene (for example, butadiene and isoprene) as other monomer thereto may be employed.

The α-olefin is not limited so far as it is a monomer having a double bond in the terminal thereof. Examples thereof include propylene, butene, heptane, hexene, and octene.

The "aromatic hot melt based adhesive" as referred to herein is a hot melt based adhesive whose base polymer contains an aromatic ring. Examples thereof include styrene based hot melt based adhesives represented by A-B-A type block copolymers.

In the foregoing A-B-A type block copolymers, the A block is a non-elastic polymer block made of a monovinyl substituted aromatic compound A such as styrene and methylstyrene; and the B block is an elastic polymer block made of a conjugated diene such as butadiene and isoprene. Specific examples thereof include a styrene-butadiene-styrene block copolymer (SBS), a styrene-isoprene-styrene block copolymer (SIS), and hydrogenated types thereof (for example, SEBS and SIPS), and mixtures thereof.

As a countermeasure for preventing a lowering of adhesive strength caused due to an increase of water of the non-hydrophilic adhesive layer, an adhesive layer obtained by further compounding a water absorptive polymer in the non-hydrophilic adhesive can be used.

The hydrophilic adhesive which constitutes the hydrophilic adhesive layer is not particularly limited so far as it contains a hydrophilic polymer or a water-soluble polymer as the major component, has adhesion and is hydrophilic as an adhesive.

Examples of the constitutional components of the hydrophilic adhesive include hydrophilic polymers (for example, polyacrylic acid), water-soluble polymers (for example, poly(sodium acrylate) and polyvinylpyrrolidone), crosslinking agents (for example, dry aluminum hydroxide and meta-silicic acid aluminic acid metal salts), softeners (for example, glycerin and propylene glycol), higher hydrocarbons (for example, soft liquid paraffin and polybutene), primary alcohol fatty acid esters (for example, isopropyl myristate), silicon-containing compounds (for example, silicone oil), fatty acid glycerin esters (for example monoglycerides), oily components (for example, vegetable oils such as olive oil), antiseptics (for example, methyl p-hydroxybenzoate and propyl p-hydroxybenzoate), solubilizing agents (for example, N-methyl-2-pyrrolidone), thickeners (for example, carboxymethyl cellulose), surfactants (for example, polyoxyethylene hardened castor oil and sorbitan fatty acid esters), hydroxycarboxylic acid (for example, tartaric acid), excipients (for example, light silicic anhydride, water absorptive polymers, and kaolin), moisturizers (for example, D-sorbitol), stabilizers (for example, sodium edetate, p-hydroxybenzoic acid esters, and tartaric acid), crosslinking type water absorptive polymers, boron compounds (for example, boric acid), and water. They may be used as an arbitrary combination.

A temporary adhering seal part is formed via a sticky layer. An adhesive which constitutes the sticky layer is a layer formed of a polymer composition which is tacky at the normal temperature and is not limited so far as it can be heat sealed after temporary adhesion.

Furthermore, the foregoing adhesives of the sticky layer can be used as the adhesive which constitutes the sticky layer as used for temporary adhesion. Of these, non-hydrophilic adhesives are preferable. With respect to the adhesive constituting the adhesive layer, it is preferable that the adhesive is well compatible with a heat seal material constituting a heat seal and that a melting point of the base polymer of the adhesive is not higher than a melting point of the heat seal material. Hot melt based adhesives are especially preferable for hot melt based bonding agents. Furthermore, in the case where the heat seal material is an olefin based raw material, preferred examples thereof include olefin based adhesives.

A bonding layer for fixing the air permeability adjusting material is constituted of a bonding agent or an adhesive which is usually used. In particular, an adhesive is useful, and the foregoing adhesives for constituting the adhesive layer can be used.

Furthermore, a method for providing a bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape.

Furthermore, in the case where an adhesive layer is employed as the hydrophilic adhesive layer, if there is a difference in a water retaining force between the hydrophilic adhesive layer and the heat generating composition molded body, transfer of water occurs via a packaging material present therebetween such as a substrate, thereby causing in-conveniences against the both. In particular, the transfer of water occurs during the storage. In order to prevent this, it is preferable that the packaging material present therebetween at least has a moisture permeability of not more than 2 g/m$^2$/day in terms of a moisture permeability according to the Lyssy method. By using this, in the case where the heat generating body is accommodated in an outer bag as an air-impermeable accommodating bag and stored, the transfer of water can be prevented.

In the case where a hydrophilic adhesive layer is used as the adhesive layer, the moisture permeability of a moisture-proof packaging material provided between the heat generating composition molded body and the hydrophilic adhesive layer is not limited so far as the transfer of water can be prevented within the range where the exothermic performance is not affected. The moisture permeability according to the Lyssy method is usually not more than 2 g/m$^2$/day, preferably not more than 1.0 g/m$^2$/day, more preferably not more than 0.5 g/m$^2$/day, and further preferably from 0.01 to 0.5 g/m$^2$/day. These values are a value under a condition under an atmospheric pressure at 40 °C and 90 % RH. Incidentally, the moisture-proof packaging material can be used as a substrate or a covering material and may be laminated singly on a substrate, a covering material, or the like.

The moisture-proof packaging material is not limited so far as the transfer of water between the heat generating composition molded body and the hydrophilic adhesive layer can be prevented. Examples thereof include metal vapor deposited films, vapor deposited films of a metal oxide, metal foil-laminated films, EVOH (ethylene/vinyl alcohol copolymer or ethylene/vinyl acetate copolymer saponified product) based films, biaxially stretched polyvinyl alcohol films, polyvinylidene chloride coated films, polyvinylidene chloride coated films obtained by coating polyvinylidene chloride on a substrate film (for example, polypropylene), metal foils such as an aluminum foil, air-impermeable packaging materials obtained by vapor depositing or sputtering a metal (for example, aluminum) on a polyester film substrate, and packaging laminates using a transparent barrier film of a structure in which silicon oxide or aluminum oxide is provided on a flexible plastic substrate. The air-impermeable packaging materials which are used in the outer bag, etc. can also be used.

Furthermore, packaging materials such as moisture-proof packaging materials as described in JP-A-2002-200108, the disclosures of which can be incorporated herein by reference, can be used.

In the case of using a water-containing hydrophilic adhesive (for example, a gel) in the adhesive layer, in order to adjust the moisture equilibrium between the heat generating composition and the adhesive layer, the content of a reaction accelerator (for example, sodium chloride) or a substance having a water holding power (for example, a water absorptive polymer) in the heat generating composition may be adjusted within the range of from 10 to 40 % by weight, preferably from 15 to 40 % by weight, and more preferably from 15 to 30 % by weight based on the heat generating composition. Furthermore, as the adhesive having good moisture permeability and low stimulation to the skin, water-containing adhesives (for example, hydrophilic adhesives and gels) as described in JP-A-10-265373 and JP-A-9-87173, adhesives which can be subjected to hot melt coating as described in JP-A-6-145050 and JP-A-6-199660, and rubber based adhesives as described JP-A-10-279466 and JP-A-10-182408, the disclosures of which are totally incorporated herein by reference, are useful.

The functional substance which is contained in the adhesive layer is not limited so far as it is a substance having any function. There can be enumerated at least one member selected from aromatic compounds, vegetable extracts, crude drugs, perfumes, slimming agents, analgesics, blood circulation promoters, swelling improvers, antibacterial agents, sterilizers, mold inhibitors, odor eaters, deodorants, percutaneously absorptive drugs, fat-splitting components, minus ion generators, far infrared ray radiants, magnetic bodies, fomentations, cosmetics, bamboo vinegar, and wood vinegar. Specific examples thereof include aromatic compounds (for example, menthol and benzaldehyde), vegetable extracts (for example, mugwort extract), crude drugs (for example, moxa) , perfumes (for example, lavender and rosemary), slimming agents (for example, aminophylline and tea extract), analgesic drugs (for example, indomethacin and dl-camphor), blood circulation promoters (for example, acidic mucopolysaccharide and chamomile), swelling improvers (for example, horse chestnut extract and flavone derivatives), fomentations (for example, aqueous boric acid, physiological saline, and aqueous alcohols), fat-splitting components (for example, jujube extract, caffeine, and tonalin), cosmetics (for example, aloe extracts, vitamin preparations, hormone preparations, anti-histamines, and amino acids), antibacterial agents and sterilizers (for example, carbolic acid derivatives, boric acid, iodine preparations, invert soaps, salicylic acid based substances, sulfur, and antibiotics), and mold inhibitors.

The percutaneously absorptive drug is not particularly limited so far as it has percutaneous absorption. Examples thereof include corticosteroids, anti-inflammatory drugs, hypertension drugs, anesthetics, hypnotic sedatives, tranquillizers, antibacterial substances, antifungal substances, skin stimulants, inflammation inhibitors, anti-epileptics, analgesics, antipyretics, anesthetics, mold inhibitors, antimicrobial antibiotics, vitamins, antiviral agents, swelling improvers, diuretics, antihypertensives, coronary vasodilators, anti-tussive expectorants, slimming agents, anti-histamines, antiarrhythmic agents, cardiotonics, adrenocortical hormones, blood circulation promoters, local anesthetics, fat-splitting components, and mixtures thereof. However, it should not be construed that the invention is limited thereto. These drugs are used singly or in admixture of two or more kinds thereof as the need arises.

The content of such a functional substance is not particularly limited so far as it falls within the range where the effect of a medicine can be expected. However, from the viewpoints of adhesive strength as well as pharmacological effect and economy, the content of the functional substance is preferably from 0.01 to 25 parts by weight, and more preferably from 0.5 to 15 parts by weight based on 100 parts by weight of the adhesive.

Furthermore, a method for providing the adhesive layer is not limited so far as a thermal packaging body for joint surroundings can be fixed. The adhesive layer may be entirely provided or partially or intermittently provided. Examples of its shape include various shapes such as a network-like shape, a stripe-like shape, a dot-like shape, and strip-like shape.

In the invention, as a heat seal material constituting a heat seal layer, a single raw material may be used, or a composite raw material having a heat seal layer may be used. The heat seal material is not limited so far as at least a part thereof can be welded upon heating. Examples thereof include hot melt based resins such as polyolefins (for example, polyethylene and polypropylene) or olefin copolymer resins, ethylene based hot melt resins (for example, ethylene-vinyl acetate copolymer resins and ethylene-acrylic acid ester copolymer resins (for example, ethylene-isobutyl acrylate copolymer resins)), polyamide based hot melt resins, butyral based hot melt resins, polyester based hot melt resins, polyamide based hot melt resins, polyester based hot melt resins, polymethyl methacrylate based hot melt resins, polyvinyl ether based hot melt resins, polyurethane based hot melt resins, polycarbonate based hot melt resins,such as polyvinyl acetate, and vinyl chloride-vinyl acetate copolymers; and films or sheets thereof. Furthermore, in these hot melt based resins or films or sheets thereof, ones having various additives (for example, an antioxidant) compounded therein can be used. In particular, low density polyethylene and polyethylene obtained by using a metallocene catalyst are useful.

In the case of interposing a heat generating composition molded body between a substrate and a covering material, the "temporary adhesion" as referred to in the invention means weak pressure-sensitive bonding or adhesion for the purpose of holding the accommodated heat generating composition molded body until at least the substrate and the covering material are adhered to each other via a sticky layer made of an adhesive and heat sealed.

Furthermore, the "deadhesion" as referred to herein means that in the temporary adhering seal part after heat seal, the heat generating composition in a non-heat sealed region is transferred to the foregoing region, thereby releasing the temporary adhesion.

The temporary adhering seal part is formed via a sticky layer. An adhesive constituting the sticky layer is not limited so far as it is a layer formed of a polymer composition which is tacky at the normal temperature and can be heat sealed after the temporary adhesion.

Furthermore, although the adhesive of the foregoing adhesive layer can be used as the adhesive constituting the sticky layer to be used for the temporary adhesion, a non-hydrophilic adhesive is preferable. As the adhesive constituting the sticky layer, one which is well compatible with the heat seal material constituting the heat seal is preferable, and a melting point of a base polymer of the adhesive is preferably not higher than a melting point of the heat seal material. In particular, hot melt based adhesives are preferable. Furthermore, in the case where the heat seal material is made of an olefin based raw material, preferred examples of the adhesive include olefin based adhesives.

Incidentally, a method for providing a sticky layer for the temporary adhesion is not limited. The sticky layer may be entirely provided or partially or intermittently provided. Examples of its shape include various shapes such as a network-like shape, a stripe-like shape, a dot-like shape, and strip-like shape.

The production process of the foot warming heat generating body of the invention will be described below in detail. The production process of the invention is a process for producing a foot warming heat generating body by a molding system, in which a heat generating composition is molded and laminated in a prescribed region of at least one place on the surface of a film-like or sheet-like substrate in a shape so as to cover an arbitrary site of the foot, and a covering material is put thereon so as to cover the molded body. More specifically, a heat generating composition is molded and laminated on a film-like or sheet-like substrate by force-through die molding, cast molding, etc., a film-like or sheet-like covering material is subsequently put thereon, and the substrate and the covering material in the periphery of the laminated heat generating composition molded body are then heat sealed, thereby obtaining a molded body. Thereafter, the molded body is punched out to produce a foot warming heat generating body.

Incidentally, a foot warming heat generating body may be produced by providing an air-permeable adhesive layer between the molded body and the covering material or by providing a non-woven fabric between the molded body and the covering material.

Furthermore, the exothermic part may be constituted of sectional exothermic parts. Its explanation is omitted because it overlaps the foregoing explanation of the foot warming heat generating body.

A preferred production process of a foot warming heat generating body in which an exothermic part thereof has sectioned sectional exothermic parts by a molding system is not limited so far as it is a molding method using a die. Examples thereof include a force-through die molding method and a cast molding method.

After the accommodating step, a foot warming heat generating body is produced through a sealing step, a cutting step, etc. The sealing step, the cutting step and the like may be properly selected and employed from conventional methods and devices.

The production process is a process for forming a foot warming heat generating body, which is characterized in that the packaging material is made of a substrate and a covering material, at least one or a part of the substrate and the covering material is permeable to air, and at least the periphery of the heat generating composition molded body is sealed.

A foot warming heat generating body may be produced by after molding a heat generating composition on a substrate or further putting a covering material thereon, passing the molded body through rolls to adjust it in a flat form and then eliminating at least a part of the substrate and the covering material, etc., or sealing the covering material and the substrate as they are and further charging in an outer bag made of an air-impermeable packaging material, followed by sealing.

Furthermore, in the production process of the invention, an adhesive layer or a non-slip layer may be formed entirely or partially on the exposed surface of either one side of the laminate.

Examples of the production process of a foot warming heat generating body of the invention will be described below in detail, but it should not be construed that the invention is limited thereto.

The production process of a foot warming heat generating body of the invention is a basic process for successively carrying out the steps including a first step which is a production step of a heat generating composition; a second step of laminating the heat generating composition in a prescribed region of at least one place on the surface of a film-like or sheet-like substrate in a form of covering an arbitrary site of the foot; and a fourth step of putting a covering material on the laminate so as to cover it.

In addition, a step selected from the following first step, second step (second A step, second B step, second C step, and second D step), third step (third A step, third B step, and third C step), fourth step (fourth A step), fifth step, sixth step (sixth A step and sixth B step), seventh step (seventh A step), eighth step, ninth step and tenth step inclusive of duplicated steps thereof can be arbitrarily mediated in the basic process, as the need arises.

First step: production step of heat generating composition

Second step: molding step (substrate and magnet), second A step: force-through die molding step (trimming die and leveling plate), second B step: cast molding method (casting die and leveling plate), second C step: force-in die molding step (trimming die and pushing plate), second D step: in-mold compression step

Third step: lamination, spraying and coating step for heat generating composition, etc., third 3A step: setting-up step of air-permeable adhesive polymer, third B step: lamination, spraying and coating step for substrate, etc., third C step: surface treatment step of heat generating composition

Fourth step: covering step (covering material), fourth A step: covering step (underlay material)

Fifth step: pressurizing step

Sixth step: sealing step, sixth A step: temporary adhesion and heat sealing step, sixth B step: deadhesion step

Seventh step: setting-up step of non-slip layer, seventh A step: setting-up step of air adjusting material

Eighth step: punching-out step of foot warming heat generating body

Ninth step: accommodating step of foot warming heat generating body in air-impermeable accommodating bag

Tenth step: punching-out step of outer bag

The foot warming heat generating body of the invention is produced by a proper combination of a first step, a second step, a second A step, a second B step, a second C step, a second D step, a third step, a third A step, a third B step, a fourth step, a fourth A step, a fifth step, a sixth step, a seventh step, an eighth step, a ninth step, and a tenth step in random order, which are employed in the invention, inclusive of duplicated steps thereof. Examples of the production process which can be constructed include a production process of carrying out the first step, the second step, the fourth A step, the fourth step, the sixth step, the eighth step, the ninth step and the tenth step in this order; a production process of carrying out the first step, the seventh step, the second step, the third A step, the fourth A step, the fourth step, the sixth step, the seventh step, the eighth step, the ninth step and the tenth step in this order; and a production process of carrying out the first step, the second step, the third A step, the fourth A step, the fourth step, the sixth step, the seventh A step, the seventh step, the eight step, the ninth step and the tenth step in this order. Incidentally, by using a substrate having a non-slip layer provided thereon, it is possible to produce a non-slip layer-provided foot warming heat generating body without providing a setting-up step of a non-slip layer in the production process.

Incidentally, the atmosphere of each step may be any of an oxygen-containing atmosphere such as air and an inert gas atmosphere such as nitrogen and argon for the purpose of preventing oxidation of an iron powder upon contact with oxygen in air. The production process may be constructed as a whole by properly employing or combining such atmospheres.

By applying a magnet and a leveling plate to this heat generating composition, not only transfer and lamination by force-through die molding, cast molding, etc. are extremely easy, but also a foot warming heat generating body in an ultra-thin form having long-term exothermic properties can be produced at a high speed. In addition, by pressurizing the laminated heat generating composition, a foot warming heat generating body in a thinner ultra-thin form having long-term exothermic properties can be produced. Moreover, since the surplus water becomes a barrier layer, the feed amount of air is reduced, whereby the exothermic reaction is substantially stopped. As a result, an exothermic loss at the time of production, a lowering of the material quality of the heat generating composition and various harmful influences caused due to flocculation of the heat generating composition are prevented, and in addition, excellent exothermic properties are revealed because of excellent draining properties. When a water absorptive polymer or the like is added and blended, an exothermic temperature characteristic exhibiting a high performance over a long period of time at the time of use is obtained because of high water retaining properties.

Next, the respective steps will be described below in detail. In the first step, first of all, prescribed amounts of an iron powder, active carbon, an oxidation promoter and water and optionally, components such as a dispersion stabilizer, a water retaining agent, a water absorptive polymer, a heat generating aid, a silicone resin, a hydrogen formation inhibitor, and an expanding agent are mixed to produce a heat generating composition. The order of charging and mixing is not particularly limited, but the following can be enumerated.

(1) The foregoing whole components are charged in a mixing machine and then uniformly mixed; (2) the foregoing respective components are successively charged in a mixing machine and then successively uniformly mixed; (3) only the solid components of the foregoing whole components are divided into some groups and successively charged; and (4) only all of the solid components are charged in a mixing machine, thereafter, these components are uniformly mixed in this mixing machine, and water or an aqueous solution or dispersion of a metal chloride is subsequently charged therein and mixed.

The mixing machine which is used in the first step of the invention is not particularly limited so far as it is able to uniformly mix the components which constitute a heat generating composition containing surplus water. Specific examples thereof include a mixing and extrusion screw, a ribbon mixer, a Spartan mixer, a roll mixer, and a Banbury mixer.

Furthermore, in producing the heat generating composition of the invention, any mixing unit is basically employable so far as it is able to mix the raw materials which constitute the heat generating composition.

The second step is a step of molding the heat generating composition as obtained in the first step in an arbitrary foot shape in a prescribed region of at least one place on a film-like or sheet-like substrate or underlay material by molding such as force-through die molding, force-in die molding, and cast molding. More specifically, the second A step and the second B step are enumerated and may be properly employed. The substrate or underlay material which is used herein is the same as that described in the foot warming heat generating body of the invention.

The "force-through die molding method" as referred to herein means a continuous formation method in which by using a molding machine for laminating a heat generating composition molded body having a trimming die shape on a longitudinal substrate by using a trimming die and a rotary seal unit capable of covering the laminate by a longitudinal covering material and heat sealing a desired sectioned part and the surroundings of the substrate and the covering material, the surroundings of the heat generating composition molded body and a necessary part of the sectioned part are heat sealed via the seal unit and subjected to a seal treatment.

The "cast molding method" as referred to herein means a molding method for laminating a heat generating composition molded body on a longitudinal substance by filling in a casting mold having a concave and transferring it into a substrate. In the continuous case, there is enumerated a continuous formation method in which by using a molding machine for laminating a heat generating composition molded body on a longitudinal substrate by filling in a concave and transferring into a substrate by a drum type body of rotation and a rotary seal unit capable of covering the laminate by a longitudinal covering material and heat sealing a desired sectioned part and the surroundings of the substrate and the covering material, the surroundings of the heat generating composition molded body and a necessary part of the sectioned part are heat sealed via the seal unit and subjected to a seal treatment.

In the second A step, by using a filling plate and a pushing roll, the heat generating composition is fed into a trimming die or a casting die having a foot shape; or by using a cylinder head equipped with a stirring unit for imparting fluidity to the heat generating composition, the heat generating composition as fed into a head is imparted with fluidity while stirring and fed into a trimming die or a casting die having a foot shape. At this time, vibration may be given to the head. A substrate, a template and a backing plate which receive them (for example, a belt of a belt conveyor) pass as one body between a leveling plate as fixedly provided downward in a slightly forward direction of the head (advancing direction of the template) and a magnet as placed beneath it. The heat generating composition is attracted onto the substrate through the die by the magnet, and at the same time, the surface of the heat generating composition is leveled along a foot part die by the leveling plate, whereby the heat generating composition is molded. Thereafter, the die is left from the substrate. The magnet is not limited so far as it has magnetism, and examples thereof include a permanent magnet and an electromagnet. Incidentally, by simplifying the stirring unit to convert it into a rotary bridge prevention unit, a bridge as generated at the time of feeding the heat generating composition into the head may be prevented. Furthermore, the "filling plate" as referred to herein means a jig for filling the heat generating composition into the die such as a leveling plate and a pushing plate.

In the second B step, a roll having a casting die with a concave is installed in the cylinder head as used in the second B step; the heat generating composition is fed into the roll from the head; the heat generating composition is pushed into the die by a filling tool such as a leveling plate, thereby leveling the surface and molding the heat generating composition; and the substrate, the template and the backing plate which receive them (for example, a belt of a belt conveyor) pass as one body between the roll and a magnet as placed beneath the roll. The heat generating composition is attracted onto the substrate through the die by the magnet, and at the same time, the surface of the heat generating composition is leveled along a die by the leveling plate, whereby the heat generating composition is molded. Thereafter, the heat generating composition within the die is transferred into the substrate by the magnet. The magnet is not limited so far as it has magnetism, and examples thereof include a permanent magnet and an electromagnet. Incidentally, by simplifying the stirring unit to convert it into a rotary bridge prevention unit, a bridge as generated at the time of feeding the heat generating composition into the head may be prevented.

In the second C step, molding such as force-in die molding, force-through die transfer, and lamination is carried out while giving vibration. The measure for giving vibration is not limited so far as it is able to cause vibration in the heat generating composition of the invention. Examples thereof include usually used vibration units using an eccentric motor, a piezoelectric element or air, etc.

In this case, pushing of the heat generating composition using a press plate may be carried out. The press plate is not limited so far as it is able to push the heat generating composition of the invention into a die. Examples thereof include spring plates which are made of a plastic such as acrylic resins, vinyl chloride resins, and polyethylene, a metal such as iron and stainless steel, or a composite thereof.

In the second D step, the heat generating composition within the die is compressed by using a sponge roll, a rubber roll, a die compression roll, etc. Any measure may be employed so far as the heat generating composition within the die can be compressed.

In the second step, this heat generating composition may be laminated in one place or two or more places in the width direction on the surface of the substrate, or may be laminated in a cross-stitch form in the longitudinal direction of the substrate.

The third step is a step of laminating or spraying at least one member selected from an iron powder, a carbon component, a ceramic powder capable of radiating far infrared rays, a fibrous material capable of radiating far infrared rays, a pyroelectric substance, a minus ion emitting substance, an aggregate, an organosilicon compound, a water absorbing agent, a binding agent, a thickener, an excipient, a flocculant, a soluble sticky raw material, a water absorptive polymer, and a netlike polymer onto the molded heat generating composition, the substrate or the underlay material.

The third step is a step of laminating or spraying at least one member selected from an iron powder, a carbon component, a ceramic powder capable of radiating far infrared rays, a fibrous material capable of radiating far infrared rays, a pyroelectric substance, a minus ion emitting substance, an aggregate, an organosilicon compound, a water absorbing agent, a binding agent, a thickener, an excipient, a flocculant, a soluble sticky raw material, a water absorptive polymer, and a netlike polymer in a prescribed region of at least one place on the molded heat generating composition laminated on the film-like or sheet-like substrate or covering material.

The third A step is a step of providing a netlike polymer in at least one member or a part selected from the substrate, the covering material and the laminated heat generating composition. This is achieved by a usual processing technology such as melt blow, printing, and coating. In this way, the laminate of the heat generating composition of the invention can be more strongly fixed to the substrate and/or the underlay material and/or the covering material. In addition, when the polymer has stickiness, the substrate and/or the underlay material and/or the heat generating composition and/or the covering material is stuck due to this viscosity.

The third B step is a step of laminating or spraying at least one member selected from an iron powder, a carbon component, a ceramic powder capable of radiating far infrared rays, a fibrous material capable of radiating far infrared rays, a pyroelectric substance, a minus ion emitting substance, an aggregate, an organosilicon compound, a water absorbing agent, a binding agent, a thickener, an excipient, a flocculant, a soluble sticky raw material, and a water absorptive polymer onto the substrate and/or the underlay material and/or the covering material.

The fourth step is a step of putting a film-like or sheet-like covering material on the heat generating composition molded body of the invention so as to cover it.

The fifth step is a step of adjusting the shape of the heat generating composition molded body by subjecting the heat generating molded body compression, flattening, etc. using a press roll, etc. That is, a desired pressure is applied to the heat generating composition molded body by a press roll, etc. to adjust the shape, thereby improving the shape holding properties.

In particular, a method for forming the heat generating composition into a sheet is not particularly limited so far as it is able to form the heat generating material into a sheet, and examples thereof include a method of using a rolling device of a one-stage press roll system by carrying out rolling once or repeating rolling plural times by a one-stage press roll and a method of using a rolling device of a multi-stage press roll system by carrying out a single rolling step plural times by a multi-stage press roll.

In this case, when it is impossible to form a sheet by one-time rolling depending upon the formulation of the heat generating composition, or when change of the thickness or realization of a high density of the heat generating sheet is required, the pressing may be carried out plural times. On this occasion, the pressure may be increased stepwise.

Then, by performing pressing by a press roll so as to contact bond the heat generating composition, thereby molding a heat generating sheet for foot and winding up this heat generating sheet in a rolled state, the preservability and the delivery properties and further, the processability and so on may be improved. In this case, by repeating the pressurization of the heat generating sheet for foot and winding plural times, the density of the heat generating sheet for foot and inflow properties may be adjusted.

The sixth step is a step of sealing the periphery of the heat generating composition molded body adaptive to a prescribed foot shape. The sealing includes heat sealing using a hot melt based bonding agent layer and contact bond sealing using a hot melt based adhesive layer. The heat sealing is not limited so far as heat sealing can be achieved. In general, examples thereof include the case where the heat sealing is performed by one group of one pair of heat seal rolls and the case where the heat sealing is performed by using multi-stage heat seal rolls resulting from connecting two groups or three groups of one pair of heat seal rolls. In the one pair of rolls, the temperature may be the same, or the temperature of one roll may be different from that of the other roll. Furthermore, the surface of one heat seal roll may be plain, the cross-sectional shape may be unevenly patterned, or a pattern mixture of a plain surface and an uneven cross-sectional shape may be employed. The "pattern mixture" as referred to herein means that the inside of the seal part is plain, whereas the outside is patterned; or that the inside of the seal part is patterned, whereas the outside is plain or partially patterned and partially plain. By combining these heat seal rolls, a seal part in which the pattern is different between the back and front surfaces may be provided, or a seal part in which the back surface is plain, whereas the front surface is patterned may be provided. Furthermore, the contact bond sealing is the same as in the case of heat sealing, except that the roll is not heated to a temperature higher than the melting point of the seal layer and that a hot melt based adhesive layer and a contact bond seal are used. However, even in the contact bond sealing, since the fluidity of the adhesive layer is increased at the time of contact bonding, warming can be performed within the range not exceeding the meting point of the seal layer. The underlay material and the covering material which are used herein are the same as those described in the foot warming heat generating body of the invention.

The sixth A step is a step of performing temporary adhesion and heat sealing by temporary adhesion by using a temporary adhering roll and using a sticky layer as provided on the substrate, the covering material, and the like upon pressurization and subsequent heat sealing by a heat seal roll. This step may be a step composed of only heat sealing while omitting the temporary adhesion.

The sixth B step is a deadhesion step by moving the heat generating composition (molded body) in a portion which is a non-heat seal part of the temporary adhering seal part into the temporary adhering part region by using a pushing roll, thereby deadhering the temporary adhering region.

The seventh step is a step of setting-up a non-slip layer. The raw material which constitutes the non-slip layer is not limited so far as it has a non-slip effect. Examples thereof include polyethylene as produced using a metallocene catalyst and a styrene based hot melt based adhesive such as SIS. These materials may be provided as a film, a sheet or a resin layer. The shape of the non-slip layer is not limited. Examples thereof include a network shape, a dotted shape, a band-like shape, an entire surface shape, a cross-stitch shape, a checkerwork shape, an oblique lattice shape, a vertical linear shape, and a horizontal linear shape. The non-slip layer includes an adhesive layer. If desired, the non-slip layer may be provided with a separator.

The seventh 7A step is a step of setting up an air permeability adjusting material for providing the air permeability adjusting material in the sectional exothermic parts and the sectioned parts. For example, a film-like or sheet-like raw material having a bonding layer constituted of an adhesive on one surface thereof is stuck onto a part or the whole of an exothermic part composed of sectional exothermic parts and sectioned parts. A region where the bonding layer occupies is not limited, and examples thereof include the both end parts of the raw material, the entire surface exclusive of the central part, a lattice shape, and a stripe-like shape.

The eighth step is a step of punching out the molded body in a prescribed foot shape while retaining a seal width. This step of punching out the molded body in a prescribed foot shape may be carried out by stopping moving of the molded body, namely it may be carried out intermittently, or it may be carried out continuously by using a cutter roll, etc. In this case, by simultaneously punching out the surroundings of plural molded bodies as disposed in the feed direction of the molded body and/or in the width direction orthogonal thereto, it is possible to form plenty of foot warming heat generating bodies at once. As a result, it is possible to design to reduce the costs. In this case, in the molding step, plural heat generating composition molded bodies are molded adaptive thereto. Furthermore, in the case of continuously punching out a molded body, since it is possible to continuously work the molded body production and punching-out consistently, plenty of foot warming heat generating bodies can be completed within a short period of time. As a result, as compared with the punching-out method by stopping moving of the molded body, it is possible to design to reduce the costs very largely.

The ninth step is a step in which a foot warming heat generating body is mediated between two films or sheets, and simultaneously with or after this mediation, in the surroundings of the foot warming heat generating body, the two films or sheets are sealed in a size exceeding the size of the foot warming heat generating body. That is, this step is a step of sealing the foot warming heat generating body into an outer bag made of an air-impermeable film. Here, with respect to the matter that the two films or sheets are sealed in a size exceeding the foot warming heat generating body, there is no particular limitation so far as the size is equal to or larger than the size the subject foot warming heat generating body. In particular, it is preferred to cut and mold the laminate into a large-sized rectangular shape as extended over the whole surrounding in a width of from several mm to about 20 mm larger than a rectangular shape of a maximum width and a maximum length of the shape of the foot warming heat generating body. Furthermore, the shape may be similar or substantially similar to the shape of the foot warming heat generating body. Furthermore, in the case of simultaneously sealing plural laminates in the feed direction of the laminate and/or in the width direction orthogonal thereto, the laminate is sealed interlocking with the preceding step.

The tenth step is a step of punching out the outer bag having a foot warming heat generating body sealed therein while retaining a seal width simultaneously with or after sealing. Thereafter, the foot warming heat generating body as sealed in an outer bag is avoided from the contact with air, stored and then provided for distribution.

Here, the ninth step and the tenth step may be combined, thereby mediating a foot warming heat generating body between two films and sheets, sealing the two films or sheets in a size exceeding the size of the foot warming heat generating body by heat sealing simultaneously with or after this mediation, and simultaneously punching out the laminate while retaining a seal part.

Here, all of the steps may be worked intermittently or continuously. According to the continuous work, it is possible to form plenty of foot warming heat generating bodies per unit time. As a result, it is possible to design to reduce the costs.

Here, with respect to the production process of a foot warming heat generating body, as one example of a continuous production process of a full foot warming heat generating body in which the first step, the second step, the fourth A step, the fourth step, the sixth step, the eighth step, the ninth step and the tenth step are carried out in this order, a heat generating composition is laminated in a full foot shape on a rolled film-like or rolled sheet-like substrate having a non-slip layer made of polyethylene as produced by using a metallocene catalyst while feeding the substrate at 50 m/min by a force-through die molding method, thereby obtaining a heat generating molded body in a full foot shape; a rolled film-like or rolled sheet-like covering material is then put on the heat generating molded body by a method for guiding the covering material thereonto by a roll; the periphery of the heat generating composition molded body is subsequently heat sealed by a seal roll; and the laminate is further punched out into a full foot shape by using a cut roll while retaining the heat seal part, thereby continuously obtaining a full foot warming heat generating body. In addition, the full foot heat generating body is mediated between two films or sheets, and simultaneously with or after this mediation, in the periphery of the foot warming heat generating body, the two films or sheets are sealed in a size exceeding the size of the foot warming heat generating body. Next, simultaneously with or after sealing, an outer bag having the foot warming heat generating body sealed therein is punched out while retaining a seal width, thereby obtaining an outer bag having foot warming heat generating body sealed therein. Incidentally, by sealing every right and left foot warming heat generating bodies in a full foot shape in an outer bag, when the outer bag is opened at the time of use, foot warming heat generating bodies in a pair of full foot shapes are obtainable, and therefore, such is convenient.

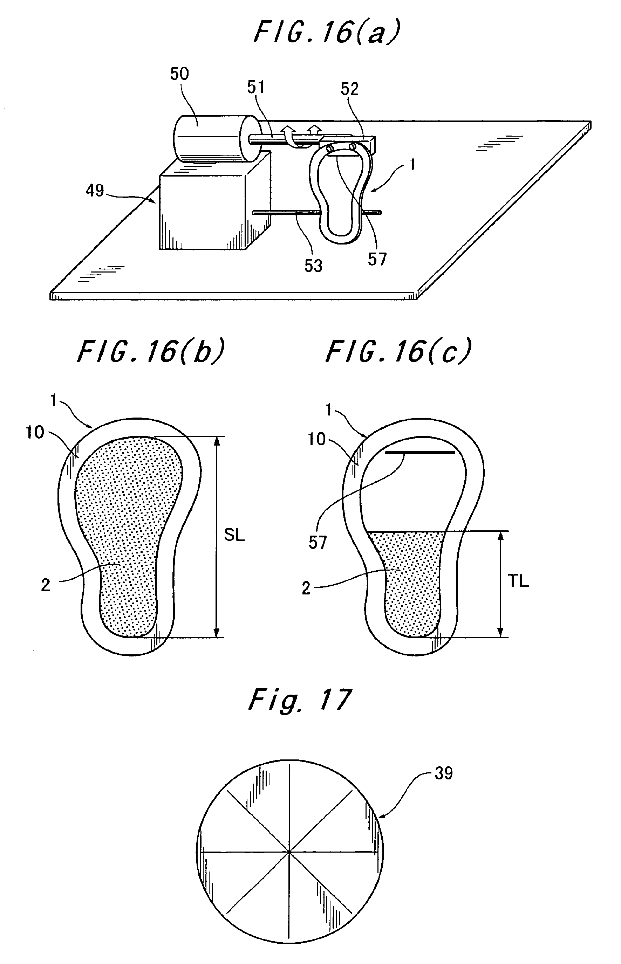

The "shape retaining degree" as referred to herein is subjective to a single independent foot warming heat generating body in which the whole surrounding of a heat generating composition is sealed, tested and calculated. In the case where plural independent foot warming heat generating bodies are present, the shape retaining degree is defined as an arithmetic mean of degrees of shape holding of the respective independent foot warming heat generating bodies. It will be described with reference to Fig. 16. First of all, a foot warming heat generating body 1 to be measured is placed in a horizontal place; and after confirming that a heat generating composition 2 is substantially uniformly present in an exothermic part, a length SL of the longest part of the exothermic part is measured. In the case where the heat generating composition is non-uniformly present, it is made uniform. Next, as shown in Fig. 16 (a), the foot warming heat generating body 1 is fixed to a fixing plate 52 as fixedly bonded to a freely rotatable rotary axis 51 by a drive unit 50 of a testing machine 49. Incidentally, the fixing portion is defined to be an upper tip part of a covering material where the heat generating composition 2 of the foot warming heat generating body 1 is not present. Next, a cut 57 having a length of 10 mm is provided in the position of 5 mm beneath from the upper tip of the exothermic part on the air-permeable surface of the covering material, thereby making the pressure of the heat generating composition 2 equal to an outer pressure (see Fig. 16 (b)). Thereafter, the fixing plate 52 is subjected to a rotational reciprocating motion at a rate of one reciprocation/sec at a movement angle of 60° by the rotation of the rotary axis 51, and the foot warming generating body 1 is subjected to a pendulum motion corresponding thereto. At that time, at least a part of the exothermic part is made to hit a sample beating member 53. After ten reciprocations, a longest length TL of the heat generating composition 2 in the vertical direction in a region where the heat generating composition of the exothermic part occupies is measured in the state that the foot warming heat generating body is installed in the fixing plate 52 (see Fig. 16(c)).

Here, a shape retaining degree (K) is defined as follows.

In the case of a foot warming heat generating body made of a single independent foot warming heat generating body:

$$K = 100 \times TL/SL \qquad\qquad (1)$$

K: Shape retaining degree
SL: Longest length of the heat generating composition of the exothermic part at the horizontal time before providing a cut
TL: Longest length of the heat generating composition of the exothermic part in the vertical plate direction after the test
In the case of a foot warming heat generating body made of two or more plural independent exothermic parts or sectional exothermic parts:

$$Km = (K1 + K2 + \bullet\bullet\bullet\bullet\bullet + Kn)/n$$

Kn: Shape retaining degree of each independent foot warming heat generating body as determined from the expression (1)
K is usually 70 or more, preferably 80 or more, and more preferably from 90 to 100. Incidentally, in the case where the foot warming heat generating body is a foot warming heat generating body made of two or more plural independent exothermic parts or sectional exothermic parts, heat generating compositions present in all of the independent exothermic parts or sectional exothermic parts which constitute the foot warming heat generating body are subjective, and a number average value of degrees of shape holding of the respective independent exothermic parts or sectional exothermic parts is usually 70 or more, preferably 80 or more, and more preferably from 90 to 100. In this way, it is possible to realize uniform warmth taking and comfortable use without causing uneven distribution of the heat generating composition such as divergence and deviation at the time of use.

The "water mobility value" as referred to herein is a value showing an amount of surplus water which can transfer to the outside of the heat generating composition in water present in the heat generating composition. This water mobility value will be described below with reference to Figs. 17 to 21.

As shown in Fig. 17, a filter paper 39 of No. 2 (second class of JIS P3801) in which eight lines are drawn radiating from the central point with an interval of 45° is placed on a stainless steel plate 43 as shown in Figs. 18 and 19; a template 40 having a size of 150 mm in length x 100 mm in width and having a hollow cylindrical hole 41 having a size of 20 mm in inner diameter x 8 mm in height is placed in the center of the filter paper 39; a sample 42 is placed in the vicinity of the hollow cylindrical hole 41; and a stuffer plate 38 is moved on and along the template 40 and inserted into the hollow cylindrical hole 41 while stuffing the sample 42, thereby leveling the sample (force-in die molding).

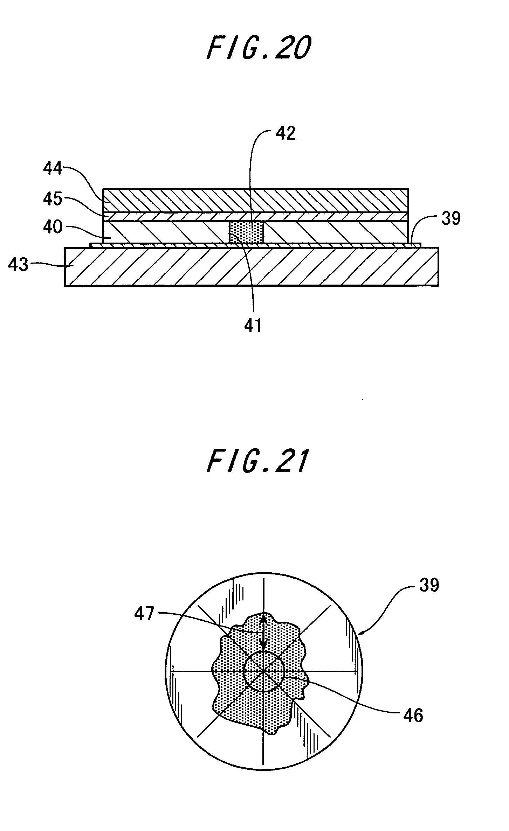

Next, as shown in Fig. 20, a non-water absorptive 70 $\mu$m-thick polyethylene film 45 is placed so as to cover the hole 41, and a flat plate 44 made of stainless steel having a size of 5 mm in thickness x 150 mm in length x 150 mm in width is further placed thereon and held for 5 minutes such that an exothermic reaction is not caused.

Thereafter, a shown in Fig. 21, the filter paper 39 is taken out, and an oozed-out locus of the water or aqueous solution is read as a distance 47 (unit: mm) from a periphery 46 as an edge of the hollow cylindrical hole to an oozed-out tip along the radiating lines. Similarly, a distance 47 from each of the lines is read, and eight values in total are obtained. Each of the eight values (a, b, c, d, e, f, g and h) which are read out is defined as a measured water content value. An arithmetic average value of the eight measured water content values is defined as a water content value (mm) of the sample.

Furthermore, the water content for the purpose of measuring a real water content value is defined as a compounded water content of the heat generating composition corresponding to the weight of the heat generating composition having a size of 20 mm in inner diameter x 8 mm in height or the like, similar measurement is conducted only with water corresponding to that water content, and a value as calculated in the same manner is defined as a real water content value (mm). A value obtained by dividing the water content value by the real water content value and then multiplying with 100 is a water mobility value.

That is, the water mobility value is represented by the following expression.

$$(\text{Water mobility value}) = \{[\text{Water content value (mm)}]/$$

$$[(\text{Real water content value (mm)})] \times 100$$

With respect to the same sample, five points are measured, and the five water mobility values are averaged, thereby defining an average value thereof as a water mobility value of the sample.

In the invention, a heat generating body can be formed only by laminating a heat generating composition molded body obtained by molding a heat generating composition having surplus water with a water mobility value of from 0.01 to 20 on a substrate, covering a covering material thereon, and sealing at least the periphery of the heat generating composition molded body. After accommodating it in a packaging material such as a substrate and a covering material, it is not necessary to add water. Accordingly, since the process is remarkably simplified, the invention is superior in view of the costs.

In the invention, the water mobility value (0 to 100) is preferably from 0.01 to 20, more preferably from 0.01 to 18, further preferably from 0.01 to 15, still further preferably from 0.01 to 13, even further preferably from 1 to 13, and even still further preferably from 3 to 13.

In a heat generating body using a heat generating composition molded body obtained by molding a moldable heat generating composition containing surplus water as a connecting substance according to the invention, the heat generating composition contains an appropriate amount of surplus water expressed by a water mobility value of from 0.01 to 20 as the connecting substance without using a flocculant aid, a dry binding agent, a flocculating agent, etc.

It is assumed that when the amount of surplus water in the heat generating composition is appropriate, the surplus water causes hydration against hydrophilic groups in the components of the composition due to a bipolar mutual action or hydrogen bond, etc. and that it is present even in the surroundings of hydrophobic groups while having high structural properties. Thus, it is assumed that the heat generating composition becomes in a state of a mud ball, thereby revealing moldability. This is connecting water as a connecting substance in some meaning. Besides, there is water in a state called as free water which can freely move, and it is thought that when the surplus water increases, the structure is softened, whereby the free water increases. Furthermore, controlling factors which an iron powder causes an oxidation reaction are an amount of existing water and a feed amount of oxygen to the surface of the iron powder. It is said that in a degree of water adsorbing film (less than 100 angstroms), the water is not sufficient and that the oxidation rate is small. When the adsorbing film becomes about 1 $\mu$m, the water content becomes sufficient. Furthermore, since the thickness of the water film is thin, feed of oxygen onto the surface of the iron powder becomes easy, whereby the oxidation rate becomes large. It is assumed that when the film becomes thicker to an extent that the adsorbing film exceeds 1 $\mu$m, the feed amount of oxygen is reduced. The present inventors have obtained knowledge that the water mobility value expressing the optimal water content at which moldability and oxidation rate in fixed levels or more are revealed is from 0.01 to 20, leading to accomplishment of the invention.

That is, by using an appropriate amount of surplus water, the respective component particles are coupled with each other by a surface tension of water, moldability is generated in the heat generating composition, and the water does not substantially function as a barrier layer. Thus, the heat generating composition comes into contact with air to generate heat. In addition, by using a heat generating composition using an active iron powder or an active heat generating composition using an active iron powder, the heat generating composition becomes a heat generating composition having remarkably excellent exothermic rising properties and high moldability. Furthermore, heat generation occurs without causing transfer of the water in the heat generating composition molded body as produced by a molding and laminating system into a packaging material or water absorptive sheet. In addition, by providing plural sectional exothermic parts of the heat generating composition molded body as sectioned by seal parts, it is possible to provide a heat generating body which has flexibility itself, is excellent in installation in places where flexibility is required, such as various places of a human body and curved bodies, and is excellent in feeling for use.

Furthermore, in the substrate, the covering material and the heat generating composition molded body, by temporarily adhering at least the covering material and the heat generating composition molded body to each other via a sticky layer and then heat sealing the periphery of the heat generating composition molded body and the surroundings of the heat generating body, certainty of heat seal is improved so that it becomes possible to design to make the production speed of a heat generating body high and make the heat seal width small.

The "moldability" as referred to in the invention exhibits that a molded body of the heat generating composition having a cavity or concave die shape is formed by force-through molding using a trimming die having a cavity or cast molding using a concave die, whereby after molding including mold release, the molding shape of the heat generating composition molded body is held.

When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, the sealing can be achieved without causing cutting in seal. The presence of the spots causes insufficient sealing.

1) Measurement device:
With respect to the measurement device, a stainless steel-made molding die (a plate having a size of 2 mm in thickness x 200 mm in length x 200 mm in width and having a cavity as treated by R5 in four corners of 60 mm in length x 40 mm in width in a central part thereof) and a fixable leveling plate are disposed above a travelable endless belt, and magnets (two magnets having a size of 12.5 mm in thickness x 24 mm in length x 24 mm in width are disposed in parallel) are disposed under the endless belt.

The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness x 200 mm in length x 200 mm in width is placed on the endless belt of the measurement device, a polyethylene film having a size of 70 μm in thickness x 200 mm in length x 200 mm in width is placed thereon, and a stainless steel-made molding die is further placed thereon.

Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1.8m/min. After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 μm is not present and the number of collapsed pieces of the heat generating composition molded body having a maximum length of from 300 to 800 μm is not more than 5, it is to be noted that the heat generating composition has moldability.

The moldability is an essential property for a heat generating composition to be used in the molding system. If the heat generating composition does not have moldability, it is impossible to produce a heat generating body by the molding system.

The "perforation" as referred to in the invention includes one which is intermittently cut for improving flexural properties of the sectioned part and one which is intermittently cut such that cutting by hand is possible. Its degree is not limited but is determined depending upon the desire. The perforation may be provided in all sectioned parts or may be partially provided. The shape is not particularly limited, and examples thereof include a circle, an ellipse, a rectangle, a square, and a cut line (linear shape). For example, in the perforation which is intermittently cut such that cutting by hand is possible, a circular hole having an aperture of from φ10 to 1,200 μm can be enumerated. The aperture of the hole is more preferably from φ20 to 500 μm. When the aperture of the hole becomes φ20 μm or less, cutting properties by hand may possibly be deteriorated due to an increase of the cutting strength of the film, or breakage or fray on the cut surface tends to be generated; and when the aperture of the hole is less than φ10 μm, such a tendency especially becomes remarkable, and therefore, such is not preferable. On the other hand, when the aperture of the hole becomes φ500 μm or more, shape destruction such as breakage may possibly be introduced due to a lowering of the cutting strength, and stability tends to be lowered due to a lowering of the workability or line aptitude at the time of production, oozing, or vaporization and volatilization; and when the aperture of the hole exceeds φ1,200 μm, such a tendency especially becomes remarkable, and therefore, such is not preferable.

It is preferable that the holes are positioned lined up in the length and width. Furthermore, a shortest space between outer peripheries of the adjacent holes in the length and width is preferably from 10 to 2,000 μm, more preferably from 10 to 1, 500 μm, further preferably from 20 to 1, 000 μm, still further preferably from 20 to 500 μm, and even further preferably from 20 to 200 μm. When the shortest space between outer peripheries of the adjacent holes in the length and width is less than 10 μm, shape destruction such as breakage may possibly be introduced due to a lowering of the cutting strength and a lowering of the workability or line aptitude at the time of production is found, and therefore, such is not preferable. On the other hand, when the shortest space between outer peripheries of the adjacent holes in the length and width exceeds 2,000 μm, cutting properties by hand may possibly be deteriorated due to an increase of the cutting strength of the film and breakage or fray on the cut surface tends to be generated, and therefore, such is not preferable. That is, the cutting properties by hand are remarkably improved by a balance between the aperture of the processed hole and the shortest space of outer peripheries of the adjacent holes in the length and width.

The hole may be a cut line, and its length may be a length corresponding to the aperture. A shortest space between ends of the adjacent cut lines in the length and width is corresponding to the shortest space between outer peripheries of the adjacent holes.

For example, an aperture of the hole of from φ10 to 2, 000 μm is corresponding to a length of from 10 to 2,000 μm, and a shortest space between outer peripheries of the adjacent holes in the length and width of from 10 to 2,000 μm is corresponding to a shortest space between ends of the adjacent cut lines in the length and width of from 10 to 2,000 μm.

The "seal strength at 60°C" as referred to herein means an average value of respective maximum values obtained by subjecting three samples to a measurement by taking a specimen of 25 mm x 250 mm from a place of a subjective sealed sample to be measured for the seal strength, allowing the specimen to stand under circumstances at 60°C for 5 minutes, grasping it under circumstances at 60°C, and measuring a maximum strength at intervals of 10 mm and at a tensile speed of 300 mm/min.

Here, the seal strength under circumstances at 20°C is a value under the same conditions as in the seal strength at 60°C, except that the measurement circumstance temperature is 20°C.

The seal strength of the temporary adhering part is preferably 0.5 kg/25 mm or more, more preferably from 0.5 to 1 kg/25 mm, further preferably from 0.5 to 0.9 kg/25 mm, and still further preferably from 0.5 to 0.8 kg/25 mm under circumstances at 20°C; and the seal strength at 60°C is preferably less than 0.8 kg/25 mm, more preferably 0.01 kg/25 mm or more but less than 0.8 kg/25 mm, further preferably 0.01 kg/25 mm or more but less than 0.5 kg/25 mm, and still further preferably 0.01 kg/25 mm or more but less than 0.4 kg/25 mm.

The sticky layer of the temporary adhering part is constituted of an adhesive, has a seal strength at 60°C of from 0.01 to 0. 8 kg/25 mm, is able to stop the movement of the heat generating composition molded body between the substrate and the covering material, and makes it possible to achieve high-speed heat seal. In addition, if desired, warming may be carried out at the time of temporary adhesion. It is preferable that the warming is carried out under pressure at a temperature of not higher than a melting point of a base polymer in a hot melt based adhesive for forming the adhesive layer.

The seal strength under circumstances at 20°C of the heat seal part which has been heat sealed after the temporary adhesion is preferably 1.0 kg/25 mm or more, more preferably 1.2 kg/25 mm or more, further preferably 1.5 kg/25 mm or more, and still further preferably from 1.5 to 3 kg/25 mm. Furthermore, the seal strength at 60°C under circumstances at 60°C is preferably 0.8 kg/25 mm or more, more preferably 1.0 kg/25 mm or more, further preferably 1.2 kg/25 mm or more, and still further preferably 1.5 kg/25 mm or more.

The core material is not limited so far as it is able to prevent the foot warming heat generating body from occurrence of twist, ply, bending, overlap, and so on at the time of use. Examples thereof include papers, plastics and rubbers each having stiffness. With respect to the method for setting up the core material, though the core material may be used alone, a rubber having both functions as a non-slip layer and a core material may be used, or the core material may be incorporated as a constitutional member into the substrate or the covering material. Also, the non-slip layer may be made of a plastic or a rubber having stiffness. Stiffness may arbitrary selected depending on the position of the foot to be applied.

The invention will be specifically described below with reference to the Examples, but it should not be construed that the invention is limited thereto.

[Brief Description of the Drawings]

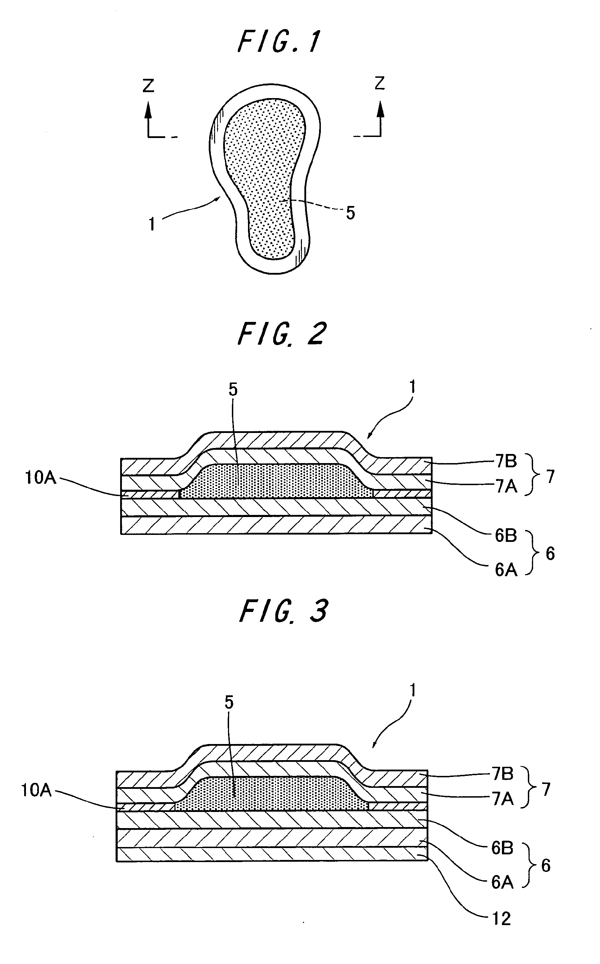

Fig. 1 is a plan view of an embodiment of the foot warming heat generating body of the invention.
Fig. 2 is a cross-sectional view along the line Z-Z of the same.
Fig. 3 is a cross-sectional view of other embodiment of the foot warming heat generating body of the invention.

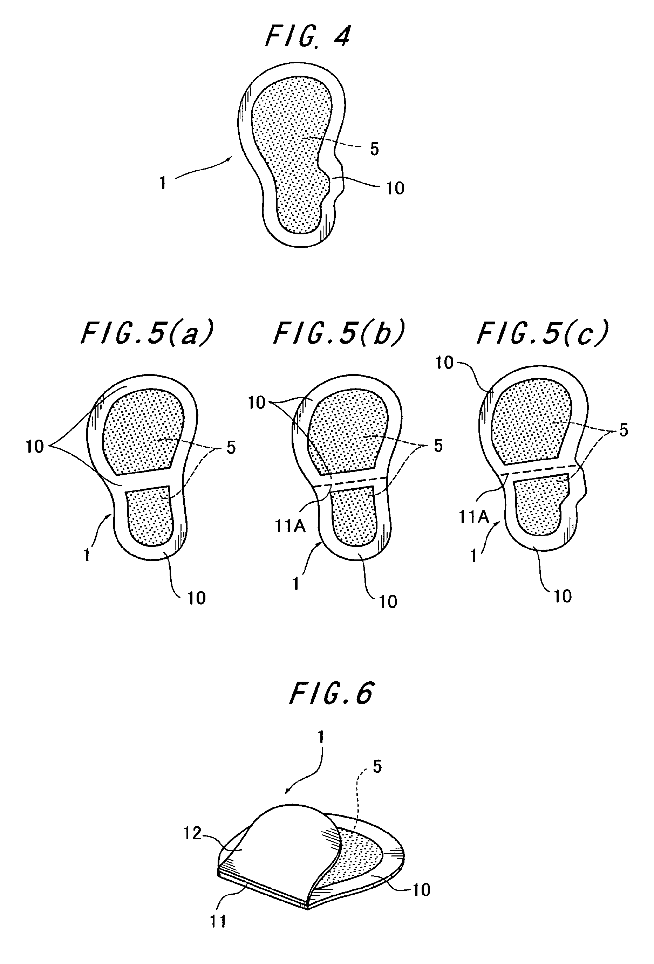

Fig. 4 is a plan view of other embodiment of the foot warming heat generating body of the invention.
Figs. 5(a), 5(b) and 5(c) are each a plan view of other embodiment of the foot warming heat generating body of the invention.
Fig. 6 is an oblique view of other embodiment of the foot warming heat generating body of the invention.

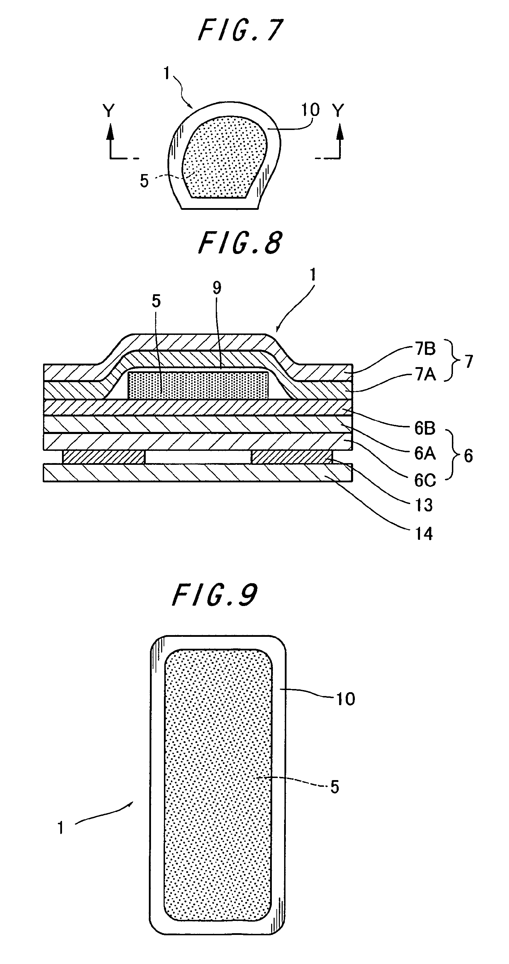

Fig. 7 is a plan view of other embodiment of the foot warming heat generating body of the invention.
Fig. 8 is a cross-sectional view along the line Y-Y of the same.
Fig. 9 is a plan view of other embodiment of the foot warming heat generating body of the invention.

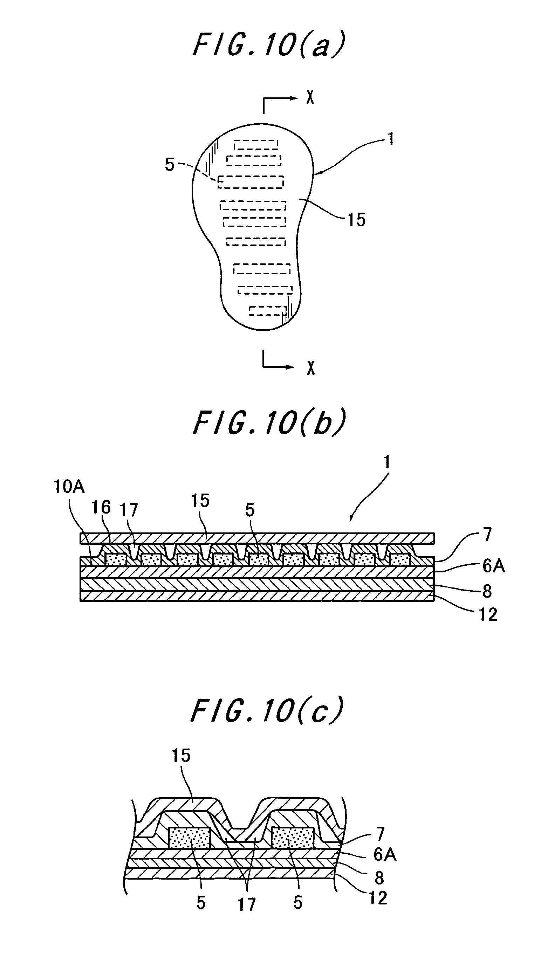

Fig. 10 (a) is a plan view of other embodiment of the foot warming heat generating body of the invention; Fig. 10 (b) is a cross-sectional view along the line X-X of the same; and Fig. 10 (c) is an enlarged cross-sectional view of other embodiment using an air permeability adjusting material.

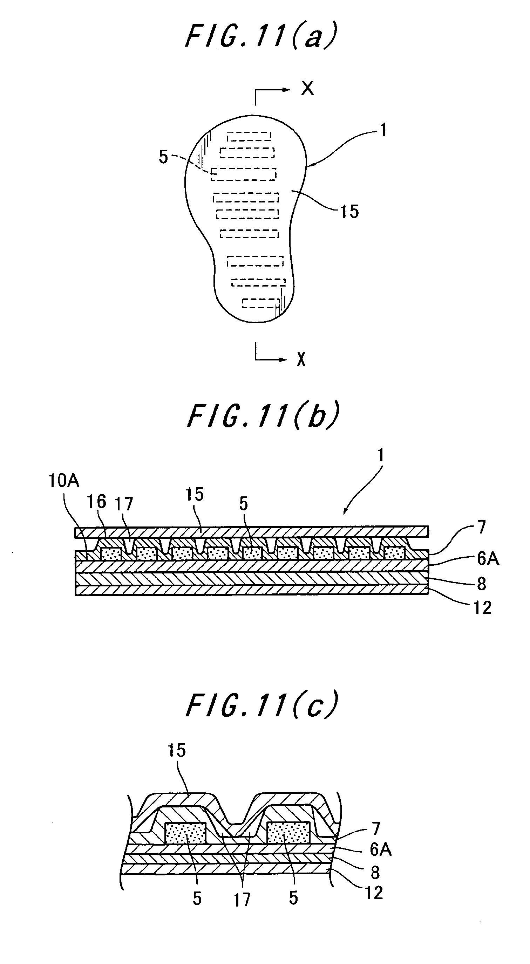

Fig. 11 (a) is a plan view of other embodiment of the foot warming heat generating body of the invention; Fig. 11(b) is a cross-sectional view along the line X-X of the same; and Fig. 11 (c) is an enlarged cross-sectional view of other embodiment using an air permeability adjusting material.

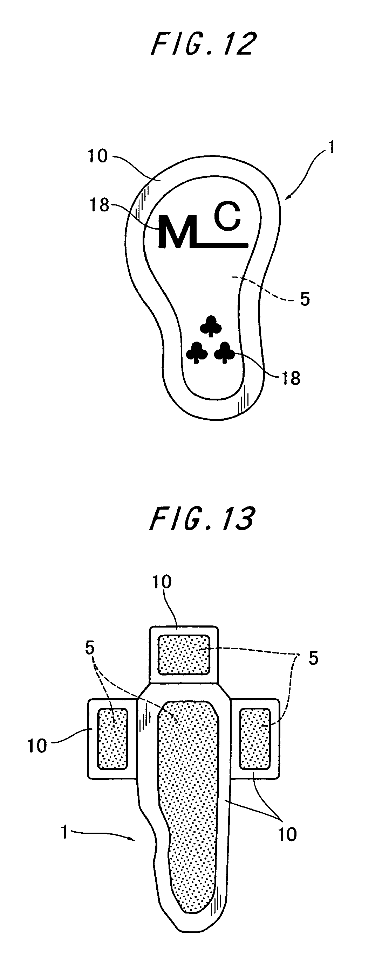

Fig. 12 is a plan view of other embodiment of the foot warming heat generating body of the invention.
Fig. 13 is a plan view of other embodiment of the foot warming heat generating body of the invention.

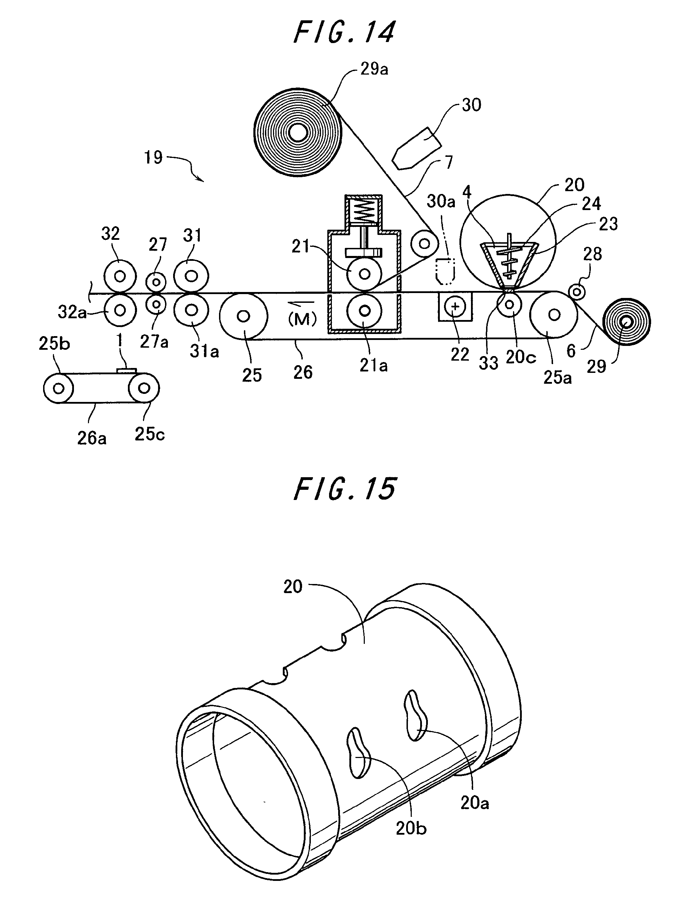

Fig. 14 is an explanatory view of the production process of the foot warming heat generating body of the invention.
Fig. 15 is an explanatory view to show one example of a foot shape molding drum for producing the foot warming heat generating body of the invention.
Fig. 16 is an explanatory view to show a measurement method of a shape retaining degree in the invention.
Fig. 17 is a plan view of a filter paper for the measurement of water mobility value in the invention.

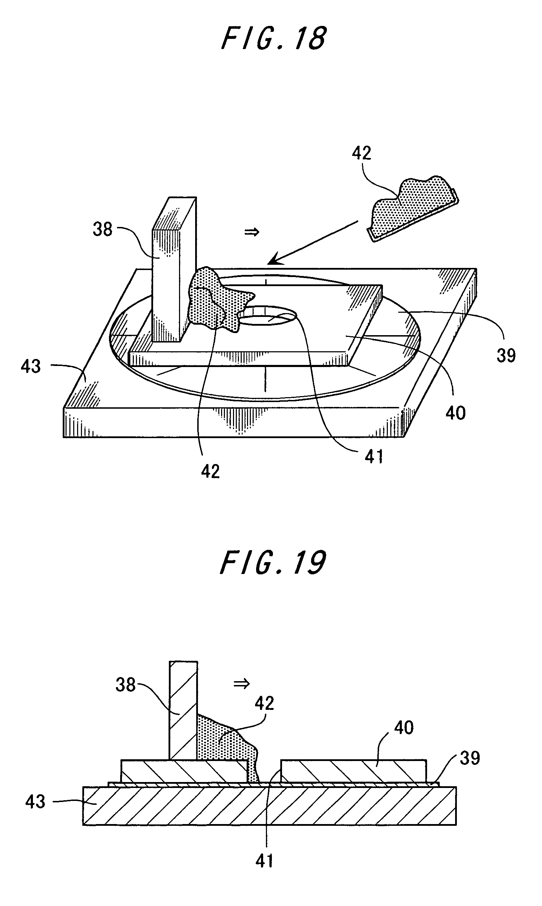

Fig. 18 is an explanatory view to show the measurement method of water mobility value in the invention.
Fig. 19 is an explanatory view to show the measurement method of water mobility value in the invention.
Fig. 20 is an explanatory view to show the measurement method of water mobility value in the invention.
Fig. 21 is a plan view of a filter paper after carrying out the measurement of water mobility value in the invention.

[Description of Reference Numerals and Signs]

1: Foot warming heat generating body
5: Heat generating composition molded body
6: Substrate
7: Covering material
9: Netlike hot melt sticky layer
10: Seal part
11: Bending part
11A: Bendable break line part (perforation)
12: Non-slip layer
13: Adhesive layer
14: Separator
15: Air permeability adjusting material
16: Sticky layer
17: Spacial part
18: Design, etc.
19: Production device of foot warming heat generating body
20: Drum type molding unit (drum having a desired shape punched therein)
23: Hopper
24: Screw
26: Belt conveyor
26a: Belt conveyor
27: Flattening roll
28: Substrate press roll
29: Feed roll
29a: Feed roll
30: Melt blow machine
30a: Melt blow machine
31: Die roll for sealing (heat roll or contact bond roll)
31a: Die roll for sealing (heat roll or contact bond roll)
32: Die cut roll
38: Pushing plate
39: Filter paper in which eight lines are drawn radiating from the central point with an interval of 45°
40: Template having a hollow cylindrical hole
41: Hollow cylindrical hole
42: Sample or water
43: Stainless plate
44: Flat plate
45: Non-water absorptive film (for example, a
polyethylene film)
46: Position corresponding to a hollow cylindrical hole on filter paper
47: Distance to the oozed-out locus of water or aqueous solution
48: Filter paper at the time of measurement of water mobility value
49: Testing machine
50: Drive unit
51: Rotary axis
52: Fixing plate
53: Sample beating member
57: Cut
SL: Maximum length of exothermic part before the test

TL: Maximum length of exothermic part after the test

[Examples]

(Example 1)

A foot warming heat generating body 1 as shown in Figs. 1 and 2 has a shape of the whole of the foot and has a structure in which a heat generating composition molded body 5 is interposed by a substrate 6 and a covering material 7 and the outside from the periphery of the heat generating composition molded body 5 is heat sealed in a width of 10 mm.

The substrate 6 is a laminate of a 50 $\mu$m-thick air-impermeable and non-water absorptive polyethylene-made film 6B as produced by using a metallocene catalyst on a 100 $\mu$m-thick corrugated cardboard liner 6A as a core material.

Furthermore, the covering material 7 is a laminate of a polypropylene-made non-woven fabric 7B with a basis weight of about 80 g/m$^2$ on a 50 $\mu$m-thick polyethylene-made porous film 7A. Its moisture permeability is 1,000 g/m$^2$/24 hr.

As a heat generating composition which constitutes the heat generating composition molded body 5, one having a water mobility value of 8, which is a mixture consisting of 100 parts by weight of an iron powder (particle size: not more than 300 $\mu$m) as an exothermic substance, 0.8 parts by weight of a water absorptive polymer (particle size: not more than 300 $\mu$m), 5.0 parts by weight of a wood meal (particle size: not more than 300 $\mu$m), 7.0 parts by weight of active carbon (particle size: not more than 300 $\mu$m), 0.15 parts by weight of calcium hydroxide and 11 % of salt water, was used.

The foot warming heat generating body 1 was sealed in an air-impermeable bag and allowed to stand for one day. Thereafter, the foot warming heat generating body 1 was taken out, put in a leather shoe and then used. As a result, it was felt warm after 3 minutes, and an excellent thermal effect was obtained over 6 hours or more. Incidentally, the shape retaining degree was 92.

In the application of the foot warming heat generating body 1, since this foot warming heat generating body was formed in an ultra-thin form, it became flexible as a whole. As a result, this foot warming heat generating body had satisfactory adhesiveness and became fit to the application site, whereby an excellent warmth taking effect was obtained.

Furthermore, when an adhesive was used for bonding of the corrugated cardboard liner 6A which constitutes the substrate 6, there were caused problems such as peeling and leakage of the heat generating composition to soil the foot at the time of use. However, in the foot warming heat generating body 1 of the invention, since heat sealing was employed, such problems were not caused.

Fig. 3 shows an example in which a non-slip layer 12 is provided in the side of the surface of the substrate 6 of Example 1. Though a polyethylene-made film resulting from polymerization in the presence of a metallocene catalyst was used as this non-slip layer 12, the non-slip layer 12 may be covered by a protective layer such as a separator. Furthermore, in order to bond the substrate 6 and the covering material 7 to each other, heat sealing may be achieved after temporary adhesion via a sticky layer made of an SIS based hot melt based adhesive as formed by a melt blow method.

(Example 2)

Fig. 4 is a modification of Example 1 in which the shape of the Example 1 is provided with the arch of the foot and a portion extending from the arch of the foot. The shape retaining degree was 93.

A heat generating composition containing an iron powder having been subjected to a contact treatment with an oxidizing gas was used as the heat generating composition of this Example. That is, the contact treatment with an oxidizing gas was carried out by using a batchwise stirring tank composed of a mixer equipped with a rotary blade as an oxidizing gas contact treatment device and air as an oxidizing gas. First of all, a reaction mixture consisting of 100 parts by weight of a reduced iron powder (particle size: not more than 300 $\mu$m) and 5 parts by weight of 11 % salt water and having a water mobility value of less than 0.01 was charged in the contact treatment device vessel. Next, the upper portion of the contact treatment device vessel was opened to air, and the reaction mixture was subjected to self heat generation with stirring in the opened state to air under circumstances at 20°C and contact treated with the oxidizing gas until the exothermic temperature reached 35°C at a maximum exothermic temperature of 68°C, thereby obtaining the contact treated reaction mixture. With respect to the contact treated reaction mixture, an integrated intensity ratio of the integrated intensity of peaks (58.28, 64.92 and 82.22 (2θ/Deg.)) on the 110 plane of iron ($\alpha$Fe) and the integrated intensity of peaks (35.24, 41.59, 60.95, 72.70 and 76.51 (2θ/Deg.)) on the 220 plane of FeO (wustite) was determined by using an X-ray analyzer, thereby determining the amount of wustite.

The amount of wustite of the foregoing reaction mixture was 10 % by weight. Next, a heat generating composition having a water mobility value of 5 as obtained by mixing 5.3 parts by weight of active carbon (particle size: not more than 300 $\mu$m), 5 parts by weight of a wood meal (particle size: not more than 300 $\mu$m), 1.2 parts by weight of a water absorptive polymer (particle size: not more than 300 $\mu$m), 0.2 parts by weight of calcium hydroxide, 0.7 parts by weight of sodium sulfite, and 11 % salt water in the foregoing contact treated reaction mixture was used. An exothermic test of the heat generating composition was carried out. As a result, one minute after starting the test, the temperature of the heat generating composition was 30°C, and after 3 minutes, the temperature of the heat generating composition was 55°C. Thus, excellent exothermic rising properties were revealed.

(Example 3)

A foot warming heat generating body was prepared by using the same heat generating composition, substrate and covering material and so on as in Example 1, and a polyethylene-made film resulting from polymerization using a metallocene catalyst was stuck as a non-slip layer onto the substrate via a sticky layer.
In a foot warming heat generating body 1 as shown in the plan view of Fig. 5 (a), a heat generating composition was molded in two regions in the substantially central part of the shape of Example 1 by a force-through die molding method, and the periphery of this heat generating composition molded body 5 was sealed by a heat seal 10. This foot warming heat generating body was so compact that it was folded and piled in the substantially central part where the heat generating composition 4 was not laminated. Since the surface area can be made small at the time of storage, it is possible to reduce the deterioration of the heat generating composition caused due to the dispersion of water. Fig. 5(b) is one in which a perforation 11A is incorporated in a region which is free from the heat generating composition, thereby making it easy to fold the foot warming heat generating body. An oblique view to show the folded state is shown in Fig. 6. Fig. 6(c) shows one in which a portion extending from the arch of the foot is added. The shape retaining degree was 95.

(Example 4)

A foot warming heat generating body 1 as shown in the plan view of Fig. 7 is an example of the foot warming heat generating body 1 for warming a region in the foot finger side in a half-foot shape. Its cross-sectional view is shown in Fig. 8. A laminate of a polypropylene non-woven fabric 7B with a basis weight of 80 g/m$^2$ on a 40 $\mu$m-thick porous film 7A via an air-permeable hot melt based sticky layer was used as a covering material 7. Its moisture permeability was 650 g/m$^2$/24 hr. As a substrate 6, one prepared by laminating a polypropylene-made non-woven fabric 6C on one surface of a core material made of a 100 $\mu$m-thick corrugated cardboard liner 6A and a polyethylene film 6B on the other surface, further partially providing an adhesive layer 13 made of an acrylic adhesive on the polypropylene-made non-woven fabric 6C, and further proving a release paper 14 thereon was used. Incidentally, the periphery of a heat generating composition molded body 6 was heat sealed to provide a seal part 10 made of a heat seal part. The shape retaining degree was 93.
In the same manner as in Example 1, the foot warming heat generating body 1 was sealed in an air-impermeable bag; and after allowing it to stand for one day, this foot warming heat generating body 1 was taken out and then directly stuck to the backside of a portion extending from the foot fingers to the pads of the finger bases and used. As a result, a thermal effect was obtained over 6 hours.
Since the foot warming heat generating body 1 was formed in an ultra-thin form, it became flexible as a whole. As a result, the foot warming head generating body had a mild touch to the foot; it was easily deformed along the curved part of the foot; it was fit to the unevenness of the backside of the foot fingers; it was deformed while following up the movement of the backside of the foot very well; and it had good adhesiveness to the application site. Furthermore, it was admitted that the foot warming heat generating body is free from peeling from the application site during the use; that an excellent warmth taking effect is obtained; and that it warms the foot fingers from the backside.
In addition, at the time of use, the heat generating composition did not cause the movement, the exothermic temperature distribution of the foot warming heat generating body 1 was uniform, and the warmth could be taken comfortably with high safety and without causing a moderate-temperature burn.

(Example 5)

In a foot warming heat generating body 1 as shown in Fig. 9, a heat generating composition the same as in Example 1, except for changing the iron powder of the Example 1 to an iron powder having a thickness of an iron oxide film of 200 nm was used. In the rectangular foot warming heat generating body, the four corners thereof were cut into a round shape. The same core material as in Example 1 was used. The shape retaining degree was 90.
In this Example, the same heat generating composition molded body was laminated on the same substrate in the same manner as in Example 1. In addition, in the same manner as in Example 4, an air-permeable covering material having an SIS based hot melt based adhesive layer provided on the same porous film is employed; the air-permeable covering material was put on the heat generating composition molded body such that the adhesive layer was faced at the heat generating composition molded body; the entire surface was pressed by a sponge-like press roll; and after temporary adhesion, the periphery of the heat generating composition molded body was sealed by heat sealing.
The foot warming heat generating body was sealed in an air-tight bag; after allowing it to stand for one day, the air-tight bag was broken to take out the foot warming heat generating body; and this foot warming heat generating body was then laid on the sole of a shoe and used. As a result, an excellent thermal effect was obtained over 7 hours.

(Example 6)

As shown in Fig. 10, in a foot warming heat generating body 1, by using the same substrate and covering material as in Example 1, seven heat generating composition molded bodies 5 are put side by side from the toe to the heel direction. Then, the surroundings of the heat generating composition molded bodies 5 and the periphery 10 of the foot warming heat generating body 1 are heat sealed. The shape retaining degree was 96. Incidentally, the heat generating composition of this Example was obtained in the following manner. First all, a reaction mixture consisting of 100 parts by weight of a reduced iron powder (particle size: not more than 300 $\mu$m), 25 parts by weight of active carbon (particle size: not more than 300 $\mu$m), 3 parts by weight of a water absorptive polymer (particle size: not more than 300 $\mu$m), 0.5 parts by weight of calcium hydroxide, 0.7 parts by weight of sodium sulfite and 5 parts by weight of 11 % salt water and having a water mobility value of less than 0.01 was charged in a contact treatment device vessel. Next, the upper portion of the contact treatment device vessel was opened to air, and the reaction mixture was subjected to a contact treatment with an oxidizing gas with stirring in the opened state to air under circumstances at 20°C, thereby undergoing self heat generation for 3 minutes after charging in the vessel and starting the stirring. The maximum exothermic temperature was 53°C. A heat generating composition as prepared by mixing 11 % salt water in the heat generating mixture so as to have a water mobility value of 10 was used.

The foot warming heat generating body was sealed in an air-tight bag; after allowing it to stand for one day, the air-tight bag was broken to take out the foot warming heat generating body; and this foot warming heat generating body was then laid on the sole of a shoe and used. As a result, an excellent thermal effect was obtained over 7 hours.

(Example 7)

A foot warming heat generating body 1 as shown in the plan view of Fig. 11(a) is an example in which an air permeability adjusting material 15 is provided in the foot warming heat generating body 1 of Example 6. Fig. 11(b) is a cross-sectional view along the line X-X of the same. Fig. 11(c) is an enlarged cross-sectional view to show an example in the air permeability adjusting material 15 is fixed such that it is stretched on the top of a heat generating composition molded body 5 and the surface of a covering material between the adjacent heat generating composition molded bodies 5 and two spaces 17 are formed between the adjacent heat generating composition molded bides 5 and the air permeability adjusting material 15.

(Example 8)

A foot warming heat generating body 1 as shown in the plan view of Fig. 12 is an example in which a design 18 composed of characters and a pattern is provided in the shape of the whole of the foot.

(Example 9)

A foot warming heat generating body 1 as shown in Fig. 13 is an example in which a swollen portion for covering the toe part of a foot shape portion and swollen portions for covering the instep of the foot are provided and a heat generating composition molded body 5 is disposed in each portion.

According to this foot warming heat generating body 1, by extremely easily turning up the swollen portions from the toe to the instep at the time of use, it is possible to warm the toe from the three directions of the backside of the foot, the toe side and the instep side.

Incidentally, in these swollen proportions, a sticky layer may be laminated and fixed.

(Example 10)

Fig. 14 shows a production device 19 for suitably producing the foot warming heat generating body according to the invention. As shown in this drawing, the production device 19 is constituted of a drum type molding unit 20; press rolls 21, 21a for covering a heat generating composition molded body 5 as molded by this drum type molding unit 20 and laminated on a substrate 6 by a covering material and pressing it; die rolls 31, 31a for heat sealing; flattening rolls for flattening an exothermic part; and die cut rolls 32, 32a for cutting. Furthermore, the production device is provided with a substrate press roll 28, flattening rolls 27, 27a, and die cut rolls 32, 32a. In the drawing, 25, 25a each stands for a supporting roll of a belt conveyor 26. Incidentally, while not illustrated, after eliminating the die rolls 31, 31a for heat sealing and the die cut rolls 32, 32a, the belt conveyor 26 and the temporary adhering rolls or press rolls 27, 27a can be reversely driven by operating a switch. Furthermore, a drive unit 22 is a drive source of the belt conveyor 26 and the temporary rolls 21, 21a. The heat generating composition as used in Example 8 was used as the heat generating composition of this Example.

A screw 24 is provided within a hopper 23 of the drum type molding unit 20. A backup roll 20c is provided beneath the hopper 23 while interposing the belt conveyor therebetween.

The temporary rolls 21, 21a are provided on the way of the advancing direction of the belt conveyor 26 positioned beneath of this molding unit 20 while vertically interposing the belt conveyor 26 therebetween.

The substrate 6 wound in a rolled shape was fed out at 20 m/min, a heat generating composition 4 was thrown into the hopper 23, and the screw 24 was then rotated. Then, the heat generating composition 4 became a sheet-like heat generating composition molded body 5 on the belt conveyor 26 from the hopper 23 through the drum type molding unit 20; and the heat generating composition of the invention was molded in a size of 204 mm x 54 mm in maximum width at intervals of 20 mm in the central part of an air-impermeable and non-water absorptive polyethylene film which is the substrate 6 by force-through die molding using a trimming die having a thickness of 2.0 mm. In addition, in this laminated heat generating composition molded body, on the way of conveyance toward the (M) direction on the belt conveyor 26, a hot melt based adhesive from a melt blow machine 30 was melt blown on the covering material 7 which had been fed out thereon, namely on the substrate 6 and the laminated heat generating composition molded body, from a feed roll 29a, thereby providing an air-permeable sticky layer; and the substrate was then covered, followed by temporary adhering by the temporary adhering rolls 21, 21a. In addition, the periphery of the heat generating composition molded body was heat sealed in a seal width of 8 mm by the heat seal rolls 31, 31a; and the exothermic part was flattened by the flattening roll, followed by cutting by die cut rolls 32, 32a, thereby obtaining a foot warming heat generating body 1 of 220 mm x 70 mm in maximum width. Incidentally, a sticky layer constituted of a hot melt based adhesive may be provided on the heat generating composition molded body and/or the substrate 6 by using a metal blow machine 30a in place of the melt blow machine 30. Furthermore, a foot warming heat generating body may be produced by using a sticky layer-free covering material. In this case, the melt blow machine 30 and the melt blow machine 30a are not used. The melt blow machine, the temporary rolls and the flattening roll may not be used, if desired or can be properly selected and used.

By using such a heat generating composition, it becomes possible to stably laminate the heat generating composition in the central part on the non-water absorptive film on the substrate 3 by force-through die molding; the control of the laminated region can be performed highly precisely; and the film thickness can be controlled very thinly and uniformly so that it is possible to form the foot warming heat generating body in an ultra-thin form.

Incidentally, in this Example, though one pair of heat seal rolls were used, two or more plural pairs of heat seal rolls are connected to achieve heat sealing as the need arises.

Incidentally, the respective cut foot warming heat generating bodies are subsequently sent to a packaging step and sealed in a non-illustrated air-tight outer bag.

Furthermore, an oblique view of a drum molding unit for molding a full foot shape is shown in Fig. 15.

**Claims**

1. A foot warming heat generating body, **characterized in that** a heat generating composition molded body made of a heat generating composition which contains, as essential components, an exothermic substance, a carbon component, a reaction accelerator and water, has a water mobility value showing a surplus water content of from 0.01 to 20, with the water in the heat generating composition not functioning as a barrier layer, and is capable of causing an exothermic reaction upon contact with air is laminated on a substrate; a covering material is put thereon; the periphery of the heat generating composition molded body is sealed; the heat generating composition molded body has a shape retaining degree of 70 or more; and at least a part of the substrate or the covering material has permeability to air.

2. The foot warming heat generating body according to claim 1, **characterized in that** the heat generating composition contains a component resulting from a contact treatment of a heat generating mixture containing at least an iron powder, a carbon component, a reaction accelerator and water as essential components with an oxidizing gas.

3. The foot warming heat generating body according to claim 1, **characterized in that** the iron powder comprising particles, a surface of which is covered at leaset covered with an iron oxide film, the iron oxide film has a thickness of 3 nm or more, and the iron powder at least contains from 20 to 100 % by weight of an active iron powder having a region of an oxygen-free iron component in at least one region selected from a central part region of the iron powder and a region beneath the iron oxide film.

4. The foot warming heat generating body according to claim 1, **characterized in that** the iron powder comprising particles, a surface of each of which is at least partially covered with a wustite film and contains from 20 to 100 % by weight of an active iron powder having an amount of wustite of from 2 to 50 % by weight in terms of an X-ray peak intensity ratio.

5. The foot warming heat generating body according to claim 1, **characterized in that** the heat generating composition molded body is compressed.

6. The foot warming heat generating body according to claim 1, **characterized in that** an air-permeable sticky layer is provided for the purpose of bonding of the substrate, the covering material or the heat generating composition molded body.

7. The foot warming heat generating body according to claim 1, **characterized in that** the sealing is heat seal.

8. The foot warming heat generating body according to claim 7, **characterized in that** the heat seal is formed by heat sealing after temporary adhesion, and an adhesive component which constitutes the sticky layer and a component of a heat seal material which constitutes the heat seal layer are copresent in the heat seal part.

9. The foot warming heat generating body according to claim 1, **characterized in that** the heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

10. The foot warming heat generating body according to claim 1, **characterized in that** a core material is provided in a part of the foot warming heat generating body.

11. The foot warming heat generating body according to claim 1, **characterized in that** the substrate, the covering material, the core material or the heat generating composition molded body is subjected to compression processing.

12. The foot warming heat generating body according to claim 1, **characterized in that** the foot warming heat generating body is formed corresponding to the shape of a prescribed site of the foot.

13. The foot warming heat generating body according to claim 1, **characterized in that** a plural number of the heat generating composition molded bodies are sectioned by a sectioned part having the seal formed therein, and a plural number of sectional exothermic parts are disposed.

14. The foot warming heat generating body according to claim 1, **characterized in that** the sectional exothermic parts and the sectioned parts have a difference of altitude, the sectional exothermic parts and the sectioned parts are covered by an air permeability adjusting material, and air is taken in from the sides of the both end parts of the air permeability adjusting material.

15. The foot warming heat generating body according to claim 14, **characterized in that** the air permeability adjusting material is an air-impermeable raw material.

16. The foot warming heat generating body according to claim 1, **characterized in that** a fixing measure is provided in at least a part of the exposed surface of the substrate or the covering material.

17. The foot warming heat generating body according to claim 1, **characterized in that** the fixing measure is an adhesive layer, and the adhesive layer contains at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous substance, a moisturizer, a functional substance, and a mixture thereof.

18. A process for producing a foot warming heat generating body according to claim 1, **characterized in that** the production process is a basic process for successively carrying out a first step, a second step, a third step and a fourth step; and a step selected from the following first step, second step (second A step, second B step, second C step, and second D step), third step (third A step, third B step, and third C step), fourth step (fourth A step), fifth step, sixth step (sixth A step and sixth B step), seventh step (seventh A step), eighth step, ninth step and tenth step inclusive of duplicated steps thereof is arbitrarily mediated in the basic process, as the need arises:

First step: production step of heat generating composition

Second step: molding step (substrate and magnet), second A step: force-through die molding step (trimming die and leveling plate), second B step: cast molding method (casting die and leveling plate), second C step: force-in die molding step (trimming die and pushing plate), second D step: in-mold compression step

Third step: lamination, spraying and coating step for heat generating composition, etc., third 3A step: setting-up step of air-permeable polymer, third B step: lamination, spraying and coating step for substrate, etc., third C step: surface treatment step of heat generating composition

Fourth step: covering step (covering material), fourth A step: covering step (underlay material)

Fifth step: pressurizing step

Sixth step: sealing step, sixth A step: temporary adhesion and heat sealing step, sixth B step: deadhesion step

Seventh step: setting-up step of non-slip layer, seventh A step: setting-up step of air adjusting material

Eighth step: punching-out step of heat generating body

Ninth step: accommodating step of foot warming heat generating body in air-impermeable accommodating bag

Tenth step: punching-out step of outer bag

## FIG. 1

## FIG. 2

## FIG. 3

FIG. 4

FIG.5(a)   FIG.5(b)   FIG.5(c)

FIG.6

## FIG.7

## FIG.8

## FIG.9

## FIG.10(a)

## FIG.10(b)

## FIG.10(c)

## FIG.11(a)

## FIG.11(b)

## FIG.11(c)

*FIG.12*

*FIG.13*

## FIG. 14

## FIG. 15

## FIG.16(a)

## FIG.16(b)          FIG.16(c)

## Fig. 17

# FIG.18

# FIG.19

# FIG.20

# FIG.21

## INTERNATIONAL SEARCH REPORT

| | International application No. |
|---|---|
| | PCT/JP2005/013011 |

A.  CLASSIFICATION OF SUBJECT MATTER
     Int.Cl⁷  A61F7/08, C09K5/16

According to International Patent Classification (IPC) or to both national classification and IPC

B.  FIELDS SEARCHED

Minimum documentation searched (classification system followed by classification symbols)
     Int.Cl⁷  A61F7/08, C09K5/16

Documentation searched other than minimum documentation to the extent that such documents are included in the fields searched
     Jitsuyo Shinan Koho            1922-1996    Jitsuyo Shinan Toroku Koho    1996-2005
     Kokai Jitsuyo Shinan Koho     1971-2005    Toroku Jitsuyo Shinan Koho    1994-2005

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| X<br><br>Y<br>A | JP 2003-334211 A  (Maikoru Kabushiki Kaisha),<br>25 November, 2003 (25.11.03),<br>Full text; Figs. 1 to 23<br>(Family: none) | 1-3,5-13,16,<br>17<br>14,15,18<br>4 |
| P,Y<br>P,A | JP 2004-208978 A  (MYCOAL PRODUCTS CORP.),<br>29 July, 2004 (29.07.04),<br>Full text; Figs. 1 to 20<br>& WO 2004/061045 A1 | 2,5-13,16,17<br>3,14,15,18 |
| A | JP 10-17907 A  (Dowa Iron Powder Co., Ltd.),<br>20 January, 1998 (20.01.98),<br>Column 3, line 26 to column 4, line 14;<br>Fig. 1<br>(Family: none) | 4 |

| ☒  Further documents are listed in the continuation of Box C. | ☐  See patent family annex. |
|---|---|

| *   Special categories of cited documents: | "T"  later document published after the international filing date or priority date and not in conflict with the application but cited to understand the principle or theory underlying the invention |
|---|---|
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search<br>     08 September, 2005 (08.09.05) | Date of mailing of the international search report<br>     11 October, 2005 (11.10.05) |
|---|---|
| Name and mailing address of the ISA/<br>     Japanese Patent Office | Authorized officer |
| Facsimile No. | Telephone No. |

Form PCT/ISA/210 (second sheet) (January 2004)

## INTERNATIONAL SEARCH REPORT

| International application No. |
|---|
| PCT/JP2005/013011 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 11-512954 A  (The Procter & Gamble Co.), 09 November, 1999 (09.11.99), Page 7, line 27 to page 10, line 1; Fig. 3 & JP 3545769 B        & WO 1997/049361 A1 & AU 735088 B | 14,15,18 |
| A | JP 2002-155273 A  (Kaoru USUI), 28 May, 2002 (28.05.02), Full text; Figs. 1 to 14 (Family: none) | 1-18 |
| A | JP 9-276317 A  (Kabushiki Kaisha Genchi Kenkyusho), 28 October, 1997 (28.10.97), Full text; Figs. 1 to 14 & US 2001/23366 A1      & US 6264681 B1 & GB 2312846 A      & DE 19715097 A & FR 2747304 A      & CA 2202296 A & CN 1180515 A | 1-18 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 61008013 A
- JP 2172460 A
- JP 62000347 A
- JP 9276317 A
- JP 3161605 B
- JP 11508314 T
- JP 2002514104 T
- JP 2001507593 T
- JP 4293989 A

- JP 6343658 A
- JP 7194641 A
- JP 2002200108 A
- JP 10265373 A
- JP 9087173 A
- JP 6145050 A
- JP 6199660 A
- JP 10279466 A
- JP 10182408 A